# EUROPEAN PATENT APPLICATION

(11) **EP 4 847 509 A1**
(43) Date of publication of application: **02.12.2026**
(21) Application number: 25781717.1
(22) Date of filing: 02.04.2025
(51) Int. Cl.: C07D 471/04, C07D 487/04, C07D 498/04, A61K 31/437, A61K 31/4025, A61K 31/403, A61K 31/407, A61P 3/10, A61P 3/06, A61P 3/04, A61P 25/00, A61P 13/12, A61P 9/12

(54) **GLP-1 RECEPTOR AGONIST, AND PREPARATION METHOD THEREFOR AND USE THEREOF**

(30) Priority: 03.04.2024 CN 202410405950; 30.09.2024 CN 202411388926; 24.12.2024 CN 202411923665; 22.01.2025 CN 202510107397
(71) Applicant: Guangzhou Unirise Pharmaceutical Co., Ltd., Guangzhou, Guangdong 510000 (CN); Guangzhou Apichope Pharmaceutical Co., Ltd., Guangzhou, Guangdong 510000 (CN); Guangzhou Runlin Pharmaceutical Technology Co., Ltd., Guangzhou, Guangdong 510000 (CN); Apichope Bio Pharmaceutical Co., Ltd., Guangzhou, Guangdong 510000 (CN)
(72) Inventor: YANG, Wenqian, Guangzhou, Guangdong 510000 (CN); GUAN, Xinyu, Guangzhou, Guangdong 510000 (CN); CHEN, Hongwei, Guangzhou, Guangdong 510000 (CN); ZHOU, Xin, Guangzhou, Guangdong 510000 (CN); SU, Qiumin, Guangzhou, Guangdong 510000 (CN); XU, Cong, Guangzhou, Guangdong 510000 (CN); WANG, Hui, Guangzhou, Guangdong 510000 (CN); YU, Tianzhu, Guangzhou, Guangdong 510000 (CN); ZENG, Dongwen, Guangzhou, Guangdong 510000 (CN); CHEN, Ming, Guangzhou, Guangdong 510000 (CN)
(74) Representative: Michalski Hüttermann & Partner mbB
(86) International application number: PCT/CN2025/086812
(87) International publication number: WO 2025/209515

(57) **Abstract**

The present invention discloses a class of compounds and use thereof in drugs. Specifically, the present invention relates to a new class of compounds and a pharmaceutical composition containing the compounds. The present invention further relates to a method for preparing the compounds, and use of the compounds or the pharmaceutical composition in preparation of drugs for treating GLP-1 receptor agonist-mediated diseases and/or disorders, particularly use in preparation of drugs for treating diabetes, non-alcoholic fatty liver disease, obesity, myocardial infarction, heart failure, diabetic nephropathy, Parkinson's disease and Alzheimer's disease.

## Description

### CROSS-REFERENCE TO RELATED APPLICATIONS

The present application claims the following priorities:
CN202410405950.6, filed on April 3, 2024; CN202411388926.2, filed on September 30, 2024; CN202411923665.X, filed on December 24, 2024; and CN202510107397.2, filed on January 22, 2025.

### TECHNICAL FIELD

The present invention relates to the field of chemical and pharmaceutical technologies, and in particular, to a GLP-1 receptor agonist, and a preparation method therefor and use thereof.

### BACKGROUND

Glucagon-like peptide-1 (GLP-1) is a hormone mainly produced by intestinal L cells and belongs to incretin. GLP-1 receptors were first discovered in 1987 by performing a radioligand binding assay with GLP-1 in a rat pancreatic islet cell culture, and shortly thereafter, GLP-1 receptors were localized in various tissues including the brain. GLP-1 receptors are expressed in various tissues, including multiple regions of pancreas, lungs, heart, kidneys, stomach, intestine, pituitary gland, vagus nerve, and the central nervous system.

A GLP-1 receptor agonist enhances insulin secretion and inhibits glucagon secretion in a glucose concentration dependent manner by activating GLP-1 receptors, and can delay gastric emptying and reduce food intake by central appetite suppression, thereby achieving the effects of lowering blood glucose (Ralph A. DeFronzo et al, 2013; Yiming Mu et, Dalong Zhu et al, 2020), reducing weight (Yingying Wang et al, 2020), and the like. GLP-1 analogues have been developed into effective therapeutic agents for diabetes and obesity, such as liraglutide and exenatide. Activation of GLP-1 receptors can reduce the area of cerebellar infarction by enhancing a cell survival signaling pathway, reducing ischemia reperfusion injury, promoting brain repair, and inhibiting inflammatory response and oxidative stress (Zhang L et al, 2021; He W et al, 2020; Basalay MV et al, 2019; Grieco M et al, 2019).

Studies have shown that GLP-1 receptor agonists are a new class of anti-diabetic drugs and an important part of treatment for type 2 diabetes patients (Maselli DB et al, 2021; Nauck MA et al, 2021; Brown E et al, 2018).

In addition, a large number of studies have shown that GLP-1 receptor agonists also have therapeutic effects on cardiovascular diseases, chronic kidney disease, obesity and overweight, dyslipidemia, hypertension, non-alcoholic fatty liver, polycystic ovary syndrome, and the like in nondiabetic patients (Dan Xu et al, 2022).

GLP-1 receptor agonists not only have hypoglycemic effects, but also have protective effects against heart and cerebral vessels, such as cerebral apoplexy, cognitive impairment, Parkinson's disease, and Alzheimer's disease (Tianyuan Guan et al, 2019). Some large-scale clinical trials have also shown that GLP-1 receptor agonists can reduce the risk of cardiovascular events (Hirsch IB et al, 2019; Wen S et al, 2021).

In addition, due to widespread distribution of GLP-1 receptors in the body and the ability thereof to act on multiple organs and tissues, semaglutide and tirzepatide are undergoing multi-indication expansion, including non-alcoholic fatty hepatitis, MACE, HFpEF, kidney disease, Alzheimer's disease, and the like, which are all treatment areas with significant unmet clinical needs.

At present, researchers have conducted some studies for the purpose of finding therapeutic agents that can effectively activate GLP-1 receptors. PCT applications WO2018056453A1, WO2018109607, WO2019239319, WO2019239371, WO2020103815, WO2020207474, WO2020263695, WO2021154796, WO2021112538, WO2021096304, WO2021096284, WO2021081207, WO2021018023, WO2021254470, WO2021249492, WO2022007979, WO2022017338A1, WO2022031994, WO2022040600, WO2022068772, and WO2022116693 have disclosed many small molecule compounds, which are used as GLP-1 receptor agonists to prevent or treat diabetes, diabetes complications, non-alcoholic fatty liver disease, obesity, hypertension, hyperlipidemia, hypercholesterolemia, hypertriglyceridemia, atherosclerosis, stroke, coronary heart disease, myocardial infarction, congestive heart failure, arrhythmia, cerebral infarction, diabetic nephropathy, Parkinson's disease, Alzheimer's disease, or dementia. However, there is still an urgent need for more and better GLP-1 receptor agonists in clinical practice.

### SUMMARY

The present invention provides a compound or a pharmaceutical composition thereof, which can act as an agonist of GLP-1 receptors. The present invention further relates to use of the compound or the pharmaceutical composition thereof for preparation of drugs, and the drugs activate GLP-1 receptors by the compound to treat diseases and/or disorders. The present invention further describes a synthesis method of the compound. The compound of the present invention exhibits excellent biological activity and pharmacokinetic properties.

Specifically:
In one aspect, the present invention relates to a compound, which is a compound represented by formula (I), or a stereoisomer, geometric isomer, tautomer, oxynitride, hydrate, solvate, metabolite, pharmaceutically acceptable salt, or prodrug of the compound represented by formula (I), 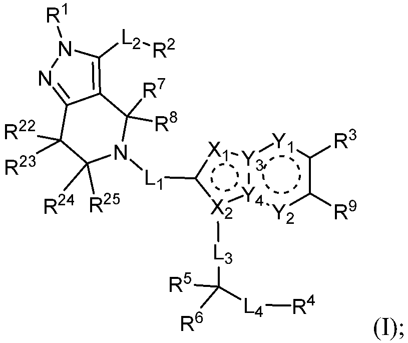 where
L₁ is -C(=O)-, -S(=O)₂-, or -CR¹⁰R¹¹-;
L₂ is 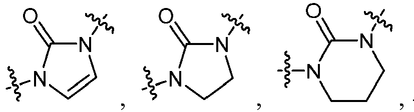 -NR¹²-, -C(=O)NR¹³-, -NR¹⁴C(=O)NR¹⁵-, or - CR¹⁶R¹⁷-;
L₃ is a bond or -CR¹⁸R¹⁹-;
L₄ is a bond or -CR²⁰R²¹-;
X₁ is CH, NH, O, or S;
X₂ is C or N;
Y₁ and Y₂ are each independently CH or N;
Y₃ and Y₄ are each independently C or N;
R¹ is phenyl or 5-10 membered heteroaryl, the phenyl and 5-10 membered heteroaryl being independently and optionally substituted with 1, 2, or 3 R^{1a};
R^{1a} is D, F, Cl, Br, I, CN, hydroxyl, oxo, amino, nitro, C₁₋₆ alkyl, C₁₋₆ alkoxyl, C₁₋₆ alkylthio, C₁₋₆ alkylamino, C₃₋₆ cycloalkyl, or 3-6 membered heterocyclyl, the C₁₋₆ alkyl, C₁₋₆ alkoxyl, C₁₋₆ alkylthio, C₁₋₆ alkylamino, C₃₋₆ cycloalkyl, and 3-6 membered heterocyclyl being independently and optionally substituted with 1, 2, or 3 substituents selected from D, F, Cl, Br, I, hydroxyl, cyano, amino, and nitro;
R² is C₁₋₆ alkyl, C₃₋₈ cycloalkyl, 3-8 membered heterocyclyl, phenyl, or 5-10 membered heteroaryl, the C₁₋₆ alkyl, C₃₋₈ cycloalkyl, 3-8 membered heterocyclyl, phenyl, and 5-10 membered heteroaryl being independently and optionally substituted with 1, 2, or 3 R^{2a};
R^{2a} is D, F, Cl, Br, I, CN, hydroxyl, oxo, amino, nitro, -P(=O)R^{2b}R^{2c}, C₁₋₆ alkyl, C₁₋₆ haloalkyl, C₁₋₆ alkoxyl, C₁₋₆ alkylthio, C₁₋₆ alkylamino, C₃₋₆ cycloalkyl, or 3-6 membered heterocyclyl, the C₁₋₆ alkyl, C₁₋₆ alkoxyl, C₁₋₆ alkylthio, C₁₋₆ alkylamino, C₃₋₆ cycloalkyl, and 3-6 membered heterocyclyl being independently and optionally substituted with 1, 2, or 3 substituents selected from D, F, Cl, Br, I, hydroxyl, cyano, amino, and nitro;
R^{2b} and R^{2c} are each independently C₁₋₆ alkyl or C₃₋₆ cycloalkyl;
or R^{2b} and R^{2c}, together with phosphorus atoms connected therewith, form 5-6 membered heterocyclyl, the 5-6 membered heterocyclyl being optionally substituted with 1, 2, or 3 substituents selected from D, F, Cl, Br, I, hydroxyl, cyano, amino, nitro, methyl, and ethyl;
R³ is 3-8 membered heterocyclyl or 5-10 membered heteroaryl, the 3-8 membered heterocyclyl and 5-10 membered heteroaryl being independently and optionally substituted with 1, 2, or 3 R^{3a};
R^{3a} is D, F, Cl, Br, I, CN, hydroxyl, oxo, amino, nitro, C₁₋₆ alkyl, C₁₋₆ alkoxyl, C₁₋₆ alkylthio, C₁₋₆ alkylamino, C₃₋₆ cycloalkyl, or 3-6 membered heterocyclyl, the C₁₋₆ alkyl, C₁₋₆ alkoxyl, C₁₋₆ alkylthio, C₁₋₆ alkylamino, C₃₋₆ cycloalkyl, and 3-6 membered heterocyclyl being independently and optionally substituted with 1, 2, or 3 substituents selected from D, F, Cl, Br, I, hydroxyl, cyano, amino, and nitro;
R⁴ is 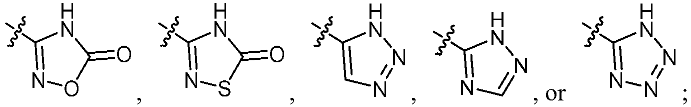
R⁵ and R⁶ are each independently H, D, C₁₋₆ alkyl, C₃₋₆ cycloalkyl, or 3-6 membered heterocyclyl, the C₁₋₆ alkyl, C₃₋₆ cycloalkyl, and 3-6 membered heterocyclyl being independently and optionally substituted with 1, 2, or 3 substituents selected from D, F, Cl, Br, I, hydroxyl, cyano, amino, nitro, and C₃₋₆ cycloalkyl;
or R⁵ and R⁶, together with carbon atoms connected therewith, form C₃₋₆ cycloalkyl, the C₃₋₆ cycloalkyl being optionally substituted with 1, 2, or 3 substituents selected from D, F, Cl, Br, I, hydroxyl, cyano, amino, nitro, C₁₋₃ alkyl, C₁₋₃ haloalkyl, C₁₋₃ alkoxyl, C₁₋₃ alkylthio, and C₁₋₃ alkylamino;
R⁷, R⁸, R¹⁰, R¹¹, R¹⁶, R¹⁷, R¹⁸, R¹⁹, R²⁰, R²¹, R²², R²³, R²⁴, and R²⁵ are each independently H, D, F, Cl, Br, I, CN, hydroxyl, oxo, amino, nitro, C₁₋₆ alkyl, C₁₋₆ alkoxyl, C₁₋₆ alkylthio, C₁₋₆ alkylamino, C₃₋₆ cycloalkyl, or 3-6 membered heterocyclyl, the C₁₋₆ alkyl, C₁₋₆ alkoxyl, C₁₋₆ alkylthio, C₁₋₆ alkylamino, C₃₋₆ cycloalkyl, and 3-6 membered heterocyclyl being independently and optionally substituted with 1, 2, or 3 substituents selected from D, F, Cl, Br, I, CN, hydroxyl, amino, or nitro, under a condition that at least one of R²², R²³, R²⁴, and R²⁵ is not hydrogen;
or R²² and R²³, R²⁴ and R²⁵, R²³ and R²⁴, independently and optionally together with carbon atoms connected therewith, form C₃₋₆ cycloalkyl, the C₃₋₆ cycloalkyl being optionally substituted with 1, 2, or 3 substituents selected from D, F, Cl, Br, I, hydroxyl, cyano, amino, nitro, C₁₋₃ alkyl, C₁₋₃ haloalkyl, C₁₋₃ alkoxyl, C₁₋₃ alkylthio, or C₁₋₃ alkylamino;
R⁹ is H, D, F, Cl, Br, I, CN, hydroxyl, amino, nitro, phenyl, C₂₋₆ alkenyl, C₂₋₆ alkynyl, C₁₋₆ alkyl, C₁₋₆ haloalkyl, C₁₋₆ alkoxyl, C₁₋₆ alkylamino, or C₃₋₆ cycloalkyl, the phenyl, C₂₋₆ alkenyl, C₂₋₆ alkynyl, C₁₋₆ alkyl, C₁₋₆ haloalkyl, C₁₋₆ alkoxyl, C₁₋₆ alkylamino, and C₃₋₆ cycloalkyl being independently and optionally substituted with 1, 2, or 3 substituents selected from D, F, Cl, Br, I, CN, hydroxyl, amino, nitro, C₁₋₆ alkoxyl, C₁₋₆ alkylamino, or C₃₋₆ cycloalkyl; and
R¹², R¹³, R¹⁴, and R¹⁵ are each independently H, C₁₋₆ alkyl, C₂₋₆ alkenyl, C₂₋₆ alkynyl, C₃₋₆ cycloalkyl, or 3-6 membered heterocyclyl, the C₁₋₆ alkyl, C₂₋₆ alkenyl, C₂₋₆ alkynyl, C₃₋₆ cycloalkyl, and 3-6 membered heterocyclyl being independently and optionally substituted with 1, 2, or 3 substituents selected from D, F, Cl, Br, I, CN, hydroxyl, amino, and nitro;
where formula (I) is not the following compound:

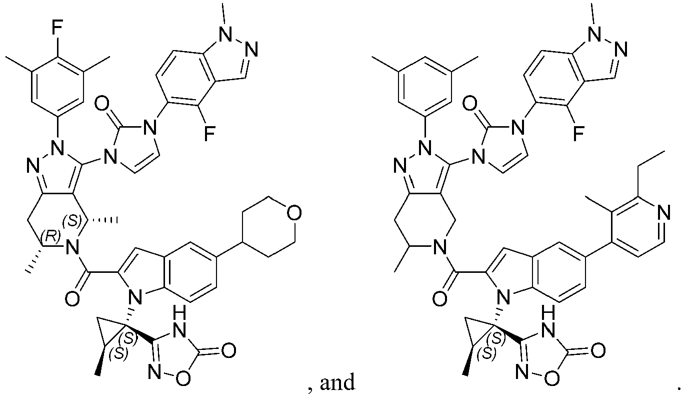

In some embodiments, 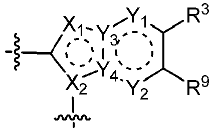 is

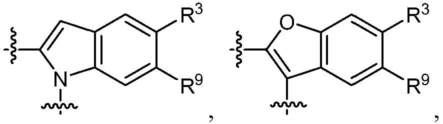

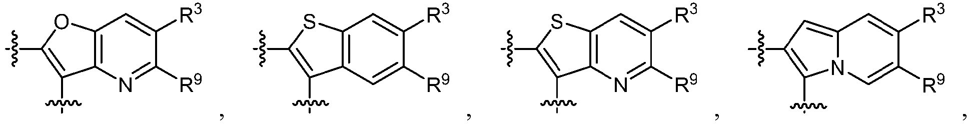

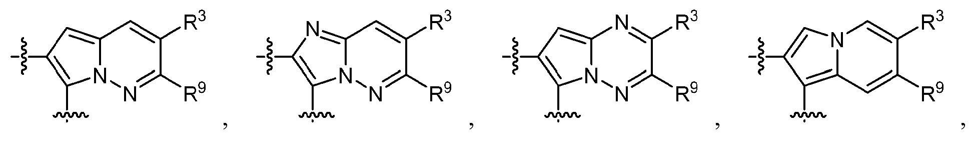

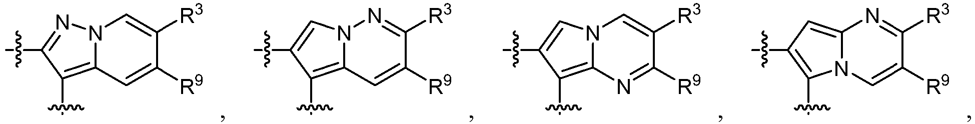

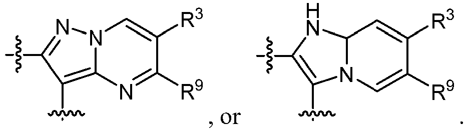

In some embodiments, R¹ is phenyl or 5-10 membered heteroaryl, the phenyl and 5-10 membered heteroaryl being independently and optionally substituted with 1, 2, or 3 R^{1a};
R^{1a} is D, F, Cl, Br, I, CN, hydroxyl, oxo, amino, nitro, C₁₋₃ alkyl, C₁₋₃ alkoxyl, C₁₋₃ alkylthio, C₁₋₃ alkylamino, C₃₋₆ cycloalkyl, or 3-6 membered heterocyclyl, the C₁₋₃ alkyl, C₁₋₃ alkoxyl, C₁₋₃ alkylthio, C₁₋₃ alkylamino, C₃₋₆ cycloalkyl, and 3-6 membered heterocyclyl being independently and optionally substituted with 1, 2, or 3 substituents selected from D, F, Cl, Br, I, hydroxyl, cyano, amino, and nitro;
R² is C₁₋₃ alkyl, C₃₋₆ cycloalkyl, 3-6 membered heterocyclyl, phenyl, or 5-10 membered heteroaryl, the C₁₋₃ alkyl, C₃₋₆ cycloalkyl, 3-6 membered heterocyclyl, phenyl, and 5-10 membered heteroaryl being independently and optionally substituted with 1, 2, or 3 R^{2a};
R^{2a} is D, F, Cl, Br, I, CN, hydroxyl, oxo, amino, nitro, -P(=O)R^{2b}R^{2c}, C₁₋₃ alkyl, C₁₋₃ haloalkyl, C₁₋₃ alkoxyl, C₁₋₃ alkylthio, C₁₋₃ alkylamino, C₃₋₆ cycloalkyl, or 3-6 membered heterocyclyl, the C₁₋₃ alkyl, C₁₋₃ alkoxyl, C₁₋₃ alkylthio, C₁₋₃ alkylamino, C₃₋₆ cycloalkyl, and 3-6 membered heterocyclyl being independently and optionally substituted with 1, 2, or 3 substituents selected from D, F, Cl, Br, I, hydroxyl, cyano, amino, and nitro;
R^{2b} and R^{2c} are each independently C₁₋₆ alkyl or C₃₋₆ cycloalkyl;
or R^{2b} and R^{2c}, together with phosphorus atoms connected therewith, form 5-6 membered heterocyclyl, the 5-6 membered heterocyclyl being optionally substituted with 1, 2, or 3 substituents selected from D, F, Cl, Br, I, hydroxyl, cyano, amino, nitro, methyl, and ethyl;
R³ is 3-6 membered heterocyclyl or 5-10 membered heteroaryl, the 3-6 membered heterocyclyl and 5-10 membered heteroaryl being independently and optionally substituted with 1, 2, or 3 R^{3a}; and
R^{3a} is D, F, Cl, Br, I, CN, hydroxyl, oxo, amino, nitro, C₁₋₃ alkyl, C₁₋₃ alkoxyl, C₁₋₃ alkylthio, C₁₋₃ alkylamino, C₃₋₆ cycloalkyl, or 3-6 membered heterocyclyl, the C₁₋₃ alkyl, C₁₋₃ alkoxyl, C₁₋₃ alkylthio, C₁₋₃ alkylamino, C₃₋₆ cycloalkyl, and 3-6 membered heterocyclyl being independently and optionally substituted with 1, 2, or 3 substituents selected from D, F, Cl, Br, I, hydroxyl, cyano, amino, and nitro.

In some embodiments, R¹ is phenyl, 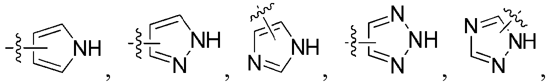 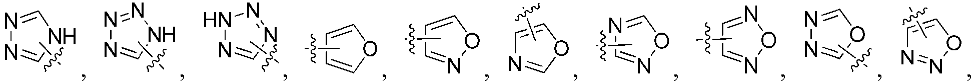 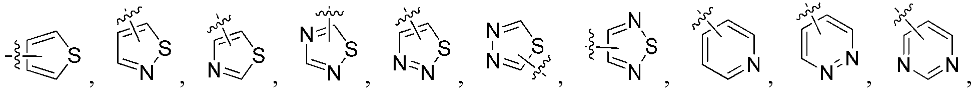 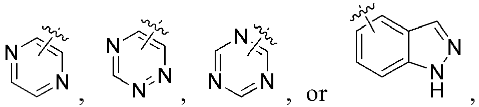 the phenyl, 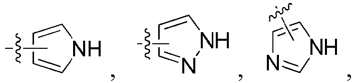 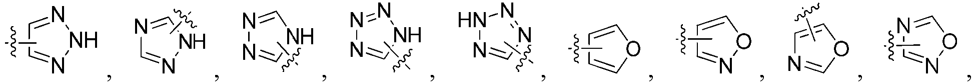 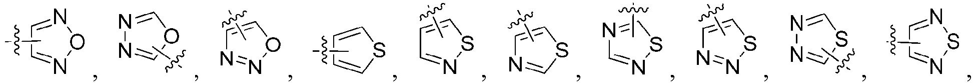 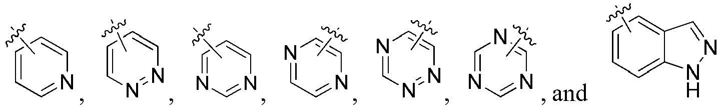 being independently and optionally substituted with 1, 2, or 3 R^{1a};
R^{1a} is D, F, Cl, Br, I, CN, hydroxyl, oxo, amino, nitro, methyl, ethyl, n-propyl, isopropyl, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, or morpholinyl, the methyl, ethyl, n-propyl, isopropyl, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, and morpholinyl being independently and optionally substituted with 1, 2, or 3 substituents selected from D, F, Cl, Br, I, hydroxyl, cyano, amino, and nitro;
R² is methyl, ethyl, n-propyl, isopropyl, cyclopropyl, cyclobutyl, cyclopentyl, cyclohexyl, oxacyclobutyl, azacyclobutyl, pyrrolidinyl, tetrahydrofuranyl, piperidyl, piperazinyl, morpholinyl, phenyl, 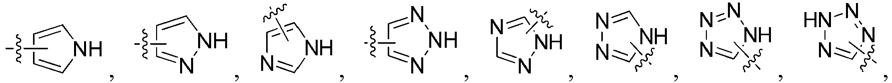 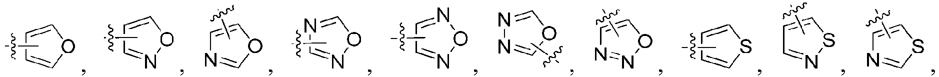 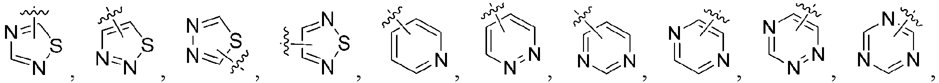 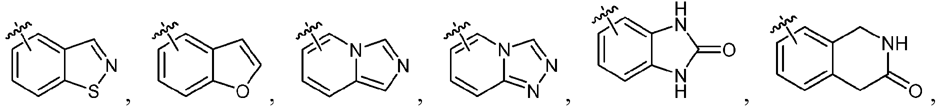 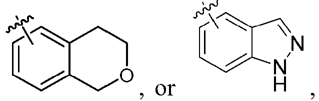 the methyl, ethyl, n-propyl, isopropyl, cyclopropyl, cyclobutyl, cyclopentyl, cyclohexyl, oxacyclobutyl, azacyclobutyl, pyrrolidinyl, tetrahydrofuranyl, piperidyl, piperazinyl, morpholinyl, phenyl, 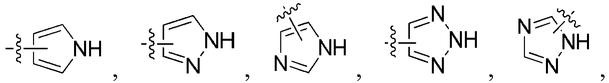 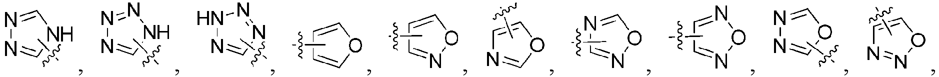 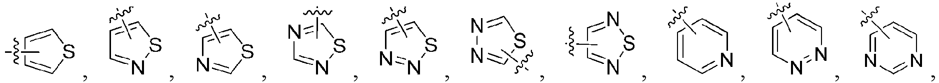 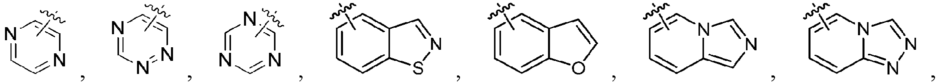 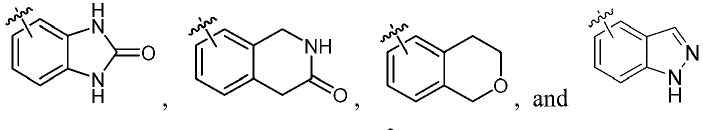 being independently and optionally substituted with 1, 2, or 3 R^{2a};
R^{2a} is D, F, Cl, Br, I, CN, hydroxyl, oxo, amino, nitro, -P(=O)R^{2b}R^{2c}, methyl, ethyl, n-propyl, isopropyl, -CHF₂, -CF₃, -CHFCH₂F, -CF₂CHF₂, -CH₂CF₃, -CH₂CF₂CHF₂, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, or morpholinyl, the methyl, ethyl, n-propyl, isopropyl, -CHF₂, -CHFCH₂F, -CF₂CHF₂, -CH₂CF₃, - CH₂CF₂CHF₂, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, and morpholinyl being independently and optionally substituted with 1, 2, or 3 substituents selected from D, F, Cl, Br, I, hydroxyl, cyano, amino, and nitro;
R^{2b} and R^{2c} are each independently methyl, ethyl, n-propyl, isopropyl, tert-butyl, cyclopropyl, cyclobutyl, cyclopentyl, or cyclohexyl;
or R^{2b} and R^{2c}, together with phosphorus atoms connected therewith, form 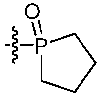 or 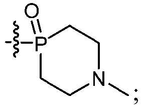
R³ is oxacyclobutyl, azacyclobutyl, pyrrolidinyl, tetrahydrofuranyl, tetrahydropyranyl, piperidyl, piperazinyl, morpholinyl, 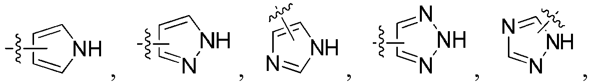 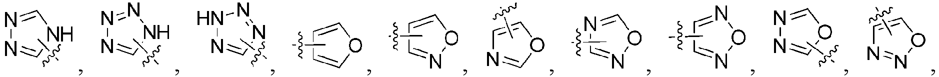 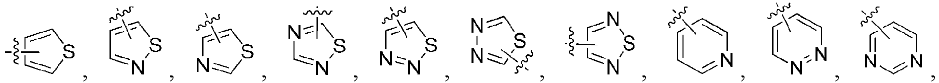 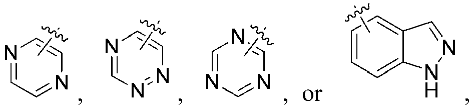 the oxacyclobutyl, azacyclobutyl, pyrrolidinyl, tetrahydrofuranyl, tetrahydropyranyl, piperidyl, piperazinyl, morpholinyl, 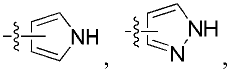 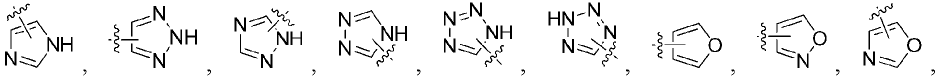 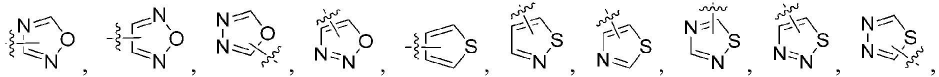 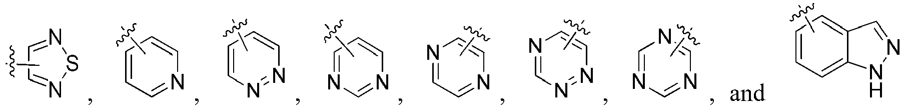 being independently and optionally substituted with 1, 2, or 3 R^{3a}; and
R^{3a} is D, F, Cl, Br, I, CN, hydroxyl, oxo, amino, nitro, methyl, ethyl, n-propyl, isopropyl, - CHF₂, -CF₃, -CHFCH₂F, -CF₂CHF₂, -CH₂CF₃, -CH₂CF₂CHF₂, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, or morpholinyl, the methyl, ethyl, n-propyl, isopropyl, -CHF₂, -CHFCH₂F, -CF₂CHF₂, -CH₂CF₃, -CH₂CF₂CHF₂, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, and morpholinyl being independently and optionally substituted with 1, 2, or 3 substituents selected from D, F, Cl, Br, **I,** hydroxyl, cyano, amino, and nitro.

**In** some embodiments, R⁵ and R⁶ are each independently H, D, methyl, ethyl, n-propyl, isopropyl, cyclopropyl, cyclobutyl, cyclopentyl, cyclohexyl, oxacyclobutyl, azacyclobutyl, pyrrolidinyl, tetrahydrofuranyl, piperidyl, piperazinyl, or morpholinyl, the methyl, ethyl, n-propyl, isopropyl, cyclopropyl, cyclobutyl, cyclopentyl, cyclohexyl, oxacyclobutyl, azacyclobutyl, pyrrolidinyl, tetrahydrofuranyl, piperidyl, piperazinyl, and morpholinyl being independently and optionally substituted with 1, 2, or 3 substituents selected from D, F, Cl, Br, **I,** hydroxyl, cyano, amino, nitro, and C₃₋₆ cycloalkyl;
or R⁵ and R⁶, together with carbon atoms connected therewith, form cyclopropyl, cyclobutyl, cyclopentyl, or cyclohexyl, the cyclopropyl, cyclobutyl, cyclopentyl, and cyclohexyl being independently and optionally substituted with 1, 2, or 3 substituents selected from D, F, Cl, Br, **I,** hydroxyl, cyano, amino, nitro, methyl, ethyl, n-propyl, isopropyl, -CHF₂, -CF₃, -CHFCH₂F, - CF₂CHF₂, -CH₂CF₃, -CH₂CF₂CHF₂, methoxyl, ethoxyl, methylthio, N-methylamino, and *N-*ethylamino;
R⁷, R⁸, R¹⁰, R¹¹, R¹⁶ , R¹⁷, R¹⁸, R¹⁹, R²⁰, R²¹, R²², R²³, R²⁴, and R²⁵ are each independently H, D, F, Cl, Br, **I,** CN, hydroxyl, oxo, amino, nitro, methyl, ethyl, n-propyl, isopropyl, -CHF₂, -CF₃, - CHFCH₂F, -CF₂CHF₂, -CH₂CF₃, -CH₂CF₂CHF₂, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, or morpholinyl, the methyl, ethyl, n-propyl, isopropyl, -CHF₂, -CHFCH₂F, -CF₂CHF₂, -CH₂CF₃, -CH₂CF₂CHF₂, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, and morpholinyl being independently and optionally substituted with 1, 2, or 3 substituents selected from D, F, Cl, Br, **I,** CN, hydroxyl, amino, and nitro, under a condition that at least one of R²², R²³, R²⁴, and R²⁵ is not hydrogen;
or R²² and R²³, R²⁴ and R²⁵, R²³ and R²⁴, independently and optionally together with carbon atoms connected therewith, form cyclopropyl, cyclobutyl, cyclopentyl, or cyclohexyl, the cyclopropyl, cyclobutyl, cyclopentyl, and cyclohexyl being independently and optionally substituted with 1, 2, or 3 substituents selected from D, F, Cl, Br, **I,** hydroxyl, cyano, amino, nitro, methyl, ethyl, n-propyl, isopropyl, -CHF₂, -CF₃, -CHFCH₂F, -CF₂CHF₂, -CH₂CF₃, -CH₂CF₂CHF₂, methoxyl, ethoxyl, methylthio, N-methylamino, and N-ethylamino;
R⁹ is H, D, F, Cl, Br, **I,** CN, hydroxyl, amino, nitro, phenyl, ethenyl, allyl, ethynyl, propargyl, 1-propynyl, methyl, ethyl, n-propyl, isopropyl, -CHF₂, -CF₃, -CHFCH₂F, -CF₂CHF₂, -CH₂CF₃, - CH₂CF₂CHF₂, methoxyl, ethoxyl, methylthio, N-methylamino, N-ethylamino, cyclopropyl, cyclobutyl, cyclopentyl, or cyclohexyl, the phenyl, ethenyl, allyl, ethynyl, propargyl, 1-propynyl, methyl, ethyl, n-propyl, isopropyl, -CHF₂, -CHFCH₂F, -CF₂CHF₂, -CH₂CF₃, -CH₂CF₂CHF₂, methoxyl, ethoxyl, methylthio, N-methylamino, N-ethylamino, cyclopropyl, cyclobutyl, cyclopentyl, and cyclohexyl being independently and optionally substituted with 1, 2, or 3 substituents selected from D, F, Cl, Br, I, CN, hydroxyl, amino, nitro, methoxyl, ethoxyl, methylthio, N-methylamino, N-ethylamino, cyclopropyl, cyclobutyl, cyclopentyl, and cyclohexyl; and
R¹², R¹³, R¹⁴, and R¹⁵ are each independently H, methyl, ethyl, n-propyl, isopropyl, ethenyl, allyl, ethynyl, propargyl, 1-propynyl, cyclopropyl, cyclobutyl, cyclopentyl, cyclohexyl, oxacyclobutyl, azacyclobutyl, pyrrolidinyl, tetrahydrofuranyl, piperidyl, piperazinyl, or morpholinyl, the methyl, ethyl, n-propyl, isopropyl, ethenyl, allyl, ethynyl, propargyl, 1-propynyl, cyclopropyl, cyclobutyl, cyclopentyl, cyclohexyl, oxacyclobutyl, azacyclobutyl, pyrrolidinyl, tetrahydrofuranyl, piperidyl, piperazinyl, and morpholinyl being independently and optionally substituted with 1, 2, or 3 substituents selected from D, F, Cl, Br, I, CN, hydroxyl, amino, and nitro.

In some embodiments, the compound described in the present invention is a compound having any one of the following structures, or a stereoisomer, geometric isomer, tautomer, oxynitride, hydrate, solvate, metabolite, pharmaceutically acceptable salt, or prodrug of the compound having any one of the following structures: 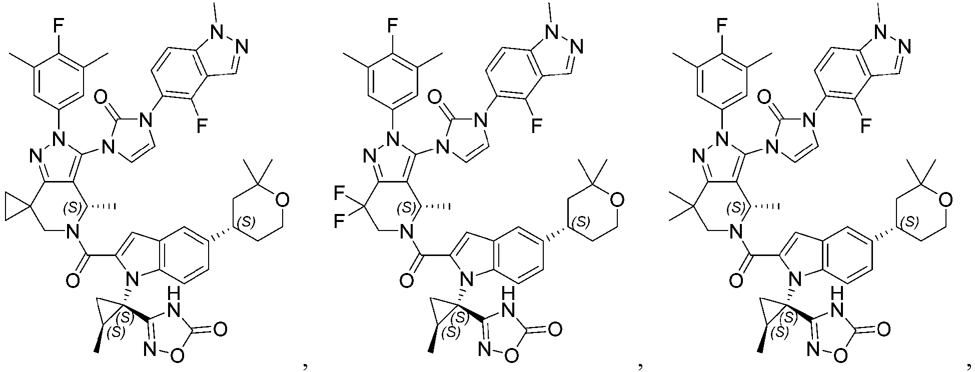 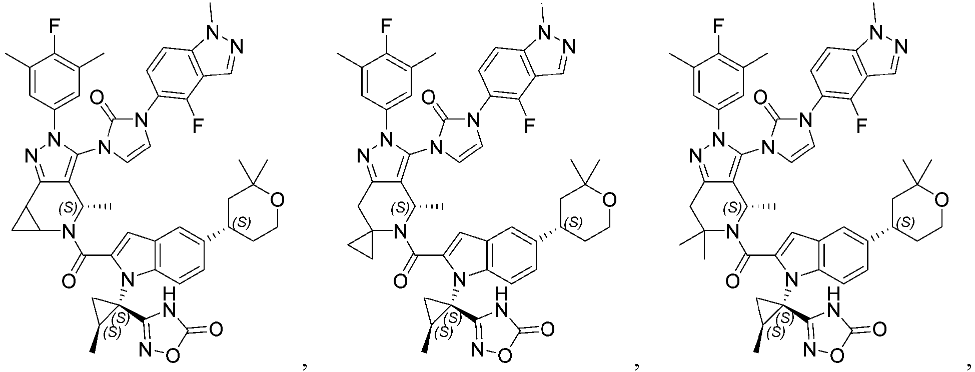 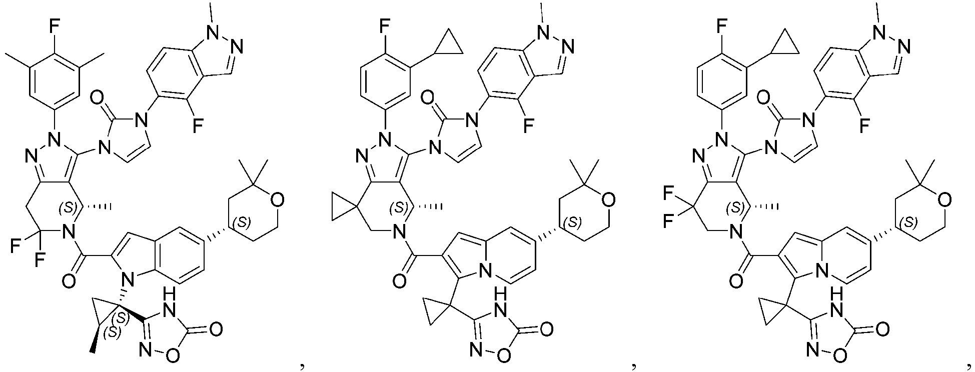 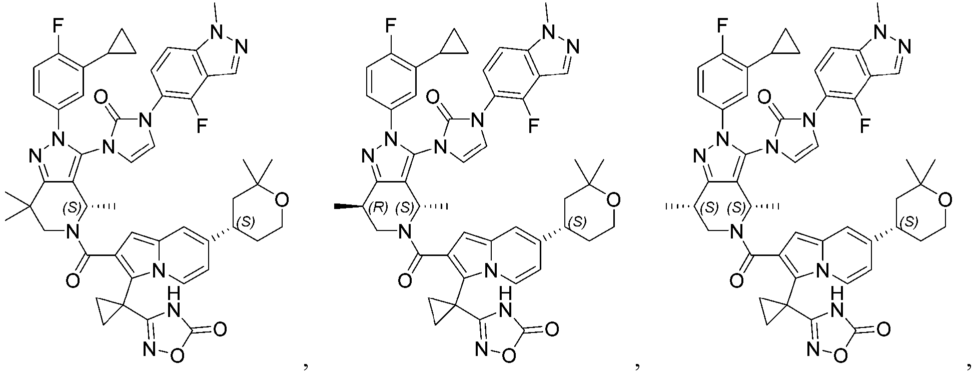 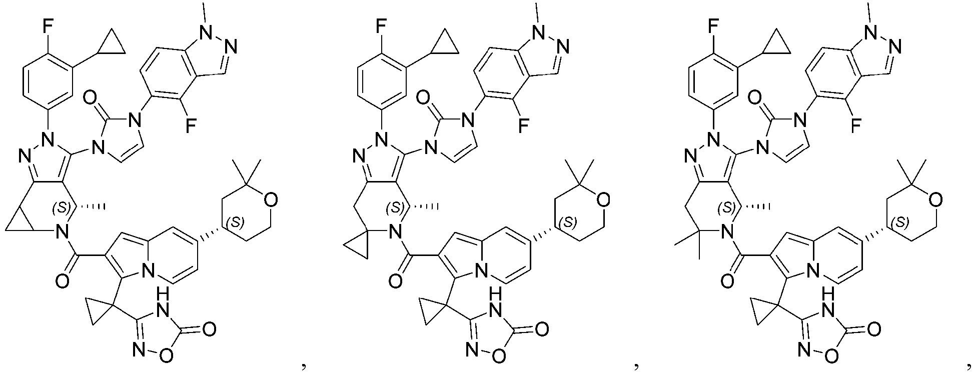 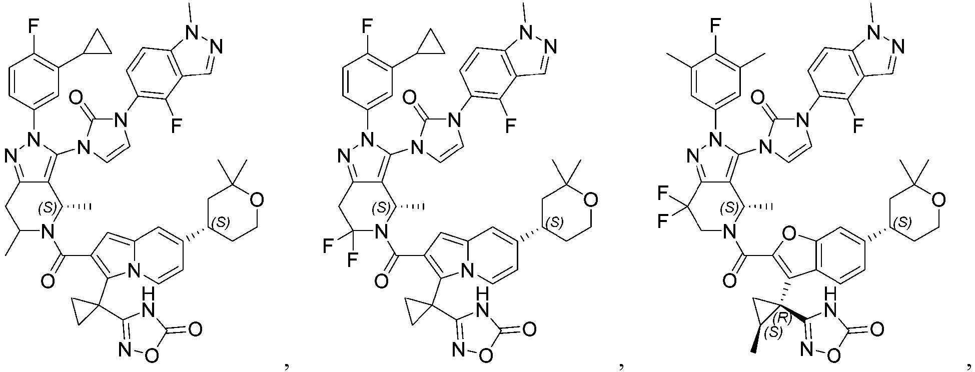 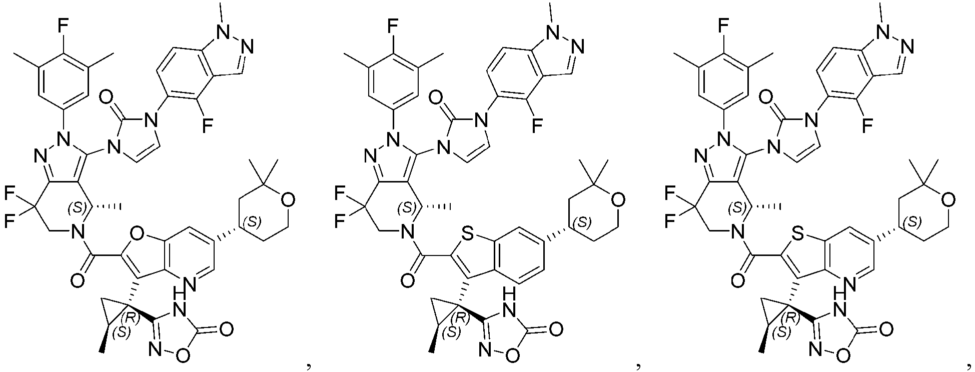 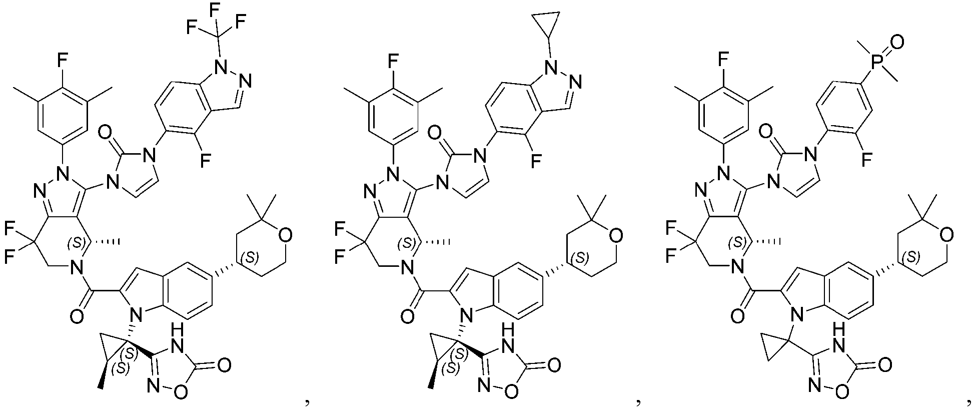 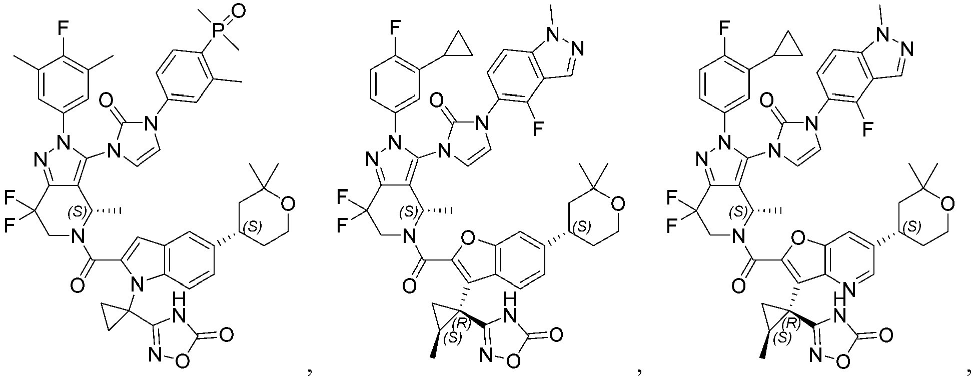 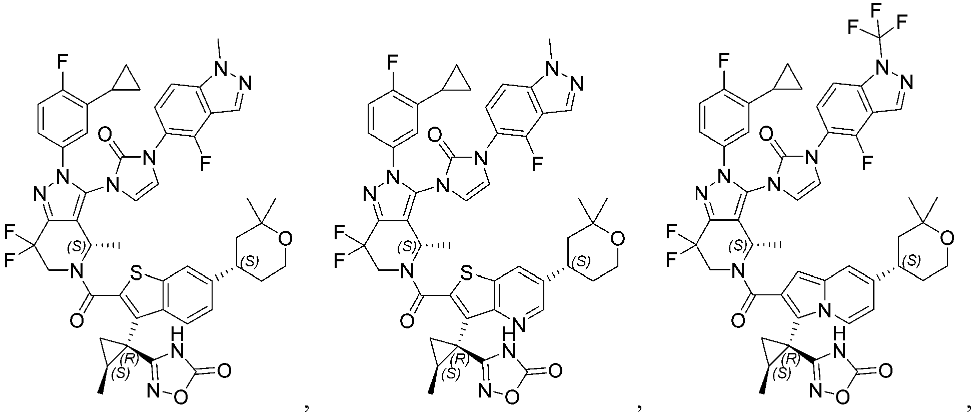 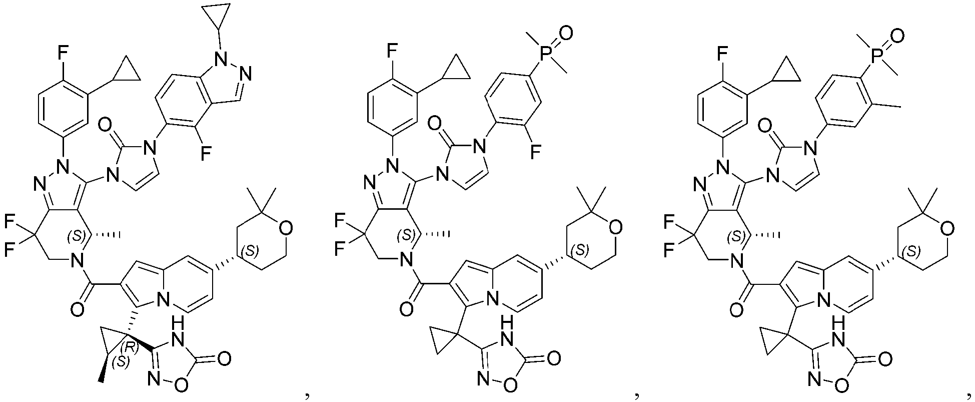 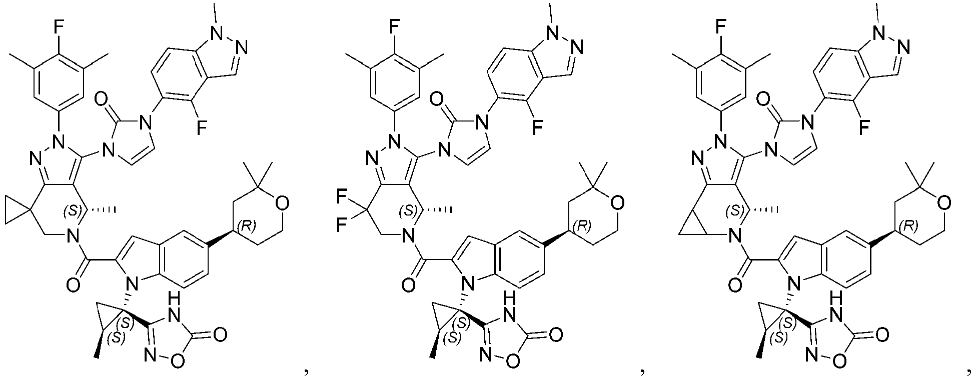 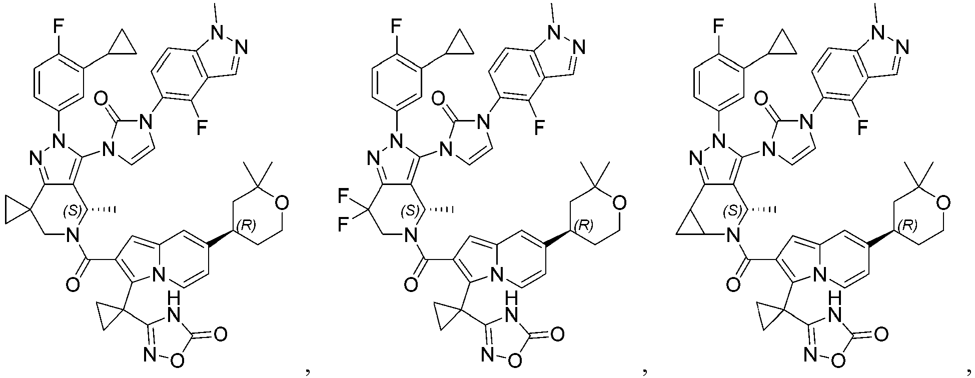 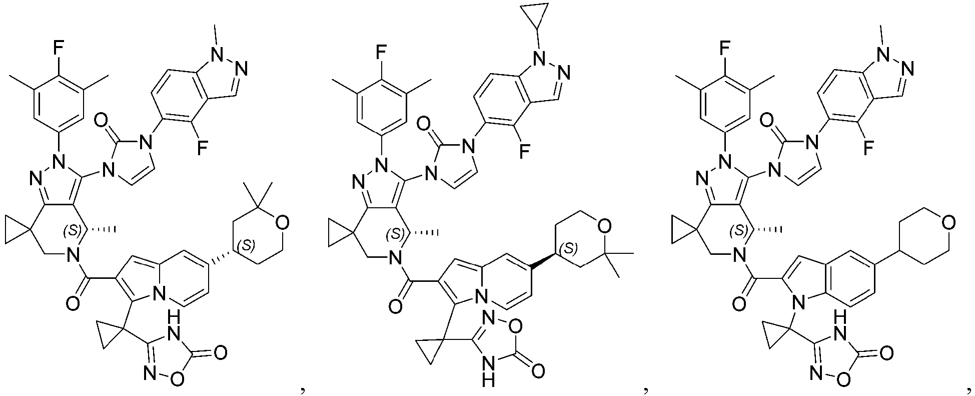 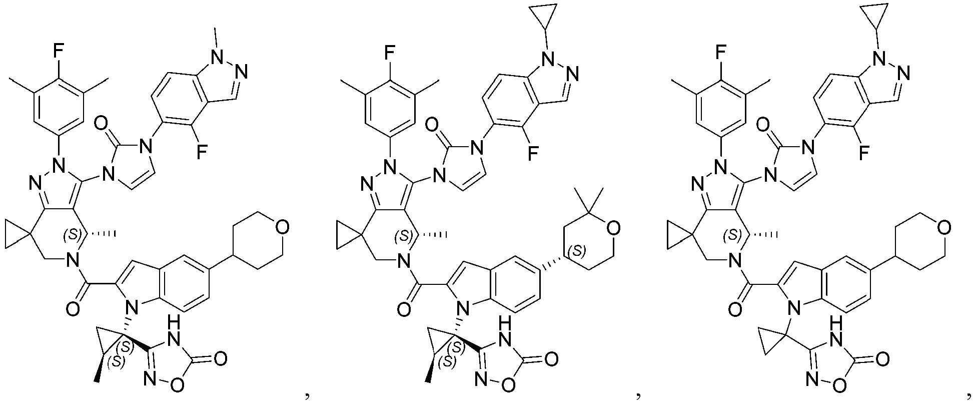 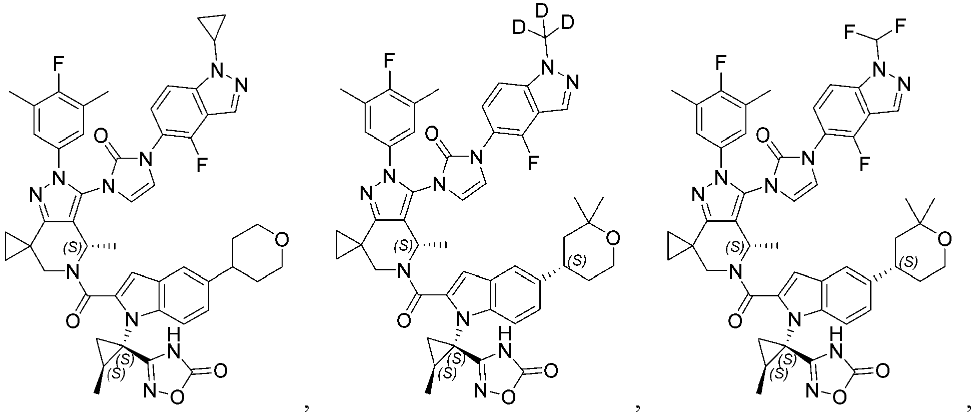 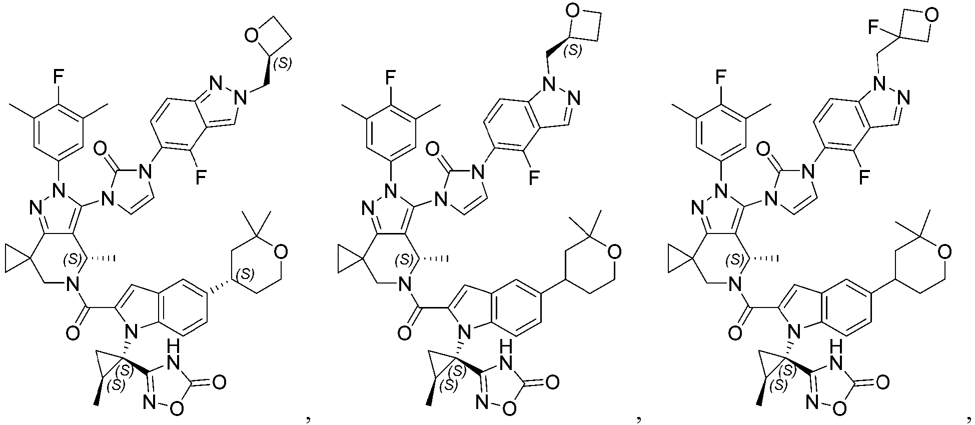 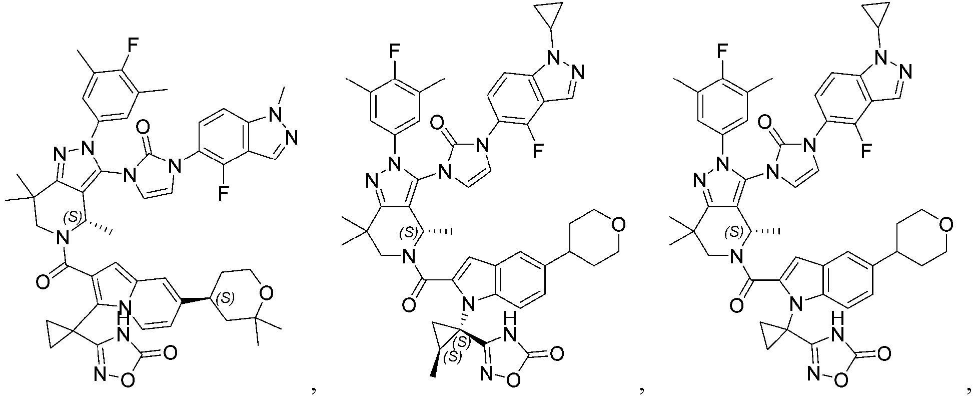 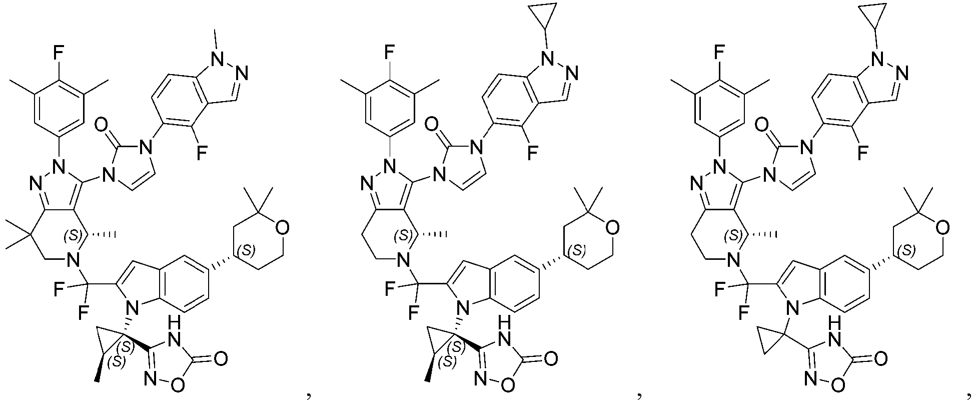 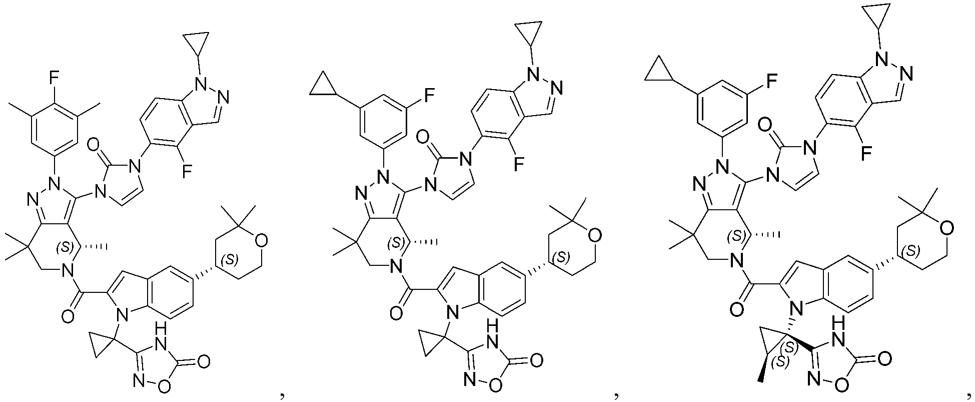 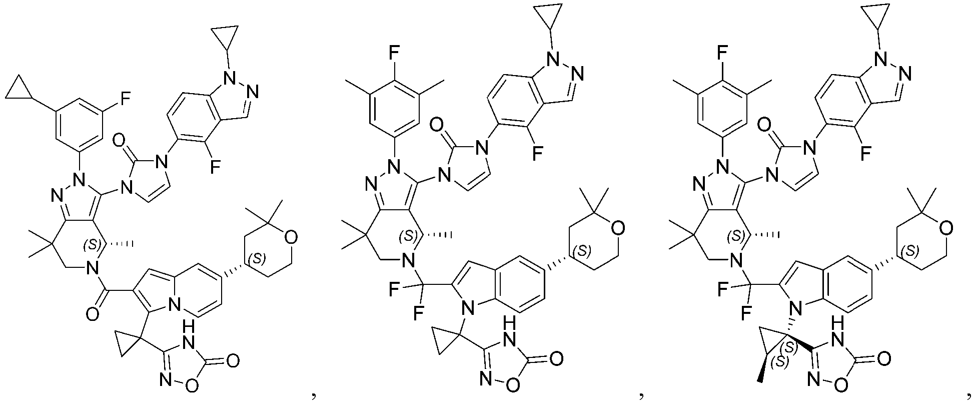 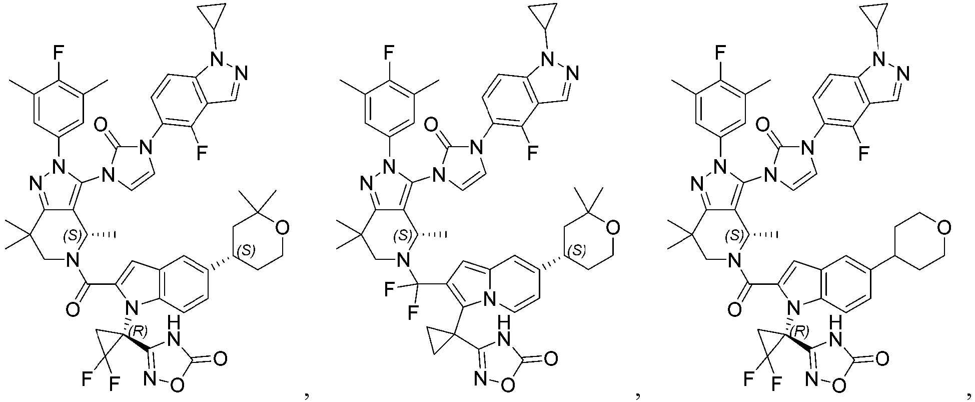 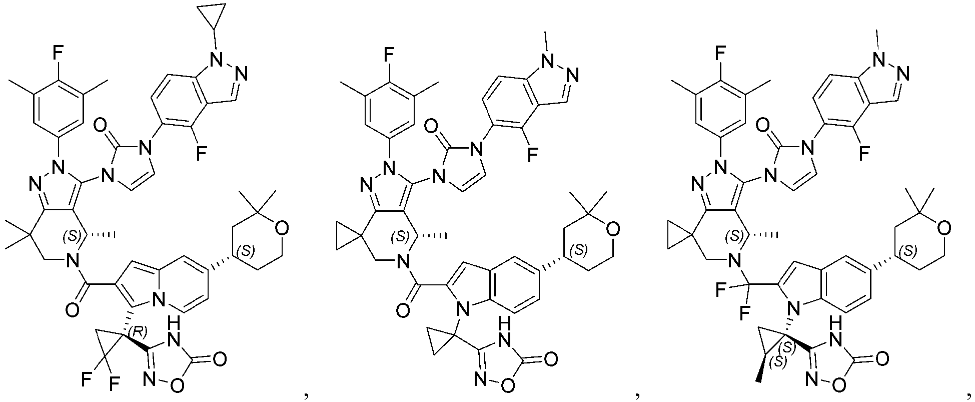 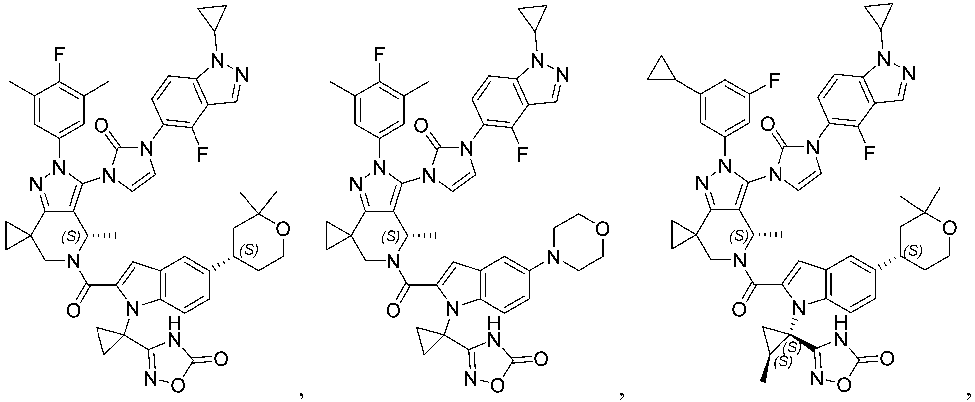 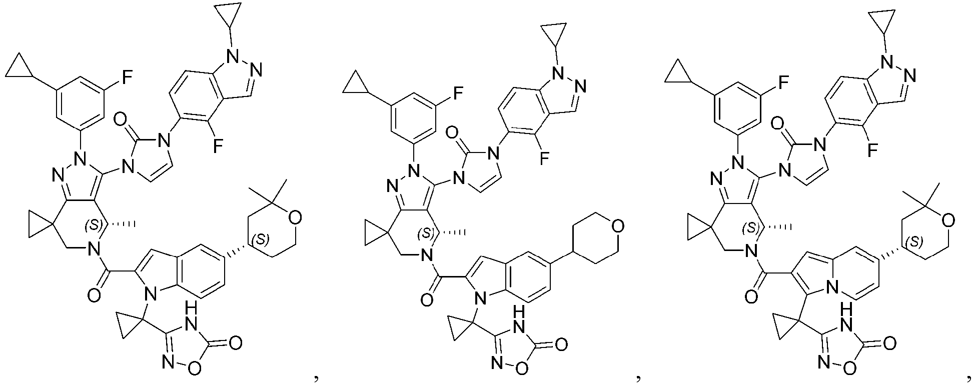 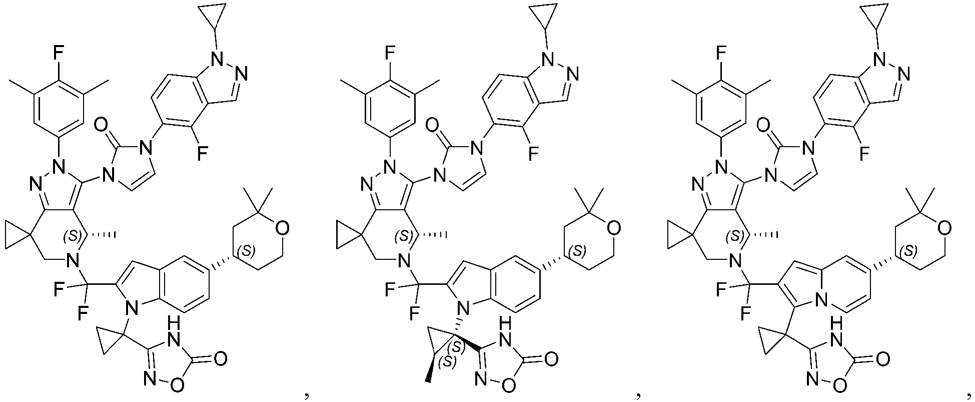 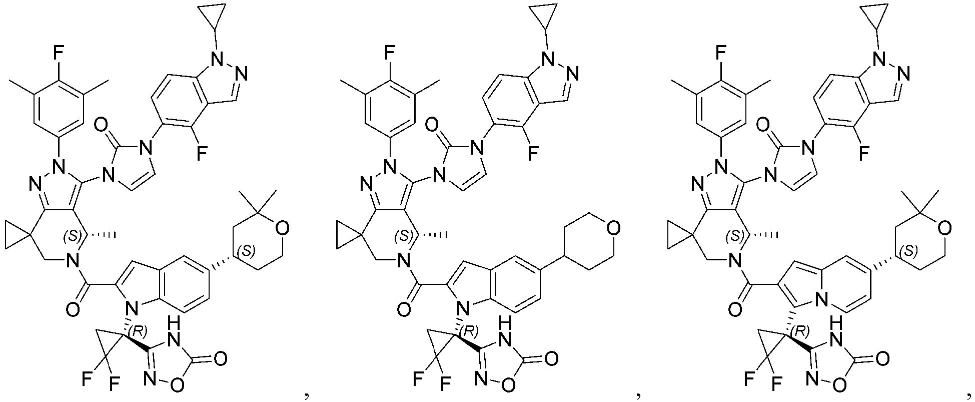 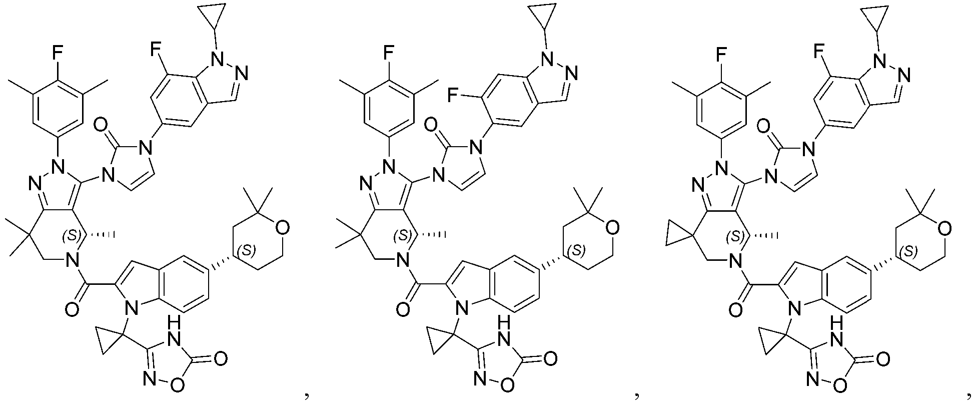 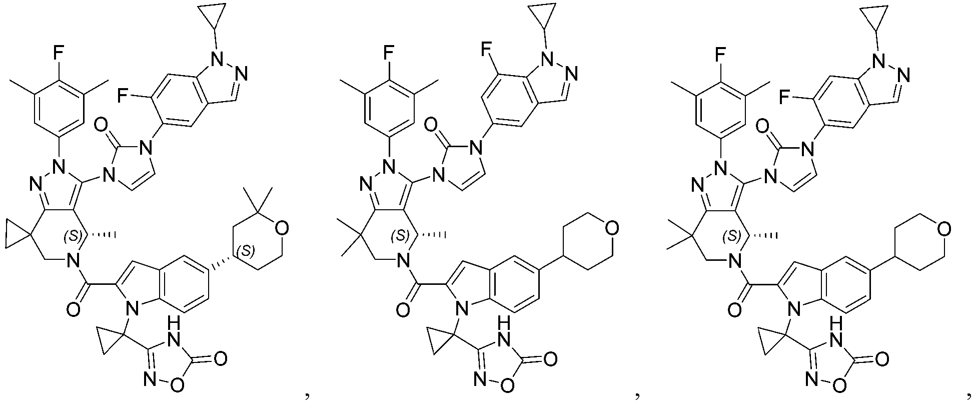 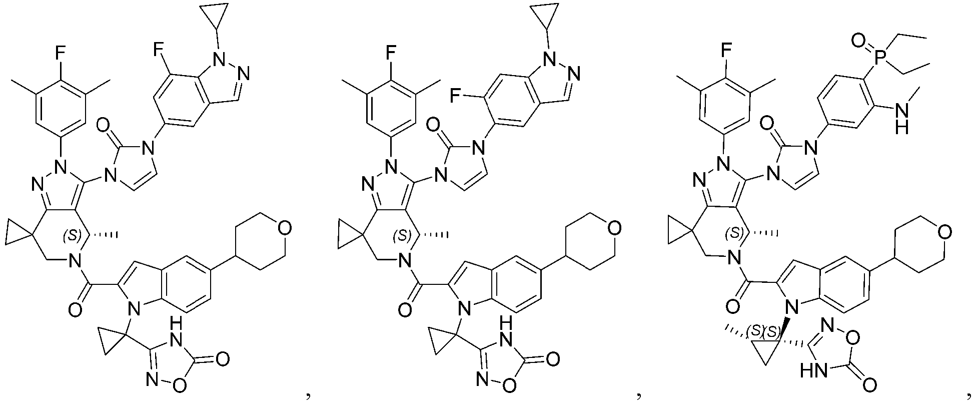 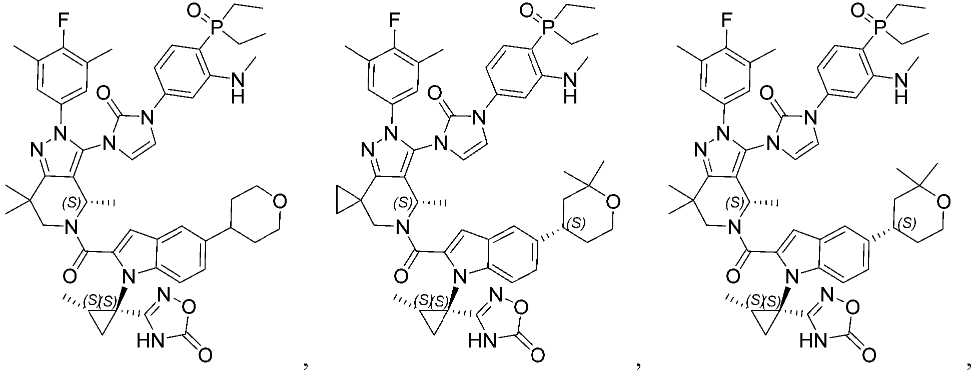 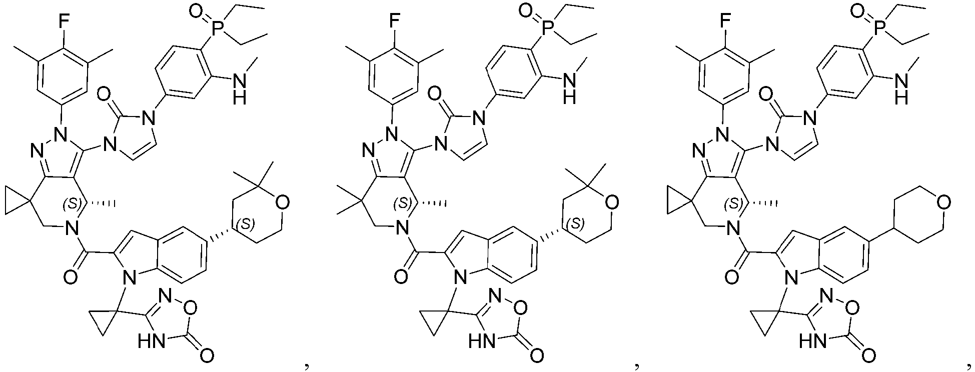 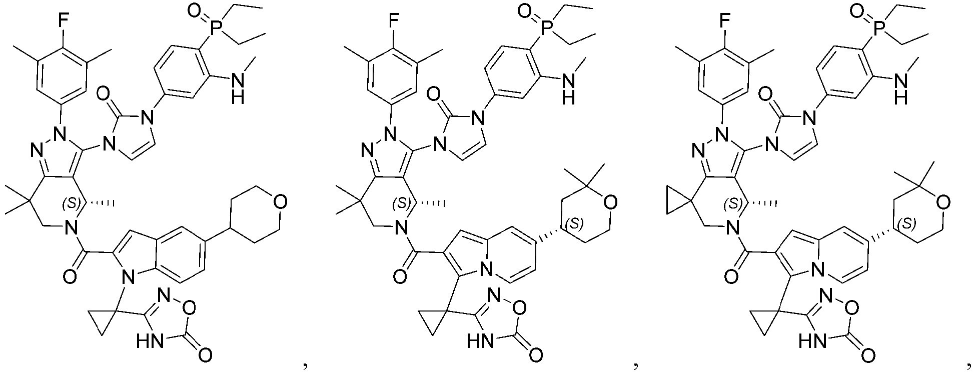 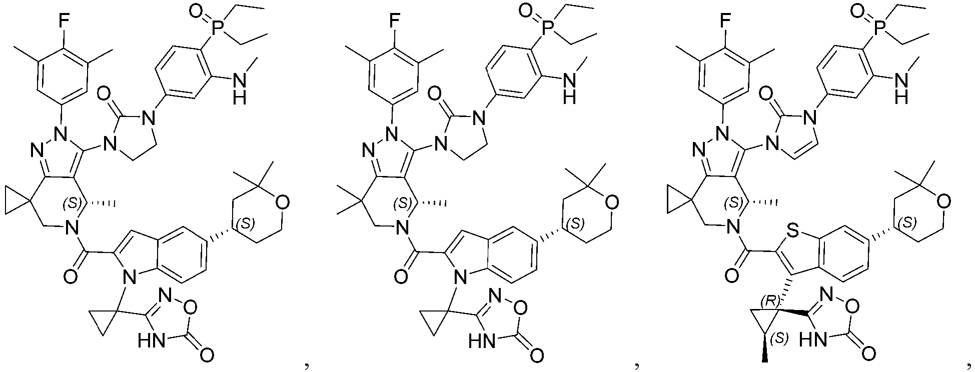 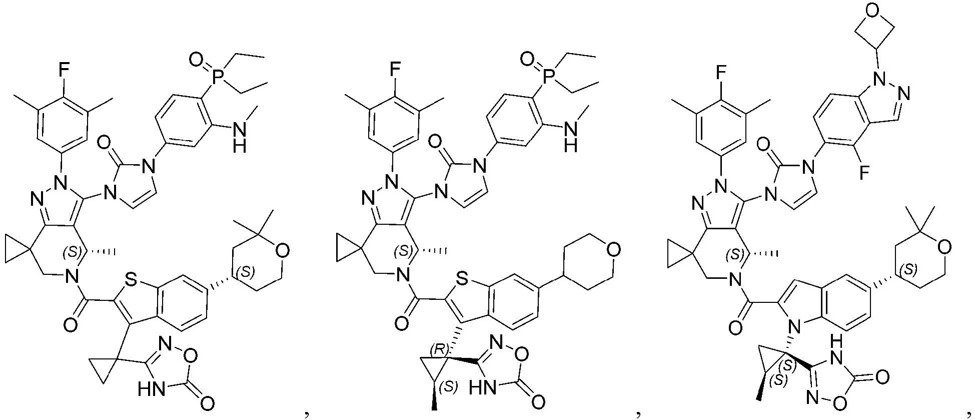 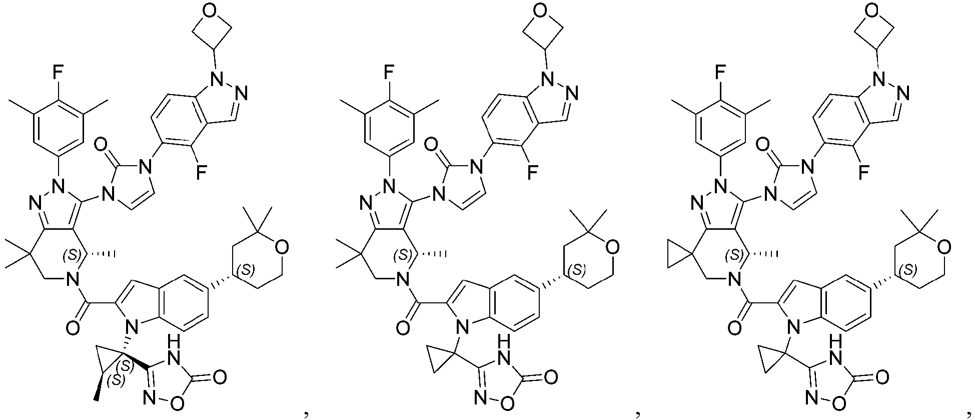 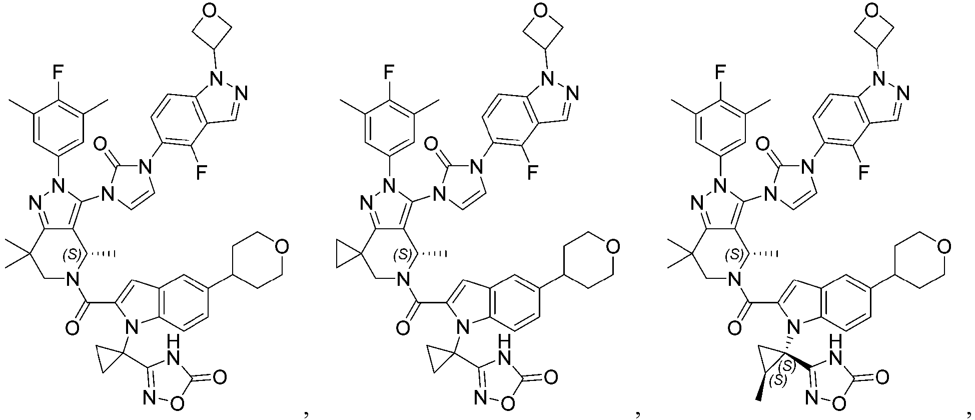 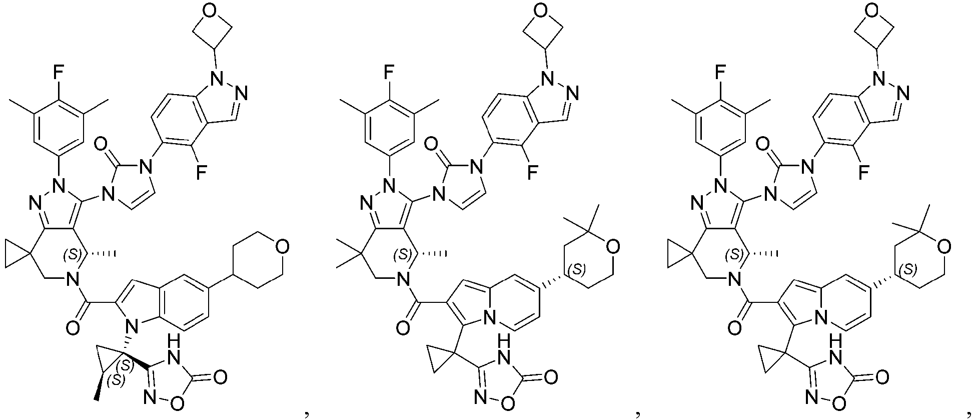 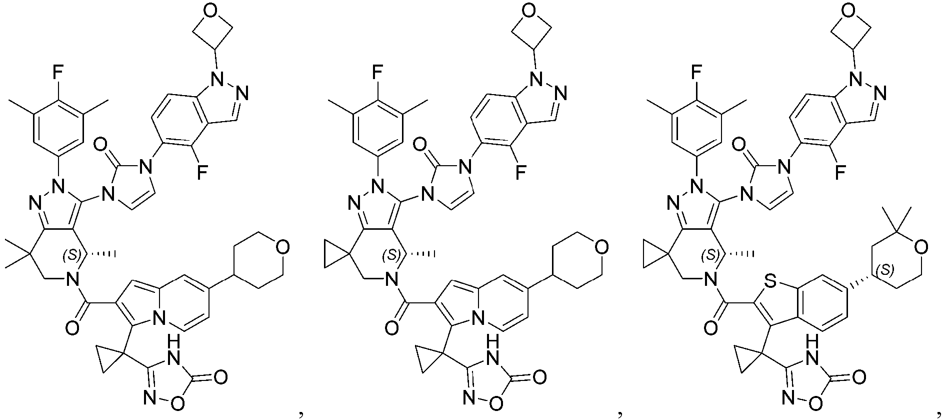 or

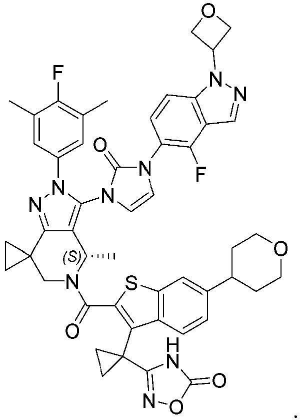

In one aspect, the present invention relates to a pharmaceutical composition, and the pharmaceutical composition includes the compound represented by formula (I) of the present invention, or a stereoisomer, geometric isomer, tautomer, oxynitride, hydrate, solvate, metabolite, pharmaceutically acceptable salt, or prodrug thereof, and a pharmaceutically acceptable carrier, excipient, diluent, adjuvant, vehicle, or combination thereof.

In one aspect, the present invention relates to use of the aforementioned compound or the pharmaceutical composition thereof in preparation of drugs, where the drugs are used for preventing, treating, or alleviating GLP-1 receptor agonist-mediated diseases in patients.

In some embodiments, the GLP-1 receptor agonist-mediated diseases as described in the present invention are diabetes, diabetes complications, non-alcoholic fatty liver disease, obesity, hypertension, hyperlipidemia, hypercholesterolemia, hypertriglyceridemia, atherosclerosis, stroke, coronary heart disease, myocardial infarction, congestive heart failure, arrhythmia, cerebral infarction, diabetic nephropathy, Parkinson's disease, Alzheimer's disease, or dementia.

In some embodiments, the diabetes as described in the present invention is type I diabetes, type II diabetes, gestational diabetes, idiopathic type I diabetes, early-onset type II diabetes, maturity onset diabetes of the young, youth-onset atypical diabetes, malnutrition-related diabetes, or latent autoimmune diabetes in adults.

In another aspect, the present invention relates to a method for preparing, isolating, and purifying the compound represented by formula (I).

The preceding content only outlines certain aspects of the present invention, but the present invention is not limited to the aspects. The content of these and other aspects will be described more specifically and comprehensively below.

### Definitions and general terms

The present invention will list in detail documents corresponding to the specific content determined, and examples will be accompanied by diagrams of structural and chemical formulas. The present invention is intended to encompass all options, variations, and equivalents that may be included in the prior art of invention as defined by the claims. Those skilled in the art may identify many methods and substances that are similar or equivalent to those described herein, and the methods and substances can be applied in practice of the present invention. The present invention is by no means limited to the description of the methods and substances. There are many documents and similar substances that differ from or conflict with the present invention application, including but not limited to definitions of terms, usage of terms, described technologies, or a scope controlled by the present invention application.

The present invention will apply the following definitions unless otherwise indicated. According to an objective of the present invention, chemical elements are defined as in the periodic table, CAS version, and Chemical Drug Manual, 75, thEd, 1994**.** In addition, general principles of organic chemistry can be found in "Organic Chemistry," Thomas Sorrell, University Science Books, Sausalito: 1999**,** and "March's Advanced Organic Chemistry," by Michael B. Smith and Jerry March, John Wiley&Sons, New York: 2007**.** Therefore, all content incorporates references.

The term "include" is an open-ended expression that includes the content specified in the present invention, but does not exclude content of other aspects.

A compound described herein can be optionally substituted with one or more substituents, for example, the compound of the general formula in the present invention, or special examples in the embodiments, subclasses, and the compounds included in the present invention. It should be understood that the term "optionally substituted" can be used interchangeably with the term "substituted or unsubstituted". Generally speaking, the term "optionally" refers to one or more hydrogen atoms in a given structure being substituted with a specific substituent, regardless of whether "optionally" comes before the term "substituted". Unless otherwise indicated, an optional substituent group may have one substituent for substitution at substitutable locations of a group. When more than one location in a given structural formula can be substituted with one or more substituents selected from a specific group, the substituents can be the same or different at each location. The substituents may include, but are not limited to, hydrogen, F, Cl, Br, I, nitro, cyano, oxo(=O), hydroxyl, alkyl, hydroxyalkyl, alkylamino, aminoalkyl, haloalkoxyl, cycloalkyl, amino, aryl, heterocyclyl, heteroaryl, alkenyl, alkynyl, cycloalkoxyl, alkoxyl, alkoxyalkyl, haloalkyl, - COOH, -alkylene-C(=O)O-alkyl, -alkylene-S(=O)₂-alkyl, -alkylene-S(=O)₂-amino, -S(=O)₂-alkyl, -S(=O)₂-amino, -S(=O)₂OH, -O-alkylene-C(=O)O-alkyl, -O-alkylene-S(=O)₂-alkyl, -O-alkyleneS(=O)₂-amino, -O-alkylene-S(=O)₂OH, -C(=O)NH₂, -C(=O)NH-alkyl, -C(=O)N(alkyl)-alkyl, - C(=O)NHS(=O)₂-alkyl, -C(=O)NHS(=O)₂-amino, -C(=O)NHS(=O)₂OH, -N(haloalkyl)-alkyl, - N(alkyl)-S(=O)₂-alkyl, -NHS(=O)₂-alkyl, -NHS(=O)₂-haloalkyl, -N(alkyl)S(=O)₂-haloalkyl, - N(alkyl)S(=O)₂-alkylamino, -NHC(=O)-alkyl, -NHC(=O)-haloalkyl, -N(alkyl)C(=O)-haloalkyl, - N(alkyl)C(=O)-alkylamino, -N(alkyl)C(=O)O-alkyl, -NHC(=O)O-alkyl, -NHC(=O)O-haloalkyl, - N(alkyl)C(=O)O-haloalkyl, -N(alkyl)C(=O)O-aminoalkyl, -NHC(=O)-NH₂, -NHC(=O)NH-(alkyl), -NHC(=O)NH(haloalkyl), -NHC(=O)N(alkyl)-alkyl, -OC(=O)-alkyl, -OC(=O)-amino, - OC(=O)-alkylamino, -OC(=O)-aminoalkyl, -OC(=O)-alkoxyl, -C(=O)N(alkyl)S(=O)₂-alkyl, - C(=O)N(alkyl)S(=O)₂-amino, -C(=O)NH-S(=O)₂OH, -C(=NH)NH₂, -C(=NH)NH-alkyl, - C(=NH)N(alkyl)-alkyl, -C(=N-alkyl)-NH₂, -C(=O)NH-alkylene-S(=O)₂OH, -C(=O)NHC(=O)OH, -C(=O)NHC(=O)O-alkyl, -C(=O)N(alkyl)C(=O)O-alkyl, -C(=O)NH-alkylene-C(=O)OH, - C(=O)NH-alkylene-C(=O)O-alkyl, and the like.

The term "alkyl" used in the present invention includes saturated linear or branched monovalent hydrocarbyl containing 1 to 20 carbon atoms, or 1 to 10 carbon atoms, or 1 to 6 carbon atoms, or 1 to 4 carbon atoms, or 1 to 3 carbon atoms, or 1 to 2 carbon atoms, where the alkyl can be independently and optionally substituted with one or more substituents as described in the present invention. Further examples of alkyl groups include, but are not limited to, methyl (Me, - CH₃), ethyl (Et, -CH₂CH₃), n-propyl (n-Pr, -CH₂CH₂CH₃), isopropyl (i-Pr, -CH(CH₃)₂), n-butyl (n-Bu, -CH₂CH₂CH₂CH₃), isobutyl (i-Bu, -CH₂CH(CH₃)₂), sec-butyl (s-Bu, -CH(CH₃)CH₂CH₃), tert-butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH (CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), n-heptyl, n-octyl, and the like. The term "alkyl" and the prefix "alkane" thereof are used here, both including saturated linear and branched carbon chains. The term "alkylene" is used here to refer to a saturated divalent hydrocarbyl obtained by removing two hydrogen atoms from a linear or branched saturated hydrocarbon, examples of which include, but are not limited to, methylene, ethylene, isopropylidene, and the like.

The term "alkylene" refers to a saturated divalent hydrocarbyl group obtained by removing two hydrogen atoms from a saturated linear or branched hydrocarbyl. Unless otherwise specified, an alkylene group contains 1 to 12 carbon atoms. In some embodiments, the alkylene group contains 1 to 6 carbon atoms; in some other embodiments, the alkylene group contains 1 to 4 carbon atoms; in still some embodiments, the alkylene group contains 1 to 3 carbon atoms; and in some further embodiments, the alkylene group contains 1 to 2 carbon atoms. Such examples include methylene (-CH₂-), ethylene (-CH₂CH₂-), and isopropylidene (-CH(CH₃)CH₂-), and the like.

The term "alkenyl" refers to a linear or branched monovalent hydrocarbyl containing 2 to 12 carbon atoms, or 2 to 8 carbon atoms, or 2 to 6 carbon atoms, or 2 to 4 carbon atoms, where at least one location is in an unsaturated state, i.e., one C-C is an sp² double bond. The alkenyl group may be independently and optionally substituted with one or more substituents as described in the present invention, including groups with "anti", "cis" or "E", and "Z" orientations. Specific examples of alkenyl include, but are not limited to, ethenyl (-CH=CH₂), allyl (-CH₂CH=CH₂), and the like.

The term "alkynyl" refers to a linear or branched monovalent hydrocarbyl containing 2 to 12 carbon atoms, or 2 to 8 carbon atoms, or 2 to 6 carbon atoms, or 2 to 4 carbon atoms, where at least one location is in an unsaturated state, i.e., one C-C is an sp triple bond. The alkynyl group may be independently and optionally substituted with one or more substituents as described in the present invention, and specific examples of alkynyl include, but are not limited to, ethynyl (-C≡CH), propargyl (-CH₂C≡CH), and the like.

The term "heteroatom" refers to one or more forms of O, S, N, P, and Si, including any oxidation state of C, N, S, and P; forms of primary, secondary, tertiary amine, and quaternary ammonium salts; or a form in which hydrogen on a nitrogen atom in a heterocyclic ring is substituted, for example, N (like N in 3,4-dihydro-2*H*-pyrrolyl), NH (like NH in pyrrolidinyl), or NR (like NR in N-substituted pyrrolidinyl); or a form in which -CH₂- in a heterocyclic ring is oxidized to form -C(=O)-.

The term "halogen" refers to F, Cl, Br, or I.

The term "deuterium" refers to heavy hydrogen, D.

The term "unsaturated" used in the present invention refers to partially containing one or more degrees of unsaturation.

The term "alkoxyl" used in the present invention relates to alkyl, as defined in the present invention, which is connected to other moieties of a compound molecule through an oxygen atom. In some examples, alkoxyl is C₁₋₄ alkoxyl. Such examples include, but are not limited to, methoxyl, ethoxyl, propoxyl, butoxyl, and the like. The alkoxyl may be independently unsubstituted or substituted with one or more substituents as described in the present invention.

The term "alkylthio" used in the present invention relates to alkyl, as defined in the present invention, which is connected to other moieties of a compound molecule through a sulfur atom. In some examples, alkylthio is C₁₋₄ alkylthio. Such examples include, but are not limited to, methylthio, ethylthio, propylthio, butylthio, and the like. The alkylthio may be independently unsubstituted or substituted with one or more substituents as described in the present invention.

The term "alkylamino" used in the present invention relates to alkyl, as defined in the present invention, which is connected to other moieties of a compound molecule through an N atom. In some examples, alkylamino is C₁₋₄ alkylamino. Such examples include, but are not limited to, methylamino, ethylamino, propylamino, butylamino, and the like. The alkylamino may be independently unsubstituted or substituted with one or more substituents as described in the present invention.

The term "cycloalkyl" or "cycloalkane" refers to a monovalent or multivalent, saturated monocyclic, bicyclic, or tricyclic carbon ring system containing 3 to 12 carbon atoms, which is a saturated ring or a ring containing one or more unsaturated bonds, but does not include aromatic rings. In one embodiment, the cycloalkyl contains 3 to 10 carbon atoms; in another embodiment, the cycloalkyl contains 3 to 8 carbon atoms; and in still another embodiment, the cycloalkyl contains 3 to 6 carbon atoms. Such examples include, but are not limited to, cyclopropyl, cyclobutyl, cyclopentyl, cyclohexyl, and the like. The cycloalkyl group may be independently unsubstituted or substituted with one or more substituents as described in the present invention.

The terms "heterocyclyl" and "heterocyclic ring" may be used interchangeably here, both referring to a saturated or partially unsaturated monocyclic, bicyclic, or tricyclic ring containing 3 to 12 ring atoms, excluding aromatic rings, where at least one ring atom is a heteroatom. In one embodiment, the "heterocyclyl" or "heterocyclic ring" contains 3 to 10 ring atoms; in one embodiment, the "heterocyclyl" or "heterocyclic ring" contains 3 to 8 ring atoms; in another embodiment, the "heterocyclyl" or "heterocyclic ring" contains 5 to 8 ring atoms; in still another embodiment, the "heterocyclyl" or "heterocyclic ring" contains 3 to 6 ring atoms; in one further embodiment, the "heterocyclyl" or "heterocyclic ring" contains 5 to 6 ring atoms; and in yet another embodiment, the "heterocyclyl" or "heterocyclic ring" contains 4 to 6 ring atoms. Unless otherwise specified, the heterocyclyl may be a carbon group or nitrogen group, and the heteroatom has the meaning as described in the present invention. Examples of the heterocyclyl include, but are not limited to: oxiranyl, azacyclobutyl, oxacyclobutyl, thiocyclobutyl, pyrrolidinyl, 2-pyrrolinyl, 3-pyrrolinyl, pyrazolinyl, pyrazolidyl, imidazolinyl, imidazolidinyl, tetrahydrofuranyl, dihydrofuranyl, tetrahydrothiophenyl, dihydrothiophenyl, 1,3-dioxolanyl, dithiolanyl, tetrahydropyranyl, dihydropyranyl, 2H-pyranyl, 4H-pyranyl, tetrahydrothiopyranyl, piperidyl, morpholinyl, thiomorpholinyl, piperazinyl, dioxanyl, dithianyl, thioxanyl, homopiperazinyl, homopiperidinyl, oxepanyl, thiepanyl, oxazepinyl, diazepinyl, thiazepinyl, and 2-oxa-5-azabicyclo[2.2.1]hept-5-yl. Examples of heterocyclyl in which a -CH₂- group is substituted with - C(=O)- include, but are not limited to: 2-oxopyrrolidinyl, oxo-1,3-thiazolidinyl, 2-piperidinonyl, 3,5-dioxopiperidinyl, and pyrimidinedionyl. Examples of heterocyclyl in which a sulfur atom is oxidized include, but are not limited to, sulfolanyl and 1,1-dioxothiomorpholinyl. The heterocyclyl group may be optionally substituted with one or more substituents as described in the present invention.

The term "aryl" refers to a monocyclic, bicyclic, or tricyclic carbon ring system containing 6 to 14 ring atoms, or 6 to 12 ring atoms, or 6 to 10 ring atoms, where at least one ring is aromatic and each ring contains a ring of 3 to 7 atoms, with one or more attachment points being connected to other moieties of a molecule. The term "aryl" may be used interchangeably with the term "aromatic ring". Examples of an aryl group may include phenyl, naphthyl, and anthryl. The aryl group may be independently and optionally substituted with one or more substituents as described in the present invention.

The term "heteroaryl" refers to a monocyclic, bicyclic, and tricyclic system containing 5 to 12 ring atoms, or 5 to 10 ring atoms, or 5 to 6 ring atoms, where at least one ring system is an aromatic ring, at least one ring system contains one or more heteroatoms, and each ring contains a ring containing 5 to 7 atoms, with one or more attachment points being connected to other moieties of a molecule. The term "heteroaryl" may be used interchangeably with the term "heteroaromatic ring" or "heteroaromatics". The heteroaryl group is optionally substituted with one or more substituents as described in the present invention. In one embodiment, heteroaryl containing 5 to 10 atoms contains 1, 2, 3, or 4 heteroatoms independently selected from O, S, and N, where the nitrogen atom may be further oxidized.

Examples of the heteroaryl group include, but are not limited to: furyl, imidazolyl (e.g., *N-*imidazolyl, 2-imidazolyl, 4-imidazolyl, and 5-imidazolyl), isoxazolyl, oxazolyl (e.g., 2-oxazolyl, 4-oxazolyl, and 5-oxazolyl), pyrrolyl (e.g., N-pyrrolyl, 2-pyrrolyl, and 3-pyrrolyl), pyridyl, pyrimidinyl (e.g., 2-pyrimidinyl, 4-pyrimidinyl, and 5-pyrimidinyl), pyridazinyl, thiazolyl (e.g., 2-thiazolyl, 4-thiazolyl, and 5-thiazolyl), tetrazyl (e.g., 5-tetrazyl), triazolyl, thienyl (e.g., 2-thienyl and 3-thienyl), pyrazolyl, isothiazolyl, 1,2,3-oxadiazolyl, 1,2,5-oxadiazolyl, 1,2,4-oxadiazolyl, 1,2,3-triazolyl, 1,2,3-thiodiazolyl, 1,3,4-thiodiazolyl, 1,2,5-thiodiazolyl, pyrazinyl, and 1,3,5-triazinyl; and also include, but are not limited to, the following bicyclic groups: benzimidazolyl, benzofuryl, benzothiophenyl, indolyl (e.g., 2-indolyl), purinyl, quinolyl (e.g., 2-quinolyl, 3-quinolyl, and 4-quinolyl), 1,2,3,4-tetrahydroisoquinolyl, 1,3-benzodioxolyl, indolinyl, isoquinolyl (e.g., 1-isoquinolyl, 3-isoquinolyl, or 4-isoquinolyl), imidazo[1,2*-a*]pyridyl, pyrazolo[1,5-*a*]pyridyl, pyrazolo[1,5-*a*]pyrimidinyl, imidazo[1,2-b]pyridazinyl, [1,2,4]triazolo[4,3-b]pyridazinyl, [1,2,4]triazolo[1,5-*a*]pyrimidinyl, [1,2,4]triazolo[1,5-*a*]pyridyl, and the like.

The term "haloalkyl" or "haloalkoxyl" refers to an alkyl or alkoxyl group being substituted with one or more halogen atoms, examples of which include, but are not limited to, trifluoromethyl, trifluoromethoxyl, and the like.

The term "hydroxyalkyl" refers to an alkyl group being substituted with one or more hydroxyl groups, examples of which include, but are not limited to, hydroxymethyl, hydroxyethyl, and the like.

The term "aminoalkyl" refers to an alkyl group being substituted with one or more amino groups, examples of which include, but are not limited to, aminomethyl, aminoethyl, and the like.

As described in the present invention, a ring system formed by connecting a substituent to a ring through a bond represents that the substituent may be substituted at any substitutable location on the ring. For example, formula (a) represents that a substituent R may be mono- or polysubstituted at any possible substitutable location on a pyridine ring.

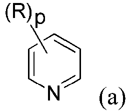

As described in the present invention, a ring system formed by connecting a connecting bond to a ring (as shown in formula b) represents that the connecting bond may be connected to other moieties of a molecule at any connectable location on the ring system. Formula b represents that any possible connectable location on an octahydrocyclopententa[c]pyrrole ring may be connected to other moieties of a molecule.

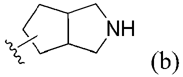

As described in the present invention, two connecting sites on a group "-O-(CR^{m}Rⁿ)ₚ-" may be connected to other moieties of a molecule, and the connection modes of the two connecting sites can be interchanged. For example, formula f represents that the group -O-(CR^{m}Rⁿ)ₚ- may be connected to other moieties of a molecule through an E end or E' end. For example, E-O-(CR^{m}Rⁿ)ₚ-E' or E'-O-(CR^{m}Rⁿ)ₚ-E.

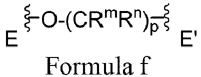

Furthermore, it should be noted that unless otherwise explicitly stated, the descriptions used throughout this specification of "each ... and ... are independently", "... and ... are each independently", and "... and ... are respectively and independently" are interchangeable and should be broadly understood. The descriptions may refer to that specific options expressed between the same symbols in different groups do not affect each other, or specific options expressed between the same symbols in the same group do not affect each other.

Unless otherwise indicated, structural formulas described in the present invention include all isomeric forms (e.g., enantiomeric isomerism, diastereoisomerism, geometric isomerism, or conformational isomerism): for example, R and S configurations containing asymmetric centers, (Z) and (*E*) isomers with double bonds, and (Z) and (E) conformational isomers. Therefore, an individual stereochemical isomer or a mixture of an enantiomer, a diastereomer, a geometric isomer, or a conformational isomer, of the compound of the present invention, is within the scope of the present invention.

Unless otherwise indicated, the structural formulas and compounds described in the present invention include all isomeric forms (e.g., enantiomeric isomerism, diastereoisomerism, geometric isomerism, or conformational isomerism), oxynitride, hydrate, solvate, metabolite, pharmaceutically acceptable salts, and prodrugs. Therefore, an individual stereochemical isomer, enantiomer, diastereomer, geometric isomer, conformational isomer, oxynitride, hydrate, solvate, metabolite, pharmaceutically acceptable salt, or prodrug, of the compound of the present invention, is also within the scope of the present invention. In addition, unless otherwise indicated, a structural formula of the compound described in the present invention includes enriched isotopes of one or more different atoms.

The "metabolite" refers to a product obtained through a metabolic process in vivo of the specific compound described in the present invention or a pharmaceutically acceptable salt, analogue, or derivative thereof, and the product exhibits activity in vivo or in vitro similar to that of the compound of formula (I). A metabolite of a compound may be identified by techniques known in the related art, and the activity thereof may be characterized by experimental methods as described in the present invention. Such a product may be obtained by methods such as oxidation, reduction, hydrolysis, acylation, deamidation, esterification, defatting, enzymatic hydrolysis, or the like performed on an administration compound. Correspondingly, the present invention includes metabolites of the compound, including a metabolite produced from the compound of the present invention being in full contact with a mammal for a period of time.

Definitions and customary use of stereochemistry in the present invention generally refer to the following document: S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley&Sons, Inc., New York, 1994. The compound of the present invention may contain an asymmetric center or chiral center, resulting in different stereoisomers. All stereoisomeric forms of the compound of the present invention, including but not limited to diastereomers, enantiomers, atropisomers, and mixtures thereof, such as a racemic mixture, constitute a part of the present invention. Many organic compounds exist in an optically active form, which means that the organic compounds have an ability to rotate a plane of plane-polarized light. When an optically active compound is described, prefixes D, L or R, S are used for representing an absolute configuration of a chiral center of a molecule. Prefixes d, l or (+), (-) are used for naming a symbol for rotation of plane-polarized light in a compound. (-) or l refers to the compound being levorotatory, while the prefix (+) or d refers to the compound being dextrorotatory. Chemical structures of the stereoisomers are the same, but stereostructures thereof are different. Specific stereoisomers may be enantiomers, and a mixture of isomers are commonly referred to as an enantiomer mixture. A 50:50 enantiomeric mixture is referred to as a racemic mixture or racemate, which may result in a lack of stereoselectivity or stereospecificity during chemical reactions. The terms "racemic mixture" and "racemate" refer to a mixture of two enantiomers with equimolar amounts, which lacks optical activity.

The term "tautomer" or "tautomeric form" refers to isomers of structures with different energies, which may be converted into each other through low energy barriers. For example, proton tautomers (i.e. prototropic tautomers) includes tautomerism through proton transfer, such as keto-enol and imine-enamine isomerization. Atomic valence (valence) tautomers include tautomerism of recombined bonding electrons.

The "pharmaceutically acceptable salt" used in the present invention refers to organic salts and inorganic salts of the compound of the present invention. Pharmaceutically acceptable salts are well-known in the related art, as documented in S. M. Berge et al., describe pharmaceutically acceptable salts in detail in J. Pharmaceutical Sciences, 66: 1-19, 1977. Pharmaceutically acceptable salts formed from non-toxic acids include, but are not limited to: inorganic acid salts formed by reaction with amino groups, such as hydrochloride, hydrobromide, phosphate, sulfate, and perchlorate; organic acid salts, such as acetate, oxalate, malate, tartrate, citrate, succinate, and malonate; or salts obtained by other methods recorded in books and documents, such as ion exchange. Other pharmaceutically acceptable salts include adipate, malate, 2-hydroxypropionate, alginate, ascorbate, aspartate, benzenesulfonate, benzoate, bisulfate, borate, butyrate, camphorate, camphorsulfonate, cyclopentyl propionate, digluconate, dodecyl sulfate, ethanesulfonate, formate, fumarate, gluceptate, glycerophosphate, gluconate, hemisulfate, heptanate, hexanoate, hydroiodate, 2-hydroxyl-ethanesulfonate, lactobionate, lactate, laurate, lauryl sulfate, malate, mesylate, 2-naphthalene sulfonate, nicotinate, nitrate, oleate, palmitate, pamoate, pectate, persulfate, 3-phenylpropionate, picrate, pivalate, propionate, stearate, rhodanate, p-toluenesulfonate, undecanoate, valerate, and the like. Salts obtained through appropriate bases include alkali metal, alkaline earth metal, ammonium, and N+(C₁₋₄ alkyl)₄. The present invention also envisions any quaternary ammonium salts formed from compounds containing groups of N. Water soluble, oil soluble, or dispersed products may be obtained through quaternization. Alkali metals or alkaline earth metals that can form salts include sodium, lithium, potassium, calcium, magnesium, and the like. Pharmaceutically acceptable salts further include appropriate, non-toxic ammonium salts, quaternary ammonium salts, and amine cations formed from counterions, such as halide, hydroxide, carboxylate, sulfate, phosphate, nitrate, C₁₋₈ sulfonate, and aromatic sulfonate.

The "hydrate" in the present invention refers to a complex formed with solvent molecules being water.

The "solvate" in the present invention refers to a complex formed from one or more solvent molecules with the compound of the present invention. Solvents that form the solvates include, but are not limited to: water, isopropanol, ethanol, methanol, dimethyl sulfoxide, ethyl acetate, acetic acid, and aminoethanol.

The "ester" in the present invention refers to an ester that can be hydrolyzed in vivo, which is formed from the compound of formula (I) containing hydroxyl. Such esters are, for example, pharmaceutically acceptable esters that can be hydrolyzed in humans or animals to produce a parent alcohol. The ester groups that can be hydrolyzed in vivo, which is formed from the compound of formula (I) containing hydroxyl, include, but are not limited to: phospho, acetoxymethoxyl, 2,2-dimethylpropanoxymethoxyl, alkanoyl, benzoyl, phenylacetyl, alkoxycarbonyl, dialkylaminoformyl, N-(dialkylaminoethyl)-N-alkylaminoformyl, and the like.

The "oxynitride" in the present invention refers to N-oxides formed by oxidizing one or more nitrogen atoms when a compound contains several amine functional groups. Special examples of N-oxides are N-oxides of tertiary amines or *N*-oxides of nitrogen-containing heterocyclic nitrogen atoms. Corresponding amine may be treated with an oxidant such as hydrogen peroxide or peracid (e.g., peroxycarboxylic acid) to form N-oxide (refer to Advanced Organic Chemistry, Wiley Interscience, 4th edition, Jerry March, pages). Especially, N-oxides may be prepared using L. W. Deady's method (Syn. Comm. 1977, 7, 509-514), for example, by reacting an amine compound with m-chloro-perbenzoic acid (MCPBA) in an inert solvent (e.g., dichloromethane).

The term "prodrug" used in the present invention refers to a compound that is converted in vivo into the compound of formula (I). Such conversion is affected by hydrolysis of the prodrug in the blood or enzymatic conversion of the prodrug into a parent structure in the blood or tissues. The prodrug compounds of the present invention may be esters, and esters in existing inventions that can serve as prodrugs include phenyl esters, aliphatic (C₁₋₂₄) esters, acyloxymethyl esters, carbonate, carbamates, and amino acid esters. For example, a compound in the present invention contains hydroxyl, which may be acylated to obtain the prodrug form of the compound. Other prodrug forms include phosphate, for example, the phosphate compounds are obtained through phosphorylation of hydroxyl on a parent. For a comprehensive discussion on prodrugs, refer to the following documents: T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al, Prodrugs: Design and Clinical Applications, Nature Review Drug Discovery, 2008, 7, 255-270, and S. J. Hecker et al, Prodrugs of Phosphates and Phosphonates, Journal of Medicinal Chemistry, 2008, 51, 2328-2345.

Unless otherwise stated herein or the context clearly indicates the opposite, the terms "a", "one", "the" and similar terms used in the context of the present invention (especially in the context of the claims) may be interpreted as including both singular and plural.

The term "GLP-1 receptor agonist" used herein refers to a substance that can activate activity of a GLP-1 receptor.

### General synthesis process

The following examples are listed for describing the present invention. However, it should be understood that the present invention is not limited to these examples, which only provide methods for practicing the present invention.

Generally, the compound of the present invention can be prepared by the method described in the present invention, unless further specified, where the definition of substituents is as described in the present invention. The following reaction solutions and examples are used for further exemplifying the content of the present invention.

Those skilled in the art will recognize that chemical reactions described in the present invention may be appropriately used for preparing other compounds of the present invention, and other methods for preparing the compound of the present invention are considered to be within the scope of the present invention. For example, the synthesis of non-illustrative compounds according to the present invention can be successfully accomplished by those skilled in the art using modification methods, such as appropriate protection of interfering groups, using other known reagents in addition to those described in the present invention, or making some conventional modifications to reaction conditions. In addition, reactions disclosed in the present invention or known reaction conditions are also generally acknowledged to be applicable for preparation of other compounds of the present invention.

In the examples described below, unless otherwise indicated, all temperatures are in degrees Celsius. The reagents are purchased from commodity suppliers such as Anhui Zesheng Technology Co., Ltd., Shanghai Shaoyuan Reagent Co., Ltd., Meryer (Shanghai) Chemical Technology Co., Ltd., and Shanghai Macklin Biochemical Co., Ltd, and are not further purified during use. Unless otherwise indicated, general reagents are purchased from Shantou Xilong Chemical Factory, Guangdong Guanghua Chemical Reagent Factory, Guangzhou Chemical Reagent Factory, Tianjin Damao Chemical Reagent Factory, Yantai Jiangyou Silicone Development Co., Ltd., and Qingdao Marine Chemical Factory.

Anhydrous tetrahydrofuran, N,N-dimethylformamide, 1,4-dioxane, and acetonitrile are obtained by drying over molecular sieves. Dichloromethane, ethyl acetate, petroleum ether, 1,2-dichloroethane, and methanol are analytically pure.

The following reactions are generally carried out under positive pressure of nitrogen or argon, or by placing a drying tube over an anhydrous solvent (unless otherwise indicated). Reaction flasks are plugged with suitable rubber stoppers, and a substrate is injected through a syringe. Glassware is dried.

A silica gel column is a Flash silica gel column purchased from Tianjin Bonna-Agela Technology Co., Ltd. Silica gel (300-400 mesh) is purchased from Qingdao Marine Chemical Factory.

A 1H NMR spectrum is recorded using a Bruker 500 MHz nuclear magnetic resonance spectrometer. The 1H NMR spectrum is obtained using CDCl₃, DMSO-*d*₆, CD₃OD, or acetone-*d*₆ as a solvent (in ppm), with TMS (0 ppm) or chloroform (7.26 ppm) as a reference standard. When multiplets appear, the following abbreviations are used: s (singlet), d (doublet), t (triplet), q (quartet), m (multiplet), br (broadened), brs (broadened singlet), dd (doublet of doublets), and dt (doublet of triplets). A coupling constant *J* is expressed in Hertz (Hz).

Conditions for determining low resolution mass spectrometry (MS) data are: Agilent G6125C quadrupole HPLC-MS (chromatographic column model: XBridge BEH C18, 4.6 x 50 mm, 2.5 microns, 6 min, flow rate of 1 mL/min). Mobile phase: the ratio of 0% to 95% (CH₃CN) in (H₂O:CH₃CN=90:10 containing 0.1% formic acid), electrospray ionization (ESI) is used, and DAD is used for testing at 210 nm/254 nm.

A compound is purified using Cheetah Pro medium-pressure rapid purification from Tianjin Bonna-Agela Technology Co., Ltd. for preparing a chromatogram, and UV is used for testing at 210 nm/254 nm.

The following abbreviations are used throughout the present invention:

| | | | |
|---|---|---|---|
| PE | Petroleum ether | EA/EtOAc | Ethyl acetate |
| mg | Milligram | mmol | Millimole |
| mL | Milliliter | g | Gram |
| M | Mole/liter | rpm | Revolutions per minute |
| µM | Micromole/liter | h | Hour |
| DCM | Dichloromethane | MeOH | Methanol |
| min | Minute | CDCl₃ | Deuterated chloroform |
| DMSO-*d*₆ | Deuterated dimethyl sulfoxide | nM | Nanomole/liter |
| HEPES | 4-hydroxyethylpiperazine ethane sulfonic acid | BSA | Bovine serum albumin |
| IBMX | 3-isobutyl-1-methylxanthine | HBSS | Hank's balanced salt solution |
| nL | Nanoliter | DIPEA | *N*,*N*-Diisopropylethylamine |
| THF | Tetrahydrofuran | CDI | *N,N*'-carbonyldiimidazole |
| DBU | 1,8-diazabicyclo[5.4.0]undec-7-ene | EDCI | 1-ethyl-(3-dimethylaminopropyl) carbodiimide hydrochloride |
| HATU | 2-(7-azabenzotriazole)-N,N,N',N'-tetramethylurea hexafluorophosphate | LiHMDS | Lithium bis(trimethylsilyl)amide or lithium hexamethyldisilazide |
| HOAT | 1-hydroxyl-7-azabenzotriazole | NMP | *N*-methylpyrrolidone |

The following solutions describe steps for preparing the compound of the present invention. Unless otherwise specified, X₁, X₂, Y₁, Y₂, Y₃, Y₄, R¹, R², R³, R⁴, R⁵, R⁶, R⁷, R⁸, R⁹, R²², R²³, L₃, and L₄ have the definitions as described in the present invention. Boc is a commonly used protecting group tert-butoxycarbonyl.

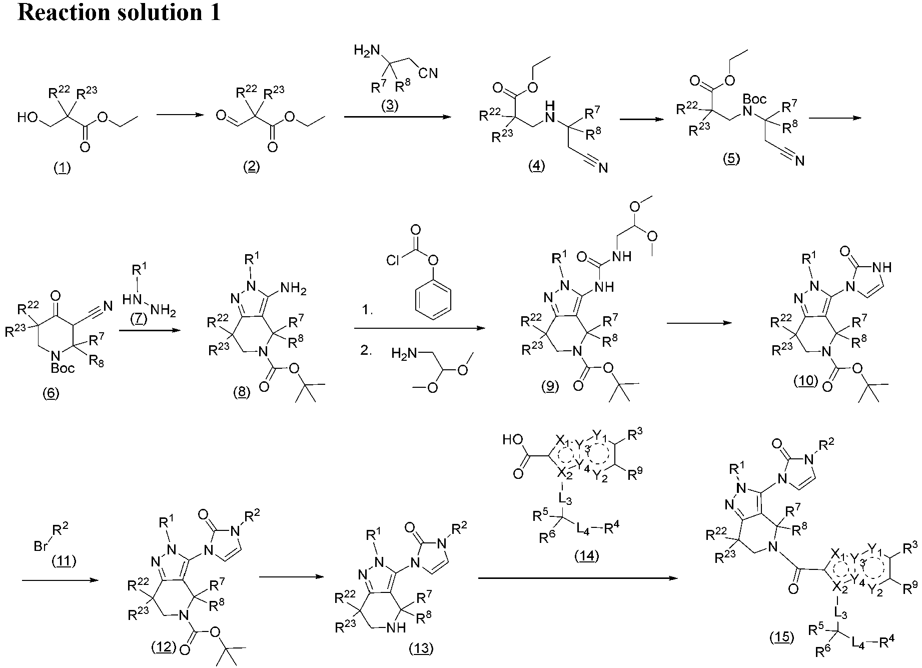

The compound of formula (15) may be prepared by the reaction solution 1: The compound of formula (1) undergoes an oxidation reaction to obtain the compound of formula (2). The compound of formula (2) reacts with the compound of formula (3) to obtain the compound of formula (4). The compound of formula (4) undergoes a Boc protection reaction to obtain the compound of formula (5). The compound of formula (5) undergoes an intramolecular cyclization reaction to obtain the compound of formula (6). The compound of formula (6) and the compound of formula (7) undergo a cyclization reaction to obtain the compound of formula (8). The compound of formula (8) sequentially reacts with phenyl chloroformate and 2,2-dimethoxyethane-1-amine to obtain the compound of formula (9). The compound of formula (9) undergoes an intramolecular cyclization reaction to obtain the compound of formula (10). The compound of formula (10) reacts with the compound of formula (11) to obtain the compound of formula (12). The compound of formula (12) undergoes removal of the N-Boc protecting group to obtain the compound of formula (13). The compound of formula (13) reacts with the compound of formula (14) to obtain the compound of formula (15). The compound of formula (14) is synthesized with reference to preparation methods of patents CN109790161A and CN116390926A.

### DETAILED DESCRIPTION

The present invention is described below with reference to specific example. It should be noted that these examples are only descriptive and do not limit the present invention in any way.

### Example 1 Synthesis of 3-((1S,2S)-1-(5-((S)-2,2-dimethyltetrahydro-2H-pyran-4-yl)-2-((S)-3'-(3-(4-fluoro-1-methyl-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro [cyclopropane-1,7'-pyrazolo[4,3-c]pyridine]-5'-carbonyl)-1H-indol-1-yl)-2-methylcyclopropyl)-1,2,4-oxadiazol-5(4H)-one (Compound 1)

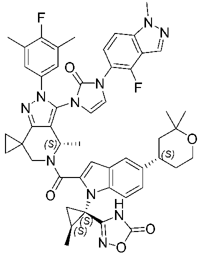

### Step 1: Synthesis of ethyl 1-formylcyclopropane-1-formate

Ethyl 1-(hydroxymethyl)cyclopropane-1-formate (10 g, 69.36 mmol) and dichloromethane (200 mL) were added in sequence to a reaction flask. Dess-Martin periodinane (44.2 g, 104.04 mmol) was added in batches at 0°C, and then the reaction mixture was heated to room temperature and stirred for 2 h. In an ice bath, the reaction was quenched with a saturated sodium thiosulfate solution, and then saturated sodium bicarbonate was added to adjust the pH to be weakly alkaline. The reaction mixture was extracted with dichloromethane (200 mL×3). The organic phases were combined, washed with a saturated saline solution (200 mL), dried with anhydrous sodium sulfate, filtered, concentrated under reduced pressure, and separated by silica gel column chromatography (PE:EA (v/v)=10:1 to 5:1) to obtain the target compound.

### Step 2: Synthesis of ethyl ((S)-1-(((1-cyanopropan-2-yl)amino)methyl)cyclopropane-1-formate

(S)-3-aminobutanenitrile hydrochloride (2 g, 16.59 mmol), methanol (30 mL), and diisopropylethylamine (2.14 g, 16.59 mmol) were added in sequence to a reaction flask, and the reaction solution was stirred for 20 min. Then, acetic acid (896 mg, 14.93 mmol) and ethyl 1-formylcyclopropane-1-formate (3.1 g, 21.73 mmol) were added and stirred for 2 h. Sodium triacetoxyborohydride (5.25 g, 24.88 mmol) was added and stirred at room temperature for 6 h. The reaction was stopped, and the reaction mixture was diluted with water and extracted with ethyl acetate (30 mL×3). The organic phases were combined, washed with a saturated sodium bicarbonate solution (50 mL) and a saturated saline solution (50 mL) in sequence, dried with anhydrous sodium sulfate, filtered, concentrated under reduced pressure, and used directly for the next step.

### Step 3: Synthesis of ethyl (S)-1-(((tert-butyloxycarbonyl)(1-cyanopropan-2-yl)amino)methyl)cyclopropane-1-formate

Ethyl ((*S*)-1-(((1-cyanopropan-2-yl)amino)methyl)cyclopropane-1-formate obtained in the previous step, dichloroethane (30 mL), and di-tert-butyl dicarbonate (4.36 g, 20.0 mmol) were added in sequence to a reaction flask. The reaction mixture was allowed to react overnight at room temperature. The reaction solution was concentrated under reduced pressure and separated by silica gel column chromatography (PE:EA (v/v)=10:1 to 5:1) to obtain the target compound.

### Step 4: Synthesis of tert-butyl (6S)-7-cyano-6-methyl-8-oxo-5-azaspiro[2.5]octane-5-carboxylate

Ethyl (*S*)-1-(((tert-butyloxycarbonyl)(1-cyanopropan-2-yl)amino)methyl)cyclopropane-1-formate (1.05 g, 3.38 mmol), tetrahydrofuran (10 mL), and a 1 M solution of potassium tert-butoxide in THF (5 mL, 5.0 mmol) were added in sequence to a reaction flask. After addition, the reaction mixture was stirred at room temperature for 1 h, quenched with a saturated ammonium chloride solution, and extracted with ethyl acetate (15 mL×3). The organic phases were combined, washed with a saturated saline solution (30 mL), dried with anhydrous sodium sulfate, filtered, concentrated under reduced pressure, and used directly for the next step.

### Step 5: Synthesis of tert-butyl (S)-3'-amino-2'-(4-fluoro-3,5-dimethylphenyl)-4'-methyl-2',4'-dihydrospiro[cyclopropane-1,7'-pyrazolo[4,3-c]pyridine]-5'(6'H)-carboxylate

(4-fluoro-3,5-dimethylphenyl)hydrazine (560 mg, 3.63 mmol), tert-butyl (6*S*)-7-cyano-6-methyl-8-oxo-5-azaspiro[2.5]octane-5-carboxylate (480 mg, 1.82 mmol), and toluene (10 mL) were added to a reaction flask. A dioxane hydrochloride solution (0.45 mL) was added under nitrogen protection. The reaction mixture was heated to 80°C to react for 1.5 h, and then cooled to room temperature. A saturated saline solution (20 mL) was added, and the reaction mixture was extracted with ethyl acetate (15 mL×3). The organic phases were combined, dried with anhydrous sodium sulfate, and separated and purified by silica gel column chromatography (PE:EA (v/v)=5:1 to 1:1) to obtain the target compound.

LC-MS (ESI): [M+H]⁺ =401.4.

### Step 6: Synthesis of tert-butyl (S)-3'-(3-(2,2-dimethoxyethyl)urea)-2'-(4-fluoro-3,5-dimethylphenyl)-4'-methyl-2',4'-dihydrospiro[cyclopropane-1,7'-pyrazolo[4,3-c]pyridine]-5'(6'H)-carboxylate

Tert-butyl (*S*)-3'-amino-2'-(4-fluoro-3,5-dimethylphenyl)-4'-methyl-2',4'-dihydrospiro[cyclopropane-1,7'-pyrazolo[4,3-*c*]pyridine]-5'(6'*H*)-carboxylate (660 mg, 1.65 mmol), DIPEA (1064 mg, 8.24 mmol), and tetrahydrofuran (10 mL) were added to a reaction flask. Phenyl chloroformate (774 mg, 4.95 mmol) was added dropwise under stirring. After 1 h of reaction at room temperature, 2,2-dimethoxyethane-1-amine (693 mg, 6.59 mmol) was added to the system and stirred for 1 h, and then the reaction was stopped. A saturated saline solution (20 mL) was added, and the reaction mixture was extracted with ethyl acetate (15 mL×3). The organic phases were combined, dried with anhydrous sodium sulfate, and separated and purified by silica gel column chromatography (PE:EA (v/v)=10:1 to 2:1) to obtain the target compound.

LC-MS (ESI): [M+H]⁺ =532.4.

### Step 7: Synthesis of tert-butyl (S)-2'-(4-fluoro-3,5-dimethylphenyl)-4'-methyl-3'-(2-oxo-2,3-dihydro-1H-imidazol-1-yl)-2',4'-dihydrospiro[cyclopropane-1,7'-pyrazolo[4,3-c]pyridine]-5'(6'H)-carboxylate

Tert-butyl (*S*)-3'-(3-(2,2-dimethoxyethyl)urea)-2'-(4-fluoro-3,5-dimethylphenyl)-4'-methyl-2',4'-dihydrospiro[cyclopropane-1,7'-pyrazolo[4,3-c]pyridine]-5'(6'*H*)-carboxylate (540 mg, 1.02 mmol), trifluoromethanesulfonic acid (137 mg, 0.91 mmol), and tetrahydrofuran (6 mL) were added to a reaction flask and heated to react for 1 h. A saturated saline solution (20 mL) was added, and the reaction mixture was extracted with ethyl acetate (15 mL×3). The organic phases were combined, dried with anhydrous sodium sulfate, and separated and purified by silica gel column chromatography (PE:EA (v/v)=10:1 to 2:1) to obtain the target compound.

LC-MS (ESI): [M+H]⁺ =468.4.

### Step 8: Synthesis of tert-butyl (S)-3'-(3-(4-fluoro-1-methyl-1H-indol-5-yl)-2-oxo-2,3-dihydro-1H-imidazol-1-yl)-2'-(4-fluoro-3,5-dimethylphenyl)-4'-methyl-2',4'-dihydrospiro[cyclopropane-1,7'-pyrazolo[4,3-c]pyridine]-5'(6'H)-carboxylate

Tert-butyl (*S*)-2'-(4-fluoro-3,5-dimethylphenyl)-4'-methyl-3'-(2-oxo-2,3-dihydro-1*H-*imidazol-1-yl)-2',4'-dihydrospiro[cyclopropane-1,7'-pyrazolo[4,3-c]pyridine]-5'(6'*H*)-carboxylate (400 mg, 0.86 mmol), 5-bromo-4-fluoro-1-methyl-1H-indole (254 mg, 1.11 mmol), cuprous iodide (135.7 mg, 0.43 mmol), (1*S*,2*S*)-(+)-*N,N*'-dimethyl-1,2-cyclohexanediamine (60.8 mg, 0.43 mmol), and NMP (15 mL) were added to a reaction flask, and heated to 130°C to react for 2 h under nitrogen protection. A saturated saline solution was added, and the reaction mixture was extracted with ethyl acetate. The organic phases were combined, dried with anhydrous sodium sulfate, and separated by column chromatography to obtain the target compound.

LC-MS (ESI): [M+H]⁺ =616.5.

### Step 9: Synthesis of (S)-1-(4-fluoro-1-methyl-1H-indol-5-yl)-3-(2'-(4-fluoro-3,5-dimethylphenyl)-4'-methyl-2',4',5',6'-tetrahydrospiro[cyclopropane-1,7'-pyrazolo[4,3-c]pyridine]-3'-yl)-1,3-dihydro-2H-imidazol-2-one

Tert-butyl (*S*)-3'-(3-(4-fluoro-1-methyl-1*H*-indol-5-yl)-2-oxo-2,3-dihydro-1*H*-imidazol-1-yl)-2'-(4-fluoro-3,5-dimethylphenyl)-4'-methyl-2',4'-dihydrospiro[cyclopropane-1,7'-pyrazolo[4,3-*c*]pyridine]-5'(6'*H*)-carboxylate (600 mg, 0.97 mmol) and dioxane hydrochloride (4 mL) were added to a reaction flask, allowed to react at room temperature for 1 h, and concentrated to obtain the target compound.

LC-MS (ESI): [M+H]⁺ =516.4.

### Step 10: Synthesis of 3-((1S,2S)-1-(5-((S)-2,2-dimethyltetrahydro-2H-pyran-4-yl)-2-((S)-3'-(3-(4-fluoro-1-methyl-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-1H-indol-1-yl)-2-methylcyclopropyl)-1,2,4-oxadiazol-5(4H)-one

(*S*)-1-(4-fluoro-1-methyl-1*H*-indol-5-yl)-3-(2'-(4-fluoro-3,5-dimethylphenyl)-4'-methyl-2',4',5',6'-tetrahydrospiro[cyclopropane-1,7'-pyrazolo[4,3-*c*]pyridine]-3'-yl)-1,3-dihydro-2*H-*imidazol-2-one (46.4 mg, 0.09 mmol), 5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indol-2-carboxylic acid (35 mg, 0.09 mmol), HATU (48.5 mg, 0.13 mmol), and DMF (4 mL) were added to a reaction flask. DIPEA (54.9 mg, 0.43 mmol) was added under stirring to react overnight at room temperature. A saturated saline solution (20 mL) was added, and the reaction mixture was extracted with ethyl acetate (15 mL×3). The organic phases were combined, dried with anhydrous sodium sulfate, concentrated under reduced pressure, and separated and purified by silica gel column chromatography (PE:EA (v/v)=1:1 to 1:3) to obtain the target compound.

LC-MS (ESI): [M+H]⁺ =909.5;
1H NMR (400 MHz, CDCl₃) δ 11.28 - 11.22 (m, 1H), 8.07 - 7.93 (m, 1H), 7.53 - 7.37 (m, 3H), 7.23 - 7.09 (m, 2H), 7.06 - 6.90 (m, 2H), 6.66 - 6.36 (m, 2H), 6.25 - 5.90 (m, 1H), 5.81 - 5.24 (m, 1H), 4.98 - 4.87 (m, 1H), 4.05 - 3.98 (m, 3H), 3.84 - 3.53 (m, 3H), 3.00 - 2.91 (m, 1H), 2.21 - 2.19 (m, 3H), 2.17 - 2.14 (m, 3H), 1.73 - 1.69 (m, 1H), 1.64 - 1.57 (m, 2H), 1.53 - 1.50 (m, 1H), 1.28 - 1.25 (m, 3H), 1.22 - 1.21 (m, 3H), 1.18 - 1.17 (m, 3H), 1.17 - 1.13 (m, 6H), 1.13 - 0.99 (m, 2H), 0.82 - 0.78 (m, 2H).

### Example 2 Synthesis of 3-((1S,2S)-1-(2-(S)-3'-(3-(1-methyl-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-5-((S)-2,2-dimethyltetrahydro-2H-pyran-4-yl)-1H-indol-1-yl)-2-methylcyclopropyl)-1,2,4-oxadiazol-5(4H)-one (Compound 2)

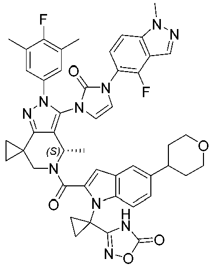

### Step 1: Synthesis of 1-(1-cyanocyclopropyl)-N-methyl-N-phenyl-5-(tetrahydro-2H-pyran-4-yl)-1H-indol-2-carboxamide

1-(cyanomethyl)-N-methyl-N-phenyl-5-(tetrahydro-2H-pyran-4-yl)-1H-indol-2-carboxamide (400 mg, 1.07 mmol) and 1,3,2-dioxathiane 2,2-dioxide (399 mg, 3.21 mmol) were added to anhydrous tetrahydrofuran (5 mL) and nitrogen replacement was performed three times. 1M lithium bis(trimethylsilyl)amide (4.3 mL) was slowly added dropwise under stirring at 0°C to react at 0°C for 3 h after the dropwise addition. The reaction was quenched by adding 1N dilute hydrochloric acid dropwise, and the reaction mixture was diluted with water (30 mL) and extracted with ethyl acetate (15 mL×3). The extract was washed with a saturated saline solution (20 mL), dried with anhydrous sodium sulfate, filtered, concentrated under reduced pressure, and separated by silica gel column chromatography (PE:EA (v/v)=10:1 to 3:1) to obtain the target compound.

LC-MS (ESI): [M+H]⁺=400.2.

### Step 2: Synthesis of N-methyl-1-(1-(5-oxo-4,5-dihydro-1,2,4-oxadiazol-3-yl)cyclopropyl)-N-phenyl-5-(tetrahydro-2H-pyran-4-yl)-1H-indol-2-carboxamide

Sodium bicarbonate (526 mg, 6.26 mmol) and hydroxylamine hydrochloride (435 mg, 6.26 mmol) were added to a solution of 1-(1-cyanocyclopropyl)-N-methyl-N-phenyl-5-(tetrahydro-2H-pyran-4-yl)-1H-indol-2-carboxamide (500 mg, 1.25 mmol) in DMSO (8 mL) to react at 60°C for 16 h. The reaction mixture was diluted with water (30 mL), and extracted with ethyl acetate (15 mL×3). The extract was washed with a saturated saline solution (20 mL×2), dried with anhydrous sodium sulfate, filtered, and concentrated under reduced pressure to obtain a crude product. The crude product, CDI (397 mg, 2.45 mmol), and DBU (466 mg, 3.06 mmol) were added to DMSO (5 mL). The reaction mixture was allowed to react at room temperature for 2 h, diluted with water (30 mL), and extracted with ethyl acetate (15 mL×3). The extract was washed with a saturated saline solution (20 mL), dried with anhydrous sodium sulfate, filtered, concentrated under reduced pressure, and separated by silica gel column chromatography (PE:EA (v/v)=3:1 to 0:1) to obtain the target compound.

LC-MS (ESI): [M+H]⁺=459.3.

### Step 3: Synthesis of 1-(1-(5-oxo-4,5-dihydro-1,2,4-oxadiazol-3-yl)cyclopropyl)-5-(tetrahydro-2H-pyran-4-yl)-1H-indol-2-formic acid

The compound *N*-methyl-1-(1-(5-oxo-4,5-dihydro-1,2,4-oxadiazol-3-yl)cyclopropyl)-*N-*phenyl-5-(tetrahydro-2H-pyran-4-yl)-1H-indol-2-carboxamide (210 mg, 0.46 mmol) and potassium hydroxide (514 mg, 9.16 mmol) were added to DMF (5 mL) to react at 125°C for 16 h. 1N dilute hydrochloric acid was added dropwise to adjust the pH to be acidic. The reaction mixture was extracted with ethyl acetate (15 mL×3), and the extract was washed with a saturated saline solution (20 mL), dried with anhydrous sodium sulfate, filtered, concentrated under reduced pressure, and subjected to preparative HPLC to obtain the target compound.

LC-MS (ESI): [M+H]⁺=370.2.

### Step 4: Synthesis of 3-((1S,2S)-1-(2-(S)-3'-(3-(1-methyl-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-5-((S)-2,2-dimethyltetrahydro-2H-pyran-4-yl)-1H-indol-1-yl)-2-methylcyclopropyl)-1,2,4-oxadiazol-5(4H)-one

(*S*)-1-(4-fluoro-1-methyl-1*H*-indazol-5-yl)-3-(2'-(4-fluoro-3,5-dimethylphenyl)-4'-methyl-2',4',5',6'-tetrahydrospiro[cyclopropane-1,7'-pyrazolo[4,3-*c*]pyridine]-3'-yl)-1,3-dihydro-2*H-*imidazol-2-one (30 mg, 0.06 mmol), 1-(1-(5-oxo-4,5-dihydro-1,2,4-oxadiazol-3-yl)cyclopropyl)-5-(tetrahydro-2H-pyran-4-yl)-1H-indol-2-formic acid (22 mg, 0.06 mmol), EDCI (28 mg, 0.15 mmol), HOAT (12 mg, 0.09 mmol), and DMF (1 mL) were added to a single-necked flask. DIPEA (23 mg, 0.17 mmol) was added under stirring to react overnight at room temperature. The reaction mixture was diluted with water (20 mL) and extracted with ethyl acetate (15 mL×3). The extract was washed with a saturated saline solution (20 mL), dried with anhydrous sodium sulfate, filtered, concentrated under reduced pressure, and subjected to preparative HPLC to obtain the target compound.

LC-MS (ESI): [M+H]⁺=867.6;
¹H NMR (400 MHz, DMSO-*d*₆) *δ* 12.11 (s, 1H), 8.32 (s, 1H), 7.61 - 7.37 (m, 4H), 7.28 - 7.21 (m, 2H), 7.18 - 7.07 (m, 3H), 6.68 (s, 1H), 4.90 (p, *J=* 6.2 Hz, 1H), 4.15 - 4.10 (m, 3H), 4.05 - 3.90 (m, 3H), 3.80 - 3.63 (m, 1H), 3.56 - 3.44 (m, 2H), 2.96 - 2.80 (m, 1H), 2.28 - 2.20 (m, 6H), 1.91 - 1.65 (m, 3H), 1.55 - 1.36 (m, 3H), 1.35 - 1.22 (m, 5H), 1.19 (d, *J =* 6.3 Hz, 2H), 0.91 - 0.84 (m, 2H).

### Example 3 Synthesis of 3-((1S,2S)-1-(2-((S)-3'-(3-(4-fluoro-1-methyl-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-5-(tetrahydro-2H-pyran-4-yl)-1H-indol-1-yl)-2-methylcyclopropyl)-1,2,4-oxadiazol-5(4H)-one (Compound 3)

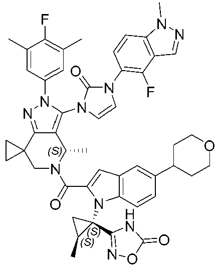

### Step 1: Synthesis of ethyl 5-(3,6-dihydro-2H-pyran-4-yl)-1H-indol-2-formate

2-(3,6-dihydro-2*H*-pyran-4-yl)-4,4,5,5-tetramethyl-1,3,2-dioxaborolane (1.57 g, 7.46 mmol), Pd (dppf) Cl₂·CH₂Cl₂ (0.61 g, 0.75 mmol), and K₂CO₃ (3.09 g, 22.38 mmol) were added to a solution of ethyl 5-bromo-1H-indol-2-formate (2.0 g, 7.46 mmol) in dioxane/H₂O (46 mL/12 mL). The reaction mixture was heated to 80°C in a nitrogen atmosphere to react under stirring for 2.5 h, cooled to room temperature, filtered, diluted with DCM (50 mL), washed with water (50 mL), dried with anhydrous sodium sulfate, concentrated under reduced pressure, and separated by silica gel column chromatography (PE:EA (v/v)=9:1 to 3:1) to obtain the target compound.

LC-MS (ESI): [M+H]⁺ =272.3.

### Step 2: Synthesis of ethyl 5-(tetrahydro-2H-pyran-4-yl)-1H-indol-2-formate

10% w/w Pd/C (200 mg) was added to a solution of ethyl 5-(3,6-dihydro-2H-pyran-4-yl)-1H-indol-2-formate (1.91 g, 7.04 mmol) in MeOH/THF (15 mL/45 mL). The mixture was stirred overnight at room temperature in an H₂ atmosphere. The reaction solution was filtered, concentrated, pulped with MeOH, and filtered to obtain the target compound.

LC-MS (ESI): [M+H]⁺ =274.1.

### Step 3: Synthesis of ethyl 1-(cyanomethyl)-5-(tetrahydro-2H-pyran-4-yl)-1H-indol-2-formate

NaH (60% in oil) (0.41 g, 10.26 mmol) was added to a solution of ethyl 5-(tetrahydro-2H-pyran-4-yl)-1H-indol-2-formate (1.87 g, 6.84 mmol) in DMF (60 mL) at 0°C, and stirred at 0°C for 0.5 h. 2-chloroacetonitrile (1.03 g, 13.68 mmol) was added and stirred overnight at room temperature. The reaction was quenched with H₂O (50 mL) at 0°C, and the suspension was filtered and dried to obtain the target compound.

LC-MS (ESI): [M+H]⁺ =313.3.

### Step 4: Synthesis of 1-(cyanomethyl)-5-(tetrahydro-2H-pyran-4-yl)-1H-indol-2-carboxylic acid

LiOH·H₂O(360 mg, 86.4 mmol) was added to a solution of ethyl 1-(cyanomethyl)-5-(tetrahydro-2H-pyran-4-yl)-1H-indol-2-formate (1.8 g, 5.76 mmol) in H₂O/THF (16 mL/28 mL) at 0°C, and stirred at room temperature for 3 h. THF was removed under vacuum and the pH was adjusted to about 4 to 5 with a 1N HCl solution. The suspension was filtered to obtain the target compound.

### Step 5: Synthesis of 1-(cyanomethyl)-N-methyl-N-phenyl-5-(tetrahydro-2H-pyran-4-yl)-1H-indol-2-carboxamide

DIPEA (2.6 mL, 15.93 mmol) was added to a mixed solution of 1-(cyanomethyl)-5-(tetrahydro-2H-pyran-4-yl)-1H-indol-2-carboxylic acid (1.51 g, 5.31 mmol) in DMF (70 mL) and HATU (3.03 g, 7.97 mmol) at 0°C, and stirred at 0°C for 1 h. Then N-methylaniline (0.9 mL, 7.97 mmol) was added and stirred overnight at room temperature. The reaction mixture was diluted with water (50 mL) and extracted with ethyl acetate (50 mL×3). The organic phases were dried with anhydrous sodium sulfate, concentrated under reduced pressure, and separated by silica gel column chromatography (PE:EA (v/v)=2:1 to 1:1) to obtain the target compound.

LC-MS (ESI): [M+H]⁺ =374.3.

### Step 6: Synthesis of 1-((1S,2S)-1-cyano-2-methylcyclopropyl)-N-methyl-N-phenyl-5-(tetrahydro-2H-pyran-4-yl)-1H-indol-2-carboxamide

LiHMDS (1.0 mol/L THF solution, 5.9 mL, 5.89 mmol) was added dropwise to a solution of 1-(cyanomethyl)-*N*-methyl-*N*-phenyl-5-(tetrahydro-2*H-*pyran-4-yl)-1*H*-indol-2-carboxamide (0.55 g, 1.47 mmol) and (*R*)-4-methyl-1,3,2-dioxathiocyclopentane 2,2-dioxide (0.51 g, 3.68 mmol) in THF (10 mL) at 0°C in an N₂ atmosphere, and stirred at 0°C for 2 h. The reaction was quenched with HCOOH (10 mL), and the reaction mixture was concentrated under vacuum and separated by silica gel column chromatography (PE:EA (v/v)=2:1 to 1:1) to obtain the target compound.

LC-MS (ESI): [M+H]⁺ =414.2.

### Step 7: Synthesis of N-methyl-1-((1S,2S)-2-methyl-1-(5-oxo-4,5-dihydro-1,2,4-oxadiazol-3-yl)cyclopropyl)-N-phenyl-5-(tetrahydro-2H-pyran-4-yl)-1H-indol-2-carboxamide

A solution of 1-((1*S*,2*S*)-1-cyano-2-methylcyclopropyl)-*N*-methyl-*N*-phenyl-5-(tetrahydro-2H-pyran-4-yl)-1H-indol-2-carboxamide (475 mg, 1.15 mmol), NH₂OH·HCl (399 mg, 5.74 mmol), and K₂CO₃ (873 mg, 6.32 mmol) in EtOH (10 mL) was stirred at 100°C for 2 h. The reaction mixture was concentrated, and then diluted with H₂O (50 mL). The suspension was filtered and dried under vacuum. The solid was dissolved in DMSO (5 mL), and then carbonyldiimidazole (373 mg, 2.3 mmol) and 1,8-diazabicycloundec-7-ene (437 mg, 2.87 mmol) were added and stirred at 80°C for 1 h. The reaction mixture was concentrated under vacuum, and separated by silica gel column chromatography (PE:EA (v/v)=2:1 to 1:1) to obtain the target compound.

LC-MS (ESI): [M+H]⁺ =473.3.

### Step 8: Synthesis of 1-((1S,2S)-2-methyl-1-(5-oxo-4,5-dihydro-1,2,4-oxadiazol-3-yl)cyclopropyl)-5-(tetrahydro-2H-pyran-4-yl)-1H-indol-2-carboxylic acid

A solution of *N*-methyl-1-((1*S*,2*S*)-2-methyl-1-(5-oxo-4,5-dihydro-1,2,4-oxadiazol-3-yl)cyclopropyl)-N-phenyl-5-(tetrahydro-2H-pyran-4-yl)-1H-indol-2-carboxamide (215 mg, 0.45 mmol) and KOH (894 mg, 15.9 mmol) in dimethoxyethane (10 mL) were stirred overnight at 125°C. The reaction solution was adjusted to pH=3 with a 5N HCl solution and the reaction solution was filtered. The solid was washed with H₂O (5 mL) and dried under reduced pressure to obtain the target compound.

LC-MS (ESI): [M+H]⁺=384.2.

### Step 9 Synthesis of 3-((1S,2S)-1-(2-((S)-3'-(3-(4-fluoro-1-methyl-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-5-(tetrahydro-2H-pyran-4-yl)-1H-indol-1-yl)-2-methylcyclopropyl)-1,2,4-oxadiazol-5(4H)-one

(*S*)-1-(4-fluoro-1-methyl-1*H*-indol-5-yl)-3-(2'-(4-fluoro-3,5-dimethylphenyl)-4'-methyl-2',4',5',6'-tetrahydrospiro[cyclopropane-1,7'-pyrazolo[4,3-*c*]pyridine]-3'-yl)-1,3-dihydro-2*H-*imidazol-2-one (50 mg, 0.1 mmol), 1-((1S,2S)-2-methyl-1-(5-oxo-4,5-dihydro-1,2,4-oxadiazol-3-yl)cyclopropyl)-5-(tetrahydro-2H-pyran-4-yl)-1H-indol-2-carboxylic acid (37 mg, 0.1 mmol), HOAT (19.8 mg, 0.15 mmol), EDCI·HCl (47 mg, 0.24 mmol), and DMF (2 mL) were added to a reaction flask. DIPEA (37.6 mg, 0.29 mmol) was added under stirring to react overnight at room temperature. A saturated saline solution was added, and the reaction mixture was extracted with ethyl acetate. The organic phases were combined, dried with anhydrous sodium sulfate, and separated by silica gel column chromatography (PE:EA (v/v)=60% to 900%) to obtain the target compound.

LC-MS (ESI): [M+H]⁺=881.5;
¹H NMR (400 MHz, DMSO-*d*₆) δ 11.73 (s, 1H), 8.33 (s, 1H), 7.66 (d, *J=* 8.8 Hz, 1H), 7.60 (d, *J= 11.2* Hz, 1H), 7.51 (d, *J=* 8.1 Hz, 1H), 7.41 (d, *J=* 8.7 Hz, 1H), 7.28 (d, *J =* 8.6 Hz, 1H), 7.15 (s, 2H), 7.09 (s, 1H), 6.98 (d, *J= 29.2* Hz, 1H), 5.75 - 5.26 (m, 1H), 4.13 (s, 3H), 3.98 (d, *J* = 11.0 Hz, 2H), 3.47 (s, 4H), 2.92 - 2.71 (m, 1H), 2.26 (s, 6H), 1.75 (d, *J=* 10.5 Hz, 8H), 1.38 (s, 4H), 1.25 (s, 4H), 1.22 - 1.11 (m, 2H).

### Example 4 Synthesis of 3-((1S,2S)-1-(2-(S)-3'-(3-(1-cyclopropyl-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-5-((S)-2,2-dimethyltetrahydro-2H-pyran-4-yl)-1H-indol-1-yl)-2-methylcyclopropyl)-1,2,4-oxadiazol-5(4H)-one (Compound 4)

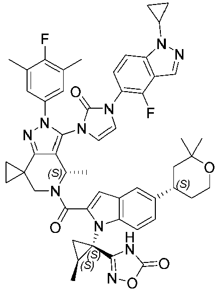

### Step 1: Synthesis of 5-bromo-1-cyclopropyl-4-fluoro-1H-indazole

5-bromo-4-fluoro-1H-indazole (350 mg, 1.63 mmol), cyclopropylboronic acid (280 mg, 3.26 mmol), copper acetate (296 mg, 1.63 mmol), 2,2'-bipyridine (156 mg, 1.63 mmol), sodium carbonate (345 mg, 3.26 mmol), and dichloroethane (10 mL) were added to a reaction flask and heated to 70°C to react for 2 h. The reaction mixture was diluted with water (20 mL), and extracted with ethyl acetate (15 mL×3). The extract was washed with a saturated saline solution (20 mL), dried with anhydrous sodium sulfate, filtered, concentrated under reduced pressure, and separated by silica gel column chromatography (PE:EA (v/v)=5:1 to 1:1) to obtain the target compound.

LC-MS (ESI): [M+H]⁺ =255.1.

### Step 2: Synthesis of tert-butyl (S)-3'-(3-(1-cyclopropyl-4-fluoro-1H-indazol-5-yl)-2-oxo-2,3-dihydro-1H-imidazol-1-yl)-2'-(4-fluoro-3,5-dimethylphenyl)-4'-methyl-2',4'-dihydrospiro[cyclopropane-1,7'-pyrazolo[4,3-c]pyridine]-5'(6'H)-carboxylate

Tert-butyl (*S*)-2'-(4-fluoro-3,5-dimethylphenyl)-4'-methyl-3'-(2-oxo-2,3-dihydro-1*H-*imidazol-1-yl)-2',4'-dihydrospiro[cyclopropane-1,7'-pyrazolo[4,3-c]pyridine]-5'(6'*H*)-carboxylate (100 mg, 0.21 mmol), 5-bromo-1-cyclopropyl-4-fluoro-1H-indole (71 mg, 0.28 mmol), cuprous iodide (32 mg, 0.17 mmol), (1*S*,2*S*)-(+)-*N,N*'-dimethyl-1,2-cyclohexanediamine (24 mg, 0.17 mmol), potassium carbonate (89 mg, 0.64 mmol), and NMP (3 mL) were added to a reaction flask, and heated to 130°C to react for 2 h under nitrogen protection. The reaction mixture was diluted with water (20 mL), and extracted with ethyl acetate (15 mL×3). The extract was washed with a saturated saline solution (20 mL), dried with anhydrous sodium sulfate, filtered, concentrated under reduced pressure, and separated by silica gel column chromatography (PE:EA (v/v)=5:1 to 1:1) to obtain the target compound.

LC-MS (ESI): [M+H]⁺ =642.4.

### Step 3: Synthesis of (S)-1-(1-cyclopropyl-4-fluoro-1H-indazol-5-yl)-3-(2'-(4-fluoro-3,5-dimethylphenyl)-4'-methyl-2',4',5',6'-tetrahydrospiro[cyclopropane-1,7'-pyrazolo[4,3-c]pyridine]-3'-yl)-1,3-dihydro-2H-imidazol-2-one

Tert-butyl (*S*)-3'-(3-(1-cyclopropyl-4-fluoro-1*H*-indazol-5-yl)-2-oxo-2,3-dihydro-1*H-*imidazol-1-yl)-2'-(4-fluoro-3,5-dimethylphenyl)-4'-methyl-2',4'-dihydrospiro[cyclopropane-1,7'-pyrazolo[4,3-*c*]pyridine]-5'(6'*H*)-carboxylate (50 mg, 0.08 mmol) and dioxane hydrochloride (1 mL) were added to a reaction flask to react at room temperature for 1 h. A saturated sodium bicarbonate solution was added to adjust the pH to be alkaline. The reaction mixture was extracted with ethyl acetate (15 mL×3). The extract was washed with a saturated saline solution (20 mL), dried with anhydrous sodium sulfate, filtered, and concentrated under reduced pressure to obtain the target compound.

LC-MS (ESI): [M+H]⁺ =542.4.

### Step 4: Synthesis of 3-((1S,2S)-1-(2-(S)-3'-(3-(1-cyclopropyl-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-5-((S)-2,2-dimethyltetrahydro-2H-pyran-4-yl)-1H-indol-1-yl)-2-methylcyclopropyl)-1,2,4-oxadiazol-5(4H)-one

(*S*)-1-(1-cyclopropyl-4-fluoro-1*H*-indazol-5-yl)-3-(2'-(4-fluoro-3,5-dimethylphenyl)-4'-methyl-2',4',5',6'-tetrahydrospiro[cyclopropane-1,7'-pyrazolo[4,3-*c*]pyridine]-3'-yl)-1,3-dihydro-2H-imidazol-2-one (50 mg, 0.09 mmol), 5-((S)-2,2-dimethyltetrahydro-2H-pyran-4-yl)-1-((1*S*,2*S*)-2-methyl-1-(5-oxo-4,5-dihydro-1,2,4-oxadiazol-3-yl)cyclopropyl)-1H-indol-2-carboxylic acid (57 mg, 0.14 mmol), EDCI (44 mg, 0.23 mmol), HOAT (19 mg, 0.14 mmol), and DMF (2 mL) were added to a reaction flask. DIPEA (36 mg, 0.28 mmol) was added under stirring to react overnight at room temperature. The reaction mixture was diluted with water (20 mL) and extracted with ethyl acetate (15 mL×3). The extract was washed with a saturated saline solution (20 mL), dried with anhydrous sodium sulfate, filtered, concentrated under reduced pressure, and subjected to preparative HPLC to obtain the target compound.

¹H NMR (400 MHz, DMSO-*d*₆) *δ* 11.73 (s, 1H), 8.29 (s, 1H), 7.72 - 7.58 (m, 2H), 7.57 - 7.48 (m, 1H), 7.40 (d, *J= 9.0* Hz, 1H), 7.27 (d, *J=* 8.6 Hz, 1H), 7.12 (d, *J=* 26.1 Hz, 3H), 6.97 (d, *J =* 31.6 Hz, 1H), 6.83 - 6.67 (m, 1H), 4.95 - 4.85 (m, 1H), 4.27 - 3.93 (m, 1H), 3.91 - 3.83 (m, 1H), 3.80 - 3.66 (m, 3H), 3.14 - 2.97 (m, 1H), 2.28 - 2.20 (m, 6H), 1.79 - 1.48 (m, 5H), 1.44 - 1.35 (m, 2H), 1.29 - 1.24 (m, 12H), 1.23 - 1.19 (m, 6H), 0.91 - 0.81 (m, 2H).

### Example 5 Synthesis of 3-((1S,2S)-1-(2-(S)-3'-(3-(1-cyclopropyl-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-5-(tetrahydro-2H-pyran-4-yl)-1H-indol-1-yl)-2-methylcyclopropyl)-1,2,4-oxadiazol-5(4H)-one (Compound 5)

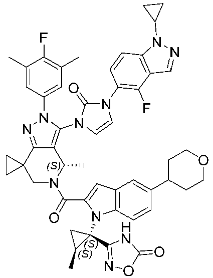

(*S*)-1-(1-cyclopropyl-4-fluoro-1*H*-indazol-5-yl)-3-(2'-(4-fluoro-3,5-dimethylphenyl)-4'-methyl-2',4',5',6'-tetrahydrospiro[cyclopropane-1,7'-pyrazolo[4,3-*c*]pyridine]-3'-yl)-1,3-dihydro-2H-imidazol-2-one (50 mg, 0.09 mmol), 1-((1S,2S)-2-methyl-1-(5-oxo-4,5-dihydro-1,2,4-oxadiazol-3-yl)cyclopropyl)-5-(tetrahydro-2H-pyran-4-yl)-1H-indol-2-formic acid (35 mg, 0.09 mmol), EDCI (44 mg, 0.23 mmol), HOAT (19 mg, 0.14 mmol), and DMF (2 mL) were added to a reaction flask. DIPEA (36 mg, 0.28 mmol) was added under stirring to react overnight at room temperature. The reaction mixture was diluted with water (30 mL) and extracted with ethyl acetate (15 mL×3). The extract was washed with a saturated saline solution (20 mL), dried with anhydrous sodium sulfate, filtered, concentrated under reduced pressure, and subjected to preparative HPLC to obtain the target compound.

¹H NMR (400 MHz, DMSO-*d*₆) δ 11.70 (s, 1H), 8.27 (s, 1H), 7.67 (d, *J =* 8.8 Hz, 1H), 7.59 - 7.56 (m, 1H), 7.54 - 7.46 (m, 1H), 7.39 (d, *J=* 7.8 Hz, 1H), 7.26 (d, *J=* 7.8 Hz, 1H), 7.13 - 7.06 (m, 3H), 7.00 - 6.91 (m, 1H), 6.79 - 6.67 (m, 1H), 5.33 - 5.30 (m, 1H), 4.21 - 3.66 (m, 5H), 3.51 - 3.37 (m, 2H), 2.87 - 2.83 (m, 1H), 2.24 (s, 6H), 2.07 - 1.90 (m, 1H), 1.79 - 1.47 (m, 7H), 1.41 - 1.22 (m, 6H), 1.20 - 1.06 (m, 6H), 0.85 - 0.81 (m, 1H).

### Example 6 Synthesis of 3-((1S,2S)-1-(5-((S)-2,2-dimethyltetrahydro-2H-pyran-4-yl)-2-((S)-3'-(3-(4-fluoro-1-(methyl-d₃)-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-1H-indol-1-yl)-2-methylcyclopropyl)-1,2,4-oxadiazol-5(4H)-one (Compound 6)

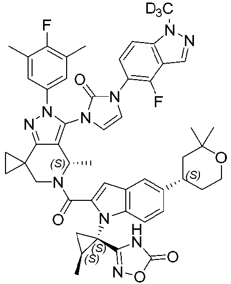

### Step 1: Synthesis of 5-bromo-4-fluoro-1-(methyl-d₃)-1H-indazole

In an ice bath, sodium hydride (60% dispersed in mineral oil) (48 mg, 1.21 mmol) was added in batches to a solution of 5-bromo-4-fluoro-1H-indazole (200 mg, 0.93 mmol) in anhydrous DMF to react for 1 h after addition. Deuterated iodomethane (175 mg, 1.21 mmol) was slowly added and the reaction mixture was heated to room temperature to react for 4 h. The reaction was quenched with water (20 mL), and the reaction mixture was extracted with ethyl acetate (15 mL×3). The extract was washed with a saturated saline solution (20 mL), dried with anhydrous sodium sulfate, filtered, concentrated under reduced pressure, and separated by silica gel column chromatography (PE:EA (v/v)=5:1 to 1:1) to obtain the target compound.

¹H NMR (400 MHz, DMSO-*d*₆) *δ* 8.24 (d, *J =* 0.9 Hz, 1H), 7.54 (dd, *J =* 8.8, 6.4 Hz, 1H), 7.41 (dd, J= 8.8, 1.0 Hz, 1H).

### Step 2: Synthesis of tert-butyl (S)-3'-(3-(4-fluoro-1-(methyl-d₃)-1H-indazol-5-yl)-2-oxo-2,3-dihydro-1H-imidazol-1-yl)-2'-(4-fluoro-3,5-dimethylphenyl)-4'-methyl-2',4'-dihydrospiro[cyclopropane-1,7'-pyrazolo[4,3-c]pyridine]-5'(6'H)-carboxylate

Tert-butyl (*S*)-2'-(4-fluoro-3,5-dimethylphenyl)-4'-methyl-3'-(2-oxo-2,3-dihydro-1*H-*imidazol-1-yl)-2',4'-dihydrospiro[cyclopropane-1,7'-pyrazolo[4,3-c]pyridine]-5'(6'*H*)-carboxylate (100 mg, 0.21 mmol), 5-bromo-4-fluoro-1-(methyl*-d*₃)-1*H*-indole (60 mg, 0.26 mmol), cuprous iodide (32 mg, 0.17 mmol), (1*S*,2*S*)-(+)-*N,N*'-dimethyl-1,2-cyclohexanediamine (24 mg, 0.17 mmol), potassium carbonate (89 mg, 0.64 mmol) and NMP (3 mL) were added to a flask, and heated to 130°C to react for 2 h under nitrogen protection. The reaction mixture was diluted with water (20 mL), and extracted with ethyl acetate (15 mL×3). The extract was washed with a saturated saline solution (20 mL), dried with anhydrous sodium sulfate, filtered, concentrated under reduced pressure, and separated by silica gel column chromatography (PE:EA (v/v)=5:1 to 1:1) to obtain the target compound.

LC-MS (ESI): [M+H]⁺ =619.4.

### Step 3: Synthesis of (S)-1-(1-4-fluoro-1-(methyl-d₃)-1H-indazol-5-yl)-3-(2'-(4-fluoro-3,5-dimethylphenyl)-4'-methyl-2',4',5',6'-tetrahydrospiro[cyclopropane-1,7'-pyrazolo[4,3-c]pyridine]-3'-yl)-1,3-dihydro-2H-imidazol-2-one

Tert-butyl (*S*)-3'-(3-(1-4-fluoro-1-(methyl-*d*₃)-1*H*-indazol-5-yl)-2-oxo-2,3-dihydro-1*H-*imidazol-1-yl)-2'-(4-fluoro-3,5-dimethylphenyl)-4'-methyl-2',4'-dihydrospiro[cyclopropane-1,7'-pyrazolo[4,3-*c*]pyridine]-5'(6'*H*)-carboxylate (78 mg, 0.13 mmol) and dioxane hydrochloride (1 mL) were added to a reaction flask to react at room temperature for 1 h. A saturated sodium bicarbonate solution was added to adjust the pH to be alkaline. The reaction mixture was extracted with ethyl acetate (15 mL×3). The extract was washed with a saturated saline solution (20 mL), dried with anhydrous sodium sulfate, filtered, and concentrated under reduced pressure to obtain the target compound.

LC-MS (ESI): [M+H]⁺ =519.4.

### Step 4: Synthesis of 3-((1S,2S)-1-(5-((S)-2,2-dimethyltetrahydro-2H-pyran-4-yl)-2-((S)-3'-(3-(4-fluoro-1-(methyl-d₃)-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro [cyclopropane-1,7'-pyrazolo[4,3-c]pyridine]-5'-carbonyl)-1H-indol-1-yl)-2-methylcyclopropyl)-1,2,4-oxadiazol-5(4H)-one

(*S*)-1-(1-4-fluoro-1-(methyl-d₃)-1*H*-indazol-5-yl)-3-(2'-(4-fluoro-3,5-dimethylphenyl)-4'-methyl-2',4',5',6'-tetrahydrospiro[cyclopropane-1,7'-pyrazolo[4,3-*c*]pyridine]-3'-yl)-1,3-dihydro-2H-imidazol-2-one (30 mg, 0.06 mmol), 5-((S)-2,2-dimethyltetrahydro-2H-pyran-4-yl)-1-((1*S*,2*S*)-2-methyl-1-(5-oxo-4,5-dihydro-1,2,4-oxadiazol-3-yl)cyclopropyl)-1*H*-indol-2-carboxylic acid (36 mg, 0.9 mmol), EDCI (28 mg, 0.14 mmol), HOAT (12 mg, 0.09 mmol), and DMF (2 mL) were added to a flask. DIPEA (22 mg, 0.17 mmol) was added under stirring to react overnight at room temperature. The reaction mixture was diluted with water (20 mL) and extracted with ethyl acetate (15 mL×3). The extract was washed with a saturated saline solution (20 mL), dried with anhydrous sodium sulfate, filtered, concentrated under reduced pressure, and subjected to preparative HPLC to obtain the target compound.

¹H NMR (400 MHz, DMSO-*d*₆) δ 11.74 (s, 1H), 8.33 (s, 1H), 7.70 - 7.60 (m, 2H), 7.51 (t, *J* = 7.8 Hz, 1H), 7.44 - 7.38 (m, 1H), 7.31 - 7.25 (m, 1H), 7.24 - 7.07 (m, 3H), 7.03 - 6.92 (m, 1H), 6.85 - 6.64 (m, 1H), 4.93 - 4.85 (m, 1H), 4.27 - 3.94 (m, 1H), 3.89 - 3.65 (m, 3H), 3.14 - 2.99 (m, 1H), 2.29 - 2.21 (m, 6H), 1.80 - 1.49 (m, 5H), 1.45 - 1.30 (m, 2H), 1.29 - 1.26 (m, 10H), 1.23 -1.16 (m, 4H), 0.92 - 0.82 (m, 2H).

### Example 7 Synthesis of 3-((1S,2S)-1-(5-(S)-2,2-dimethyltetrahydro-2H-pyran-4-yl)-2-(S)-3'-(3-(4-fluoro-2-(((S)-oxetan-2-yl)methyl)-2H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-1H-indol-1-yl)-2-methylcyclopropyl)-1,2,4-oxadiazol-5(4H)-one (Compound 7)

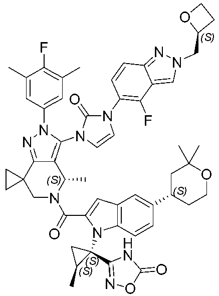

### Step 1: Synthesis of (S)-5-bromo-4-fluoro-2-(oxetan-2-ylmethyl)-2H indazole

5-bromo-4-fluoro-1H-indazole (200 mg, 0.93 mmol), oxetan-2-ylmethyl (S)-4-methylbenzenesulfonate (180 mg, 0.74 mmol), cesium carbonate (606 mg, 1.86 mmol), and acetonitrile (4 mL) were added to a reaction flask and heated to 80°C to react for 3 h. The reaction mixture was diluted with water (20 mL), and extracted with ethyl acetate (15 mL×3). The extract was washed with a saturated saline solution (20 mL), dried with anhydrous sodium sulfate, filtered, concentrated under reduced pressure, and separated by silica gel column chromatography (PE:EA (v/v)=5:1 to 1:1) to obtain the target compound.

LC-MS (ESI): [M+H]⁺ =285.0.

### Step 2: Synthesis of tert-butyl (S)-3'-(3-(4-fluoro-2-(((S)-oxetan-2-yl)methyl)-2H-indazol-5-yl)-2-oxo-2,3-dihydro-1H-imidazol-1-yl)-2'-(4-fluoro-3,5-dimethylphenyl)-4'-methyl-2',4'-dihydrospiro[cyclopropane-1,7'-pyrazolo[4,3-c]pyridine]-5'(6'H)-carboxylate

Tert-butyl (*S*)-2'-(4-fluoro-3,5-dimethylphenyl)-4'-methyl-3'-(2-oxo-2,3-dihydro-1*H-*imidazol-1-yl)-2',4'-dihydrospiro[cyclopropane-1,7'-pyrazolo[4,3-c]pyridine]-5'(6'*H*)-carboxylate (50 mg, 0.11 mmol), (S)-5-bromo-4-fluoro-2-(oxetan-2-ylmethyl)-2H-indazole (39 mg, 0.13 mmol), cuprous iodide (16 mg, 0.09 mmol), (1*S*,2*S*)-(+)-*N,N*'-dimethyl-1,2-cyclohexanediamine (12 mg, 0.09 mmol), potassium carbonate (44 mg, 0.32 mmol), and NMP (2 mL) were added to a flask, and heated to 130°C to react for 2 h under nitrogen protection. The reaction mixture was diluted with water (20 mL), and extracted with ethyl acetate (15 mL×3). The extract was washed with a saturated saline solution (20 mL), dried with anhydrous sodium sulfate, filtered, concentrated under reduced pressure, and separated by silica gel column chromatography (PE:EA (v/v)=5:1 to 1:1) to obtain the target compound.

LC-MS (ESI): [M+H]⁺ =672.4.

### Step 3: Synthesis of 1-(4-fluoro-2-(((S)-oxetan-2-yl)methyl)-2H-indazol-5-yl)-3-((S)-2'-(4-fluoro-3,5-dimethylphenyl)-4'-methyl-2',4',5',6'-tetrahydrospiro[cyclopropane-1,7'-pyrazolo[4,3-c]pyridine]-3'-yl)-1,3-dihydro-2H-imidazol-2-one

Tert-butyl (*S*)-3'-(3-(4-fluoro-2-(((*S*)-oxetan-2-yl)methyl)-2*H*-indazol-5-yl)-2-oxo-2,3-dihydro-1*H*-imidazol-1-yl)-2'-(4-fluoro-3,5-dimethylphenyl)-4'-methyl-2',4'-dihydrospiro[cyclopropane-1,7'-pyrazolo[4,3-*c*]pyridine]-5'(6'*H*)-carboxylate (40 mg, 0.09 mmol), 2,6-dimethylpyridine (23 mg, 0.22 mmol) and dichloromethane (1 mL) were added to a reaction flask. A solution of TMSOTf (41 mg, 0.19 mmol) dissolved in dichloromethane was slowly added dropwise at room temperature to react for 10 h after dropwise addition. A saturated sodium bicarbonate solution was added to adjust the pH to be alkaline. The reaction mixture was extracted with ethyl acetate (15 mL×3). The extract was washed with a saturated saline solution (20 mL), dried with anhydrous sodium sulfate, filtered, and concentrated under reduced pressure to obtain the target compound to be used directly for the next step without being purified.

LC-MS (ESI): [M+H]⁺ =572.3.

### Step 4: Synthesis of 3-((1S,2S)-1-(5-(S)-2,2-dimethyltetrahydro-2H-pyran-4-yl)-2-(S)-3'-(3-(4-fluoro-2-(((S)-oxetan-2-yl)methyl)-2H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-1H-indol-1-yl)-2-methylcyclopropyl)-1,2,4-oxadiazol-5(4H)-one

1-(4-fluoro-2-(((*S*)-oxetan-2-yl)methyl)-2*H*-indazol-5-yl)-3-((*S*)-2'-(4-fluoro-3,5-dimethylphenyl)-4'-methyl-2',4',5',6'-tetrahydrospiro[cyclopropane-1,7'-pyrazolo[4,3-c]pyridine]-3'-yl)-1,3-dihydro-2H-imidazol-2-one (40 mg, 0.07 mmol), 5-((S)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1H-indol-2-carboxylic acid (43 mg, 0.1 mmol), EDCI (33 mg, 0.17 mmol), HOAT (14 mg, 0.15 mmol), and DMF (2 mL) were added to a reaction flask. DIPEA (27 mg, 0.21 mmol) was added under stirring to react overnight at room temperature. The reaction mixture was diluted with water (20 mL) and extracted with ethyl acetate (15 mL×3). The extract was washed with a saturated saline solution (20 mL), dried with anhydrous sodium sulfate, filtered, concentrated under reduced pressure, and subjected to preparative HPLC to obtain the target compound.

1H NMR (400 MHz, DMSO-*d*₆) *δ* 11.71 (s, 1H), 8.72 (s, 1H), 7.61 - 7.59 (m, 1H), 7.39 - 7.37 (m, 2H), 7.30 - 7.26 (m, 2H), 7.13 - 7.06 (m, 3H), 6.98 - 6.91 (m, 1H), 6.79 - 6.64 (m, 1H), 5.34 - 5.31 (m, 1H), 5.18 - 5.11 (m, 1H), 4.79 - 4.72 (m, 2H), 4.50 (dd, *J=* 13.6, 7.4 Hz, 1H), 4.33 - 4.26 (m, 1H), 4.18 - 3.99 (m, 1H), 3.76 - 3.70 (m, 3H), 3.09 - 2.97 (m, 1H), 2.74 - 2.66 (m, 1H), 2.48 - 2.37 (m, 1H), 2.24 (s, 6H), 2.03 - 1.96 (m, 1H), 1.81 - 1.47 (m, 7H), 1.36 - 1.30 (m, 3H), 1.27 - 1.23 (m, 7H), 1.14 - 1.10 (m, 4H), 0.93 - 0.75 (m, 1H).

### Example 8 Synthesis of 3-((1S,2S)-1-(5-(S)-2,2-dimethyltetrahydro-2H-pyran-4-yl)-2-(S)-3'-(3-(4-fluoro-1-(((S)-oxetan-2-yl)methyl)-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-1H-indol-1-yl)-2-methylcyclopropyl)-1,2,4-oxadiazol-5(4H)-one (Compound 8)

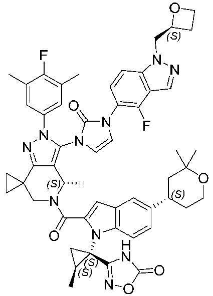

### Step 1: Synthesis of (S)-5-bromo-4-fluoro-1-(oxetan-2-ylmethyl)-1H-indazole

5-bromo-4-fluoro-1H-indazole (200 mg, 0.93 mmol), (S)-4-methylbenzenesulfonic acid oxetan-2-ylmethyl ester (180 mg, 0.74 mmol), cesium carbonate (606 mg, 1.86 mmol), and acetonitrile (4 mL) were added to a reaction and heated to 80°C to react for 3 h. The reaction mixture was diluted with water (20 mL), and extracted with ethyl acetate (15 mL×3). The extract was washed with a saturated saline solution (20 mL), dried with anhydrous sodium sulfate, filtered, concentrated under reduced pressure, and separated by silica gel column chromatography (PE:EA (v/v)=5:1 to 1:1) to obtain the target compound.

LC-MS (ESI): [M+H]⁺ =285.0.

### Step 2: Synthesis of tert-butyl (S)-3'-(3-(4-fluoro-1-(((S)-oxetan-2-yl)methyl)-1H-indazol-5-yl)-2-oxo-2,3-dihydro-1H-imidazol-1-yl)-2'-(4-fluoro-3,5-dimethylphenyl)-4'-methyl-2',4'-dihydrospiro[cyclopropane-1,7'-pyrazolo[4,3-c]pyridine]-5'(6'H)-carboxylate

Tert-butyl (*S*)-2'-(4-fluoro-3,5-dimethylphenyl)-4'-methyl-3'-(2-oxo-2,3-dihydro-1*H-*imidazol-1-yl)-2',4'-dihydrospiro[cyclopropane-1,7'-pyrazolo[4,3-c]pyridine]-5'(6'*H*)-carboxylate (50 mg, 0.11 mmol), (S)-5-bromo-4-fluoro-1-(oxetan-2-ylmethyl)-1H-indazole (39 mg, 0.13 mmol), cuprous iodide (16 mg, 0.09 mmol), (1*S*,2*S*)-(+)-*N,N*'-dimethyl-1,2-cyclohexanediamine (12 mg, 0.09 mmol), potassium carbonate (44 mg, 0.32 mmol), and NMP (2 mL) were added to a reaction flask, and heated to 130°C to react for 2 h under nitrogen protection. The reaction mixture was diluted with water (20 mL), and extracted with ethyl acetate (15 mL×3). The extract was washed with a saturated saline solution (20 mL), dried with anhydrous sodium sulfate, filtered, concentrated under reduced pressure, and separated by silica gel column chromatography (PE:EA (v/v)=5:1 to 1:1) to obtain the target compound.

LC-MS (ESI): [M+H]⁺ =672.4.

### Step 3: Synthesis of 1-(4-fluoro-1-(((S)-oxetan-2-yl)methyl)-1H-indazol-5-yl)-3-((S)-2'-(4-fluoro-3,5-dimethylphenyl)-4'-methyl-2',4',5',6'-tetrahydrospirolcyclopropane-1,7'-pyrazolo[4,3-c]pyridine]-3'-yl)-1,3-dihydro-2H-imidazol-2-one

Tert-butyl (*S*)-3'-(3-(4-fluoro-1-(((*S*)-oxetan-2-yl)methyl)-1*H*-indazol-5-yl)-2-oxo-2,3-dihydro-1*H*-imidazol-1-yl)-2'-(4-fluoro-3,5-dimethylphenyl)-4'-methyl-2',4'-dihydrospiro[cyclopropane-1,7'-pyrazolo[4,3-*c*]pyridine]-5'(6'*H*)-carboxylate (61 mg, 0.09 mmol), 2,6-dimethylpyridine (29 mg, 0.27 mmol), and dichloromethane (1 mL) were added to a reaction flask. A solution of TMSOTf (50 mg, 0.23 mmol) dissolved in dichloromethane was slowly added dropwise at room temperature to react for 10 h after dropwise addition. A saturated sodium bicarbonate solution was added to adjust the pH to be alkaline. The reaction mixture was extracted with ethyl acetate (15 mL×3). The extract was washed with a saturated saline solution (20 mL), dried with anhydrous sodium sulfate, filtered, and concentrated under reduced pressure to obtain the target compound to be used directly for the next step without being purified.

LC-MS (ESI): [M+H]⁺ =572.4.

### Step 4: Synthesis of 3-((1S,2S)-1-(5-(S)-2,2-dimethyltetrahydro-2H-pyran-4-yl)-2-(S)-3'-(3-(4-fluoro-1-(((S)-oxetan-2-yl)methyl)-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-1H-indol-1-yl)-2-methylcyclopropyl)-1,2,4-oxadiazol-5(4H)-one

1-(4-fluoro-1-(((*S*)-oxetan-2-yl)methyl)-1*H*-indazol-5-yl)-3-((*S*)-2'-(4-fluoro-3,5-dimethylphenyl)-4'-methyl-2',4',5',6'-tetrahydrospiro[cyclopropane-1,7'-pyrazolo[4,3-c]pyridine]-3'-yl)-1,3-dihydro-2H-imidazol-2-one (40 mg, 0.07 mmol), 5-((S)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1H-indol-2-carboxylic acid (43 mg, 0.1 mmol), EDCI (33 mg, 0.17 mmol), HOAT (14 mg, 0.15 mmol), and DMF (2 mL) were added to a reaction flask. DIPEA (27 mg, 0.21 mmol) was added under stirring to react overnight at room temperature. The reaction mixture was diluted with water (20 mL) and extracted with ethyl acetate (15 mL×3). The extract was washed with a saturated saline solution (20 mL), dried with anhydrous sodium sulfate, filtered, concentrated under reduced pressure, and subjected to preparative HPLC to obtain the target compound.

¹H NMR (400 MHz, DMSO-*d*₆) *δ* 11.71 (s, 1H), 8.37 (s, 1H), 7.72 (d, *J=* 8.7 Hz, 1H), 7.60 - 7.56 (m, 1H), 7.50 - 7.40 (m, 1H), 7.40 - 7.32 (m, 1H), 7.27 - 7.23 (m, 1H), 7.13 - 7.08 (m, 3H), 6.98 - 6.93 (m, 1H), 6.78 - 6.65 (m, 1H), 5.34 - 5.31 (m, 1H), 5.12 - 5.05 (m, 1H), 4.81 - 4.62 (m, 2H), 4.45 - 4.41 (m, 1H), 4.22 - 4.05 (m, 2H), 3.76 - 3.71 (m, 3H), 3.06 - 3.01 (m, 1H), 2.69 - 2.65 (m, 1H), 2.46 - 2.37 (m, 1H), 2.28 - 2.24 (m, 6H), 2.02 - 1.98 (m, 1H), 1.80 - 1.44 (m, 7H), 1.41 - 1.28 (m, 3H), 1.29 - 1.21 (m, 7H), 1.18 - 1.12 (m, 4H), 0.88 - 0.82 (m, 1H).

### Example 9 Synthesis of 3-((1S,2S)-1-(5-(S)-2,2-dimethyltetrahydro-2H-pyran-4-yl)-2-((S)-3'-(3-(4-fluoro-1-(3-fluorooxetan-3-yl)methyl)-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-1H-indol-1-yl)-2-methylcyclopropyl)-1,2,4-oxadiazol-5(4H)-one (Compound 9)

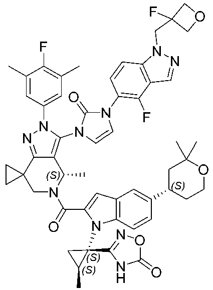

### Step 1: Synthesis of 5-bromo-4-fluoro-1-((3-fluorooxetan-3-yl)methyl)-1H-indazole

5-bromo-4-fluoro-1H-indazole (150 mg, 0.7 mmol), (3-fluorooxetan-3-yl)methyl 4-methylbenzenesulfonate (145 mg, 0.56 mmol), cesium carbonate (454 mg, 1.4 mmol), and acetonitrile (3 mL) were added to a reaction flask and heated to 80°C to react for 2 h. The reaction mixture was diluted with water (20 mL), and extracted with ethyl acetate (15 mL×3). The extract was washed with a saturated saline solution (20 mL), dried with anhydrous sodium sulfate, filtered, concentrated under reduced pressure, and separated by silica gel column chromatography (PE:EA (v/v)=5:1 to 1:1) to obtain the target compound.

LC-MS (ESI): [M+H]⁺ =303.0;
¹H NMR (400 MHz, CDCl₃) δ 8.12 (d, *J =* 1.0 Hz, 1H), 7.52 (dd, *J=* 8.9, 6.2 Hz, 1H), 7.17 (dt, *J=* 8.9, 1.2 Hz, 1H), 4.94 - 4.76 (m, 6H).

### Step 2: Synthesis of (S)-1-(2'-(4-fluoro-3,5-dimethylphenyl)-4'-methyl-2',4',5',6'-tetrahydrospiro[cyclopropane-1,7'-pyrazolo[4,3-c]pyridine]-3'-yl)-1,3-dihydro-2H-imidazol-2-one

Tert-butyl (*S*)-2'-(4-fluoro-3,5-dimethylphenyl)-4'-methyl-3'-(2-oxo-2,3-dihydro-1*H-*imidazol-1-yl)-2',4'-dihydrospiro[cyclopropane-1,7'-pyrazolo[4,3-c]pyridine]-5'(6'*H*)-carboxylate (68 mg, 0.15 mmol) and dioxane hydrochloride (1 mL) were added to a reaction flask to react at room temperature for 1 h, and concentrated under reduced pressure to obtain the target compound.

### Step 3: Synthesis of 3-((1S,2S)-1-(5-(S)-2,2-dimethyltetrahydro-2H-pyran-4-yl)-2-((S)-2'-(4-fluoro-3,5-dimethylphenyl)-4'-methyl-3'-(2-oxo-2,3-dihydro-1H-imidazol-1-yl)-2',4',5',6'-tetrahydrospiro[cyclopropane-1,7'-pyrazolo[4,3-c]pyridine]-5'-carbonyl)-1H-indol-1-yl)-2-methylcyclopropyl)-1,2,4-oxadiazol-5(4H)-one

(*S*)-1-(2'-(4-fluoro-3,5-dimethylphenyl)-4'-methyl-2',4',5',6'-tetrahydrospiro[cyclopropane-1,7'-pyrazolo[4,3-*c*]pyridine]-3'-yl)-1,3-dihydro-2*H*-imidazol-2-one (50 mg, 0.14 mmol), 5-((S)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1H-indol-2-carboxylic acid (73 mg, 0.18 mmol), EDCI (65 mg, 0.34 mmol), HOAT (28 mg, 0.2 mmol), and DMF (2 mL) were added to a reaction flask. DIPEA (352 mg, 2.72 mmol) was added under stirring to react overnight at room temperature. The reaction mixture was diluted with water (20 mL), and extracted with ethyl acetate (15 mL×3). The extract was washed with a saturated saline solution (20 mL), dried with anhydrous sodium sulfate, filtered, concentrated under reduced pressure, and separated by silica gel column chromatography (PE:EA (v/v)=3:1 to 0:1) to obtain the target compound.

### Step 4: Synthesis of 3-((1S,2S)-1-(5-(S)-2,2-dimethyltetrahydro-2H-pyran-4-yl)-2-((S)-3'-(3-(4-fluoro-1-(3-fluorooxetan-3-yl)methyl)-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-1H-indol-1-yl)-2-methylcyclopropyl)-1,2,4-oxadiazol-5(4H)-one

3-((1*S*,2*S*)-1-(5-(*S*)-2,2-dimethyltetrahydro-2*H*-pyran-4-yl)-2-((*S*)-2'-(4-fluoro-3,5-dimethylphenyl)-4'-methyl-3'-(2-oxo-2,3-dihydro-1*H*-imidazol-1-yl)-2',4',5',6'-tetrahydrospiro[cyclopropane-1,7'-pyrazolo[4,3-*c*]pyridine]-5'-carbonyl)-1*H*-indol-1-yl)-2-methylcyclopropyl)-1,2,4-oxadiazol-5(4H)-one (30 mg, 0.04 mmol), 5-bromo-4-fluoro-1-((3-fluorooxetan-3-yl)methyl)-1H-indazole (18 mg, 0.06 mmol), cuprous iodide (6 mg, 0.03 mmol), (1*S*,2*S*)-(+)-*N,N*'-dimethyl-1,2-cyclohexanediamine (4.5 mg, 0.03 mmol), potassium carbonate (16 mg, 0.12 mmol), and NMP (2 mL) were added to a reaction flask, and heated to 130°C to react for 2 h under nitrogen protection. The reaction mixture was diluted with water (20 mL) and extracted with ethyl acetate (15 mL×3). The extract was washed with a saturated saline solution (20 mL), dried with anhydrous sodium sulfate, filtered, concentrated under reduced pressure, and subjected to preparative HPLC to obtain the target compound.

¹H NMR (400 MHz, DMSO-*d*₆) *δ* 11.98 (s, 1H), δ 8.41 (s, 1H), 7.68 (d, *J=* 9.1 Hz, 1H), 7.55 - 7.51 (m, 1H), 7.42 - 7.37 (m, 2H), 7.18 - 6.96 (m, 5H), 6.72 - 6.52 (m, 1H), 5.33 - 5.31 (m, 1H), 5.15 - 5.09 (m, 2H), 4.86 (dd, *J=* 21.4, 8.2 Hz, 2H), 4.65 (dd, *J=* 20.3, 8.2 Hz, 2H), 4.14 - 4.05 (m, 1H), 3.75 - 3.70 (s, 3H), 3.05 - 2.97 (m, 1H), 2.23 (s, 6H), 2.03 - 1.97 (m, 1H), 1.76 - 1.51 (m, 7H), 1.35 - 1.30 (m, 3H), 1.31 - 1.21 (m, 7H), 1.18 - 1.15 (m, 4H), 0.92 - 0.84 (m, 1H).

### Example 10: Synthesis of 3-((1S,2S)-1-(5-((S)-2,2-dimethyltetrahydro-2H-pyran-4-yl)-2-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4,7,7-trimethyl-4,5,6,7-tetrahydro-2H-pyrazolo[4,3-c]pyridine-5-carbonyl)-1H-indol-1-yl)-2-methylcyclopropyl)-1,2,4-oxadiazol-5(4H)-one (Compound 10)

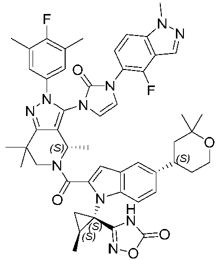

5-((*S*)-2,2-dimethyltetrahydro-2H-pyran-4-yl)-1-((1*S*,2*S*)-2-methyl-1-(5-oxo-4,5-dihydro-1,2,4-oxadiazol-3-yl)cyclopropyl)-1H-indol-2-carboxylic acid (50 mg, 0.12 mmol), (S)-1-(4-fluoro-1-methyl-1H-indazol-5-yl)-3-(2-(4-fluoro-3,5-dimethylphenyl)-4,7,7-trimethyl-4,5,6,7-tetrahydro-2H-pyrazolo[4,3-c]pyridin-3-yl)-1,3-dihydro-2H-imidazol-2-one (75 mg, 0.15 mmol), HOAT (25 mg, 0.18 mmol), EDCI·HCl (58 mg, 0.3 mmol), and DMF (2 mL) were added to a reaction flask. DIPEA (47 mg, 0.36 mmol) was added under stirring to react overnight at room temperature. The reaction mixture was diluted with water (20 mL) and extracted with ethyl acetate (15 mL×3). The organic phases were combined, washed with a saturated saline solution (20 mL), dried with anhydrous sodium sulfate, and separated by silica gel column chromatography (PE:EA (v/v)=2:1 to 1:1) to obtain the target compound.

¹H NMR (400 MHz, DMSO-*d*₆) *δ* 11.72 (s, 1H), 8.34 (s, 1H), 7.67 (d, *J=* 8.9 Hz, 1H), 7.62 (s, 1H), 7.53 - 7.48 (m, 1H), 7.42 (q, *J=* 7.9 Hz, 1H), 7.30 (d, *J= 8.7* Hz, 1H), 7.18 (d, *J=* 6.6 Hz, 2H), 7.00 (s, 1H), 6.80 (d, *J=* 11.6 Hz, 1H), 5.58 (s, 1H), 4.14 (s, 6H), 3.74 (d, *J=* 8.3 Hz, 3H), 3.25 - 2.90 (m, 2H), 2.28 (s, 9H), 1.73 - 1.66 (m, 6H), 1.38 - 1.24 (m, 6H), 1.25 - 1.14 (m, 8H).

### Example 11 Synthesis of 3-((1S,2S))-1-(2-(S)-3'-(3-(1-(difluoromethyl)-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro [cyclopropane-1,7'-pyrazolo [4,3-c]pyridine]-5'-carbonyl)-5-((S)-2,2-dimethyltetrahydro-2H-pyran-4-yl)-1H-indol-1-yl)-2-methylcyclopropyl)-1,2,4-oxadiazol-5(4H)-one (Compound 11)

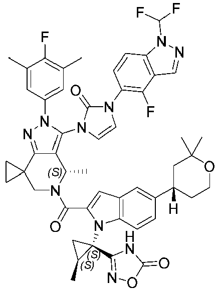

### Step 1: Synthesis of 5-bromo-1-(difluoromethyl)-4-fluoro-1H-indazole

5-bromo-4-fluoro-1H-indazole (500 mg, 2.33 mmol) was dissolved in DCM (10 mL), and a solution of KOH (782.82 mg, 13.95 mmol) in water (1.44 mL) was added dropwise at 0°C. After addition, (chlorodifluoromethyl)trimethylsilane (368.90 mg, 2.33 mmol) was added dropwise. The reaction continued at 0°C for 6 h. The reaction solution was diluted with DCM (5 mL) and H₂O (5 mL) and extracted with DCM (5 mL×3). The organic phases were dried with Na₂SO₄ and filtered, and the filtrate was concentrated under reduced pressure and separated by silica gel column chromatography (PE:EA (v/v)=1:0 to 9:1) to obtain the target compound.

### Step 2: Synthesis of tert-butyl (S)-3'-(3-(1-(difluoromethyl)-4-fluoro-1H-indazol-5-yl)-2-oxo-2,3-dihydro-1H-imidazol-1-yl)-2'-(4-fluoro-3,5-dimethylphenyl)-4'-methyl-2',4'-dihydrospiro[cyclopropane-1,7'-pyrazolo[4,3-c]pyridine]-5'(6'H)-carboxylate

Tert-butyl (S)-2'-(4-fluoro-3,5-dimethylphenyl)-4'-methyl-3'-(2-oxo-2,3-dihydro-1H-imidazol-1-yl)-2',4'-dihydrospiro[cyclopropane-1,7'-pyrazolo[4,3-c]pyridine]-5'(6'*H*)-carboxylate (100 mg, 0.21 mmol), 5-bromo-1-(difluoromethyl)-4-fluoro-1H-indazole (85.03 mg, 0.32 mmol), (1*S*,2*S*)-(+)-*N,N'*-dimethylcyclohexane-1,2-diamine (15.21 mg, 0.11 mmol), cuprous iodide (33.94 mg, 0.11 mmol), potassium carbonate (88.68 mg, 0.64 mmol), and NMP (5 mL) were added to a reaction flask and transferred under nitrogen protection to 130°C to react under stirring for 2 h. The reaction was stopped, a saturated saline solution (10 mL) was added to the reaction solution, and the reaction mixture was extracted with ethyl acetate (5 mL×3). The organic phases were combined, dried with anhydrous sodium sulfate, concentrated under reduced pressure, and separated and purified by silica gel column chromatography (eluent EA:PE (v/v)=20 to 40%) to obtain the target compound.

LC-MS (ESI): [M+H]⁺ =652.3.

### Step 3: Synthesis of (S)-1-(1-(difluoromethyl)-4-fluoro-1H-indazol-5-yl)-3-(2'-(4-fluoro-3,5-dimethylphenyl)-4'-methyl-2',4',5',6'-tetrahydrospiro[cyclopropane-1,7'-pyrazolo[4,3-c]pyridine]-3'-yl)-1,3-dihydro-2H-imidazol-2-one

Tert-butyl (*S*)-3'-(3-(1-(difluoromethyl)-4-fluoro-1*H*-indazol-5-yl)-2-oxo-2,3-dihydro-1*H-*imidazol-1-yl)-2'-(4-fluoro-3,5-dimethylphenyl)-4'-methyl-2',4'-dihydrospiro[cyclopropane-1,7'-pyrazolo[4,3-*c*]pyridine]-5'(6'*H*)-carboxylate (76 mg, 0.12 mmol) was added to a reaction flask, and then a dioxane hydrochloride solution (2 mL) was added to the reaction flask. After addition, the reaction mixture was stirred at room temperature for 1 h, and then distilled and spin dried under reduced pressure to obtain the target compound hydrochloride to be directly used for the next step.

LC-MS (ESI): [M+H]⁺ =552.3.

### Step 4: Synthesis of 3-((1S,2S))-1-(2-(S)-3'-(3-(1-(difluoromethyl)-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-5-((S)-2,2-dimethyltetrahydro-2H-pyran-4-yl)-1H-indol-1-yl)-2-methylcyclopropyl)-1,2,4-oxadiazol-5(4H)-one

5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1H-indol-2-formic acid (80 mg, 0.19 mmol), (S)-1-(1-(difluoromethyl)-4-fluoro-1H-indazol-5-yl)-3-(2'-(4-fluoro-3,5-dimethylphenyl)-4'-methyl-2',4',5',6'-tetrahydrospiro[cyclopropane-1,7'-pyrazolo[4,3-*c*]pyridine]-3'-yl)-1,3-dihydro-2*H-*imidazol-2-one hydrochloride (50 mg, 0.09 mmol), HOAt (17.37 mg, 0.13 mmol), EDCI (40.77 mg, 0.21 mmol), and DMF (5 mL) were added to a reaction flask. DIPEA (219.89 mg, 1.70 mmol) was added under stirring to react overnight at room temperature. A saline solution (10 mL) was added, and the reaction mixture was extracted with ethyl acetate (5 mL×3). The organic phases were combined, dried with anhydrous sodium sulfate, concentrated under reduced pressure, and separated by silica gel column chromatography (PE:EA (v/v)=1:3) to obtain the target compound.

¹H NMR (500 MHz, DMSO-*d*₆) δ 11.66 (s, 1H), 9.20 (d, *J =* 64.5 Hz, 1H), 8.13 (t, *J =* 59.3 Hz, 1H), 7.61 (d, *J=* 68.0 Hz, 2H), 7.38 (d, *J=* 36.6 Hz, 2H), 7.20 (s, 1H), 7.02 (d, *J=* 52.1 Hz, 3H), 6.83 - 6.55 (m, 1H), 5.59 - 5.27 (m, 1H), 3.66 (s, 4H), 2.99 (s, 1H), 2.19 (s, 5H), 1.73 - 1.43 (m, 7H), 1.35 (d, *J= 21.0* Hz, 4H), 1.21 (d, *J =* 15.0 Hz, 6H), 1.13 (s, 5H), 0.84 (d, *J=* 41.3 Hz, 3H).

### Example 12 Synthesis of 3-(1-(7-((S)-2,2-dimethyltetrahydro-2H-pyran-4-yl)-2-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4,7,7-trimethyl-4,5,6,7-tetrahydro-2H-pyrazolo[4,3-c]pyridine-5-carbonyl)indolizin-3-yl)cyclopropyl)-1,2,4-oxadiazol-5(4H)-one (Compound 12)

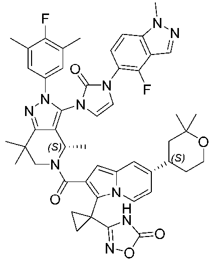

### Step 1: Synthesis of ethyl 7-bromoindolizin-2-carboxylate

Sodium bicarbonate (19.53 g, 232.52 mmol) was added to a solution of 4-bromo-2-methylpyridine (20 g, 116.26 mmol) and ethyl 3-bromopyruvate (34.01 g, 174.39 mmol) in acetonitrile (200 mL) to react at 90°C for 16 h. The reaction mixture was filtered through diatomaceous earth and the filter residue was washed twice with ethyl acetate. The filtrate was washed with water (60 mL×3) and a saturated saline solution (30 mL×2) in sequence, dried with anhydrous sodium sulfate, filtered, concentrated under reduced pressure, and separated by silica gel column chromatography (PE:EA (v/v)=1:0 to 9:1) to obtain the target compound.

LC-MS (ESI): [M+H]⁺= 268.1.

### Step 2: Synthesis of ethyl 7-(2,2-dimethyltetrahydro-2H-pyran-4-yl)indolizin-2-carboxylate

Zinc powder (3.90 g, 59.68 mmol) was added to DMF (20 mL), and nitrogen replacement was performed. The reaction mixture was stirred in an ice water bath. Trimethylchlorosilane (0.81 g, 7.46 mmol) and 1,2-dibromoethane (1.40 g, 7.46 mmol) were added dropwise and stirred at room temperature for 5 min. A solution of 4-iodo-2,2-dimethyltetrahydro-2H-pyran (10.75 g, 44.76 mmol) in DMF (10 mL) was slowly added dropwise under stirring in an ice water bath, and reacted at room temperature for 30 min. Under nitrogen protection, a solution of ethyl 7-bromoindolizin-2-formate (4.00 g, 14.92 mmol), [(4-(N,N-dimethylamino)phenyl]di-tert-butylphosphine (0.40 g, 1.49 mmol), and palladium acetate (0.33 g, 1.49 mmol) dissolved in DMF (10 mL) was added and heated to 50°C to react for 1 h. The reaction mixture was stirred in an ice water bath, and 5N dilute hydrochloric acid was added dropwise to adjust the pH to be acidic (12 mL). The reaction mixture was diluted with a saturated saline solution (60 mL), dissolved in ethyl acetate (60 mL), and filtered through diatomaceous earth. The filtrate was extracted with ethyl acetate (120 mL×3), and the organic phases were collected, washed with a saturated saline solution (120 mL×3), dried with anhydrous sodium sulfate, concentrated under reduced pressure, and separated by silica gel column chromatography (PE:EA (v/v)=1:0 to 9:1) to obtain the target compound.

LC-MS (ESI): [M+H]⁺ = 302.3;
¹H NMR (500 MHz, DMSO-*d*₆) δ 8.21 (d, *J=* 7.3 Hz, 1H), 7.99 (d, *J=* 1.7 Hz, 1H), 7.21 (s, 1H), 6.65 - 6.59 (m, 2H), 4.25 (q, *J=* 7.1 Hz, 2H), 3.74 - 3.66 (m, 2H), 2.89 - 2.81 (m, 1H), 1.69 - 1.62 (m, 2H), 1.55 - 1.38 (m, 2H), 1.30 (t, *J= 7.1* Hz, 3H), 1.24 (s, 3H), 1.18 (s, 3H).

### Step 3: Synthesis of ethyl (S)-7-(2,2-dimethyltetrahydro-2H-pyran-4-yl)indolizin-2-carboxylate

By supercritical fluid chromatography, stereoisomers in the compound ethyl 7-(2,2-dimethyltetrahydro-2H-pyran-4-yl)indolizin-2-carboxylate (4.98 g) synthesized by the method in step 2 were separated, and the postpeak was taken to obtain the target compound.

### Step 4: Synthesis of ethyl (S)-3-(cyanomethyl)-7-(2,2-dimethyltetrahydro-2H-pyran-4-yl)indolizin-2-carboxylate

Ethyl (S)-7-(2,2-dimethyltetrahydro-2H-pyran-4-yl)indolizin-2-carboxylate (2.7 g, 8.96 mmol), 2-bromoacetonitrile (1.18 g, 9.85 mmol), ferrous sulfate heptahydrate (1.25 g, 4.48 mmol), and NaI (1.34 g, 8.96 mmol) were added to DMSO (30 mL). 30% hydrogen peroxide (4.6 mL, 8.96 mmol) was added dropwise at 0°C to react at 0°C for 10 min after dropwise addition. The reaction mixture was diluted with water (50 mL) and extracted with ethyl acetate (20 mL×3). The organic phases were combined, washed with water (20 mL×3) and a saturated saline solution (20 mL×2) in sequence, dried with anhydrous sodium sulfate, filtered, concentrated under reduced pressure, and separated by silica gel column chromatography (PE:EA (v/v)=1:0 to 3:2) to obtain the target compound.

LC-MS (ESI): [M+H]⁺= 341.3.

### Step 5: Synthesis of ethyl (S)-3-(1-cyanocyclopropyl)-7-(2,2-dimethyltetrahydro-2H-pyran-4-yl)indolizin-2-carboxylate

The compound ethyl (S)-3-(cyanomethyl)-7-(2,2-dimethyltetrahydro-2H-pyran-4-yl)indolizin-2-carboxylate (1.5 g, 4.41 mmol) and 1,3,2-dioxathiocyclopentane 2,2-dioxide (1.64 g, 13.22 mmol) were added to THF (10 mL). LiHMDS (39.7 mL, 39.66 mmol) was added dropwise at 0°C to react at 0°C for 2 h. The reaction solution was added to water (30 mL) and extracted with ethyl acetate (20 mL×3), and the organic phases were combined. The organic phase was washed with water (20 mL×3) and a saturated saline solution (20 mL×2) in sequence, dried with anhydrous sodium sulfate, filtered, concentrated under reduced pressure, and separated by silica gel column chromatography (PE:EA (v/v)=1:0 to 3:2) to obtain the target compound.

LC-MS (ESI): [M+H]⁺=367.4.

### Step 6: Synthesis of (S)-3-(1-cyanocyclopropyl)-7-(2,2-dimethyltetrahydro-2H-pyran-4-yl)indolizin-2-carboxylic acid

The compound ethyl (S)-3-(1-cyanocyclopropyl)-7-(2,2-dimethyltetrahydro-2H-pyran-4-yl)indolizin-2-carboxylate (1.58 g, 4.31 mmol) and lithium hydroxide monohydrate (0.9 g, 21.56 mmol) were added to 1,4-dioxane (12 mL) and water (6 mL) to react at 40°C for 2 h. The pH was adjusted to 4 with 2N dilute hydrochloric acid in an ice bath. Water (30 mL) was added, and the reaction mixture was extracted with ethyl acetate (15 mL×3). The organic phases were combined, dried with anhydrous sodium sulfate, concentrated under reduced pressure, and separated by silica gel column chromatography (MeOH:DCM (v/v)= 5%) to obtain the target compound.

LC-MS (ESI): [M+H]⁺= 339.3.

### Step 7: Synthesis of ethyl 2,2-dimethyl-3-oxopropionate

Ethyl 3-hydroxy-2,2-dimethylpropionate (5 g, 34.2 mmol) and dichloromethane (100 mL) were added in sequence to a reaction flask. Dess-Martin periodinane (21.76 g, 51.3 mmol) was added in batches at 0°C, and then the reaction mixture was heated to room temperature and stirred for 2 h. In an ice bath, the reaction was quenched with a saturated sodium thiosulfate solution, and then saturated sodium bicarbonate was added to neutralize a large amount of acetic acid. The reaction mixture was extracted with dichloromethane (100 mL×3). The organic phases were combined, washed with a saturated saline solution (100 mL), dried with anhydrous sodium sulfate, filtered, concentrated under reduced pressure, and separated by silica gel column chromatography (PE:EA (v/v)=10:1 to 5:1) to obtain the target compound.

### Step 8: Synthesis of ethyl (S)-3-((1-cyanopropan-2-yl)amino)-2,2-dimethylpropionate

(*S*)-3-aminobutanenitrile hydrochloride (300 mg, 2.49 mmol), methanol (10 mL), and diisopropylethylamine (322 mg, 2.49 mmol) were added in sequence to a reaction flask, and the reaction solution was stirred for 20 min. Then, acetic acid (134 mg, 2.24 mmol) and ethyl 2,2-dimethyl-3-oxopropionate (470 mg, 3.26 mmol) were added and stirred for 2 h after addition. Then, sodium triacetoxyborohydride (1.05 g, 4.98 mmol) was added and stirred at room temperature for 6 h. The reaction mixture was diluted with water and extracted with ethyl acetate (3×30 mL). The organic phases were combined, washed with a saturated sodium bicarbonate solution and a saturated saline solution (50 mL), dried with anhydrous sodium sulfate, filtered, and concentrated under reduced pressure to obtain the target compound to be direct used for the next step.

### Step 9: Synthesis of ethyl (S)-3-((tert-butoxycarbonyl)(1-cyanopropan-2-yl)amino)-2,2-dimethylpropionate

The crude product of ethyl (*S*)-3-((1-cyanopropan-2-yl)amino)-2,2-dimethylpropionate and di-tert-butyl dicarbonate (2 mL) were added in sequence to a reaction flask. The reaction solution was stirred at room temperature overnight after addition, concentrated under reduced pressure, and separated by silica gel column chromatography (PE:EA (v/v)=10:1 to 5:1) to obtain the target compound.

LC-MS (ESI): [M+H-Boc]⁺ =213.1.

### Step 10: Synthesis of tert-butyl (2S)-3-cyano-2,5,5-trimethyl-4-oxopiperidine-1-formate

Ethyl (*S*)-3-((tert-butoxycarbonyl)(1-cyanopropan-2-yl)amino)-2,2-dimethylpropionate (263 mg, 0.84 mmol), tetrahydrofuran (6 mL), and a solution of 1 M potassium tert-butoxide (1.3 mL, 1.26 mmol) in THF were added in sequence to a reaction flask. After addition, the reaction mixture was stirred at room temperature for 1 h, quenched with a saturated ammonium chloride solution, and extracted with ethyl acetate (3×15 mL). The organic phases were combined, washed with a saturated saline solution (30 mL), dried with anhydrous sodium sulfate, filtered, and concentrated under reduced pressure to obtain the target compound to be used directly for the next step.

LC-MS (ESI): [M+H]⁺ =267.3.

### Step 11: Synthesis of tert-butyl (S)-3-amino-2-(4-fluoro-3,5-dimethylphenyl)-4,7,7-trimethyl-2,4,6,7-tetrahydro-5H-pyrazolo[4,3-c]pyridine-5-carboxylate

(4-fluoro-3,5-dimethylphenyl)hydrazine (200 mg, 1.3 mmol), tert-butyl (2*S*)-3-cyano-2,5,5-trimethyl-4-oxopiperidine-1-formate (173 mg, 0.65 mmol), and ethanol (10 mL) were added to a reaction flask. A dioxane hydrochloride solution (0.16 mL) was added under nitrogen protection. The reaction mixture was heated to 80°C to react for 1.5 h. A saturated saline solution was added, and the reaction mixture was extracted with ethyl acetate. The organic phases were combined, dried with anhydrous sodium sulfate, and separated by silica gel column chromatography (PE:EA (v/v)=9:1 to 2:1) to obtain the target compound.

LC-MS (ESI): [M+H]⁺ =403.4.

### Step 12: Synthesis of tert-butyl (S)-3-(3-(2,2-dimethoxyethyl)ureido)-2-(4-fluoro-3,5-dimethylphenyl)-4,7,7-trimethyl-2,4,6,7-tetrahydro-5H-pyrazolo[4,3-c]pyridine-5-carboxylate (813-6)

Tert-butyl (*S*)-3-amino-2-(4-fluoro-3,5-dimethylphenyl)-4,7,7-trimethyl-2,4,6,7-tetrahydro-5*H*-pyrazolo[4,3-*c*]pyridine-5-carboxylate (173 mg, 0.43 mmol), DIPEA (277 mg, 2.15 mmol), and tetrahydrofuran (6 mL) were added to a reaction flask. Phenyl chloroformate (134 mg, 0.86 mmol) was added dropwise under stirring to react at room temperature for 1 h. 2,2-dimethoxyethane-1-amine (181 mg, 1.72 mmol) was added and stirred for 1 h. A saturated saline solution was added, and the reaction mixture was extracted with ethyl acetate. The organic phases were combined, dried with anhydrous sodium sulfate, and separated by silica gel column chromatography (PE:EA (v/v)=2:1 to 1:1) to obtain the target compound.

LC-MS (ESI): [M+H]⁺ =534.4.

### Step 13: Synthesis of tert-butyl (S)-2-(4-fluoro-3,5-dimethylphenyl)-4,7,7-trimethyl-3-(2-oxo-2,3-dihydro-1H-imidazol-1-yl)-2,4,6,7-tetrahydro-5H-pyrazolo[4,3-c]pyridine-5-carboxylate

Tert-butyl (*S*)-3-(3-(2,2-dimethoxyethyl)ureido)-2-(4-fluoro-3,5-dimethylphenyl)-4,7,7-trimethyl-2,4,6,7-tetrahydro-5*H*-pyrazolo[4,3-c]pyridine-5-carboxylate (213 mg, 0.4 mmol), methanesulfonic acid (34.52 mg, 0.36 mmol), and tetrahydrofuran (5 mL) were added to a reaction flask, and heated to react for 1 h. A saturated saline solution was added, and the reaction mixture was extracted with ethyl acetate. The organic phases were combined, dried with anhydrous sodium sulfate, and separated by silica gel column chromatography (PE:EA (v/v)=2:1 to 1:1) to obtain the target compound.

LC-MS (ESI): [M+H]⁺ =470.3.

### Step 14: Synthesis of tert-butyl (S)-3-(3-(4-fluoro-1-methyl-1H-indazol-5-yl)-2-oxo-2,3-dihydro-1H-imidazol-1-yl)-2-(4-fluoro-3,5-dimethylphenyl)-4,7,7-trimethyl-2,4,6,7-tetrahydro-5H-pyrazolo[4,3-c]pyridine-5-carboxylate

Tert-butyl (*S*)-2-(4-fluoro-3,5-dimethylphenyl)-4,7,7-trimethyl-3-(2-oxo-2,3-dihydro-1*H-*imidazol-1-yl)-2,4,6,7-tetrahydro-5*H*-pyrazolo[4,3-*c*]pyridine-5-carboxylate (190 mg, 0.4 mmol), 5-bromo-4-fluoro-1-methyl-1*H*-indole (120 mg, 0.53 mmol), cuprous iodide (64.2 mg, 0.2 mmol), (1*S*,2*S*)-(+)-*N,N'*-dimethyl-1,2-cyclohexanediamine (29 mg, 0.2 mmol), and NMP (5 mL) were added to a reaction flask, and heated to 130°C to react for 2 h under nitrogen protection. A saturated saline solution was added, and the reaction mixture was extracted with ethyl acetate. The organic phases were combined, dried with anhydrous sodium sulfate, and separated by silica gel column chromatography (PE:EA (v/v)=2:1 to 1:1) to obtain the target compound.

LC-MS (ESI): [M+H]⁺ =618.4.

### Step 15: Synthesis of (S)-1-(4-fluoro-1-methyl-1H-indazol-5-yl)-3-(2-(4-fluoro-3,5-dimethylphenyl)-4,7,7-trimethyl-4,5,6,7-tetrahydro-2H-pyrazolo[4,3-c]pyridin-3-yl)-1,3-dihydro-2H-imidazol-2-one

Tert-butyl (*S*)-3-(3-(4-fluoro-1-methyl-1*H*-indazol-5-yl)-2-oxo-2,3-dihydro-1*H*-imidazol-1-yl)-2-(4-fluoro-3,5-dimethylphenyl)-4,7,7-trimethyl-2,4,6,7-tetrahydro-5*H*-pyrazolo[4,3-*c*]pyridine-5-carboxylate (250 mg, 0.4 mmol) and a 4N dioxane hydrochloride solution (5.1 mL) were added to a reaction flask to react at room temperature for 1 h, and the reaction mixture was directly concentrated under reduced pressure to obtain the target compound hydrochloride.

LC-MS (ESI): [M+H]⁺ =518.4.

### Step 16: Synthesis of 1-(7-((S)-2,2-dimethyltetrahydro-2H-pyran-4-yl)-2-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4,7,7-trimethyl-4,5,6,7-tetrahydro-2H-pyrazolo[4,3-c]pyridine-5-carbonyl)indolizin-3-yl)cyclopropane-1-nitrile

DIPEA (764 mg, 5.91 mmol) was added to a solution of (*S*)-3-(1-cyanocyclopropyl)-7-(2,2-dimethyltetrahydro-2*H*-pyran-4-yl)indolizin-2-carboxylic acid (100 mg, 0.30 mmol), (*S*)-1-(4-fluoro-1-methyl-1*H*-indazol-5-yl)-3-(2-(4-fluoro-3,5-dimethylphenyl)-4,7,7-trimethyl-4,5,6,7-tetrahydro-2*H*-pyrazolo[4,3-c]pyridin-3-yl)-1,3-dihydro-2H-imidazol-2-one hydrochloride (166 mg, 0.30 mmol), and HATU (169 mg, 0.44 mmol) in DMF (4 mL), and reacted at room temperature for 1 h. The reaction mixture was diluted with ethyl acetate (20 mL), washed with a saline solution (10 mL×2), dried with anhydrous sodium sulfate, filtered, concentrated under reduced pressure, and separated by silica gel column chromatography (PE:EA (v/v)=1:1 to 1:4) to obtain the target compound.

LC-MS (ESI): [M+H]⁺ =838.6.

### Step 17: Synthesis of (Z)-1-(7-((S)-2,2-dimethyltetrahydro-2H-pyran-4-yl)-2-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4,7,7-trimethyl-4,5,6,7-tetrahydro-2H-pyrazolo[4,3-c]pyridine-5-carbonyl)indolizin-3-yl)-N'-hydroxycyclopropane-1-carboxamidine

Sodium bicarbonate (281 mg, 3.34 mmol) and hydroxylamine hydrochloride (232 mg, 3.34 mmol) were added to a solution of 1-(7-((*S*)-2,2-dimethyltetrahydro-2*H*-pyran-4-yl)-2-((*S*)-3-(3-(4-fluoro-1-methyl-1*H*-indazol-5-yl)-2-oxo-2,3-dihydro-1*H*-imidazol-1-yl)-2-(4-fluoro-3,5-dimethylphenyl)-4,7,7-trimethyl-4,5,6,7-tetrahydro-2H-pyrazolo[4,3-*c*]pyridine-5-carbonyl)indolizin-3-yl)cyclopropane-1-nitrile (140 mg, 0.17 mmol) in DMSO (10 mL), and reacted at 60°C for 16 h. The reaction mixture was washed with a saturated saline solution (10 mL×2), dried with anhydrous sodium sulfate, filtered, concentrated under reduced pressure, and separated by silica gel column chromatography (PE:EA (v/v)=1:1 to 1:4) to obtain the target compound.

LC-MS (ESI): [M+H]⁺ =871.6.

### Step 18: Synthesis of 3-(1-(7-((S)-2,2-dimethyltetrahydro-2H-pyran-4-yl)-2-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4,7,7-trimethyl-4,5,6,7-tetrahydro-2H-pyrazolo[4,3-c]pyridine-5-carbonyl)indolizin-3-yl)cyclopropyl)-1,2,4-oxadiazol-5(4H)-one

The compound (*Z*)-1-(7-((*S*)-2,2-dimethyltetrahydro-2*H*-pyran-4-yl)-2-((*S*)-3-(3-(4-fluoro-1-methyl-1*H*-indazol-5-yl)-2-oxo-2,3-dihydro-1*H*-imidazol-1-yl)-2-(4-fluoro-3,5-dimethylphenyl)-4,7,7-trimethyl-4,5,6,7-tetrahydro-2*H*-pyrazolo[4,3-*c*]pyridine-5-carbonyl)indolizin-3-yl)-*N*'-hydroxycyclopropane-1-carboxamidine (116 mg, 0.13 mmol), CDI (43 mg, 0.19 mmol), and DBU (51 mg, 0.33 mmol) were added to DMSO (10 mL), and reacted at room temperature for 2 h. The reaction mixture was diluted with water (15 mL) and extracted with ethyl acetate (8 mL×3), and the organic phases were combined. The organic phase was washed with water (10 mL×3) and a saturated saline solution (10 mL×2) in sequence, dried with anhydrous sodium sulfate, filtered, concentrated under reduced pressure, and separated by silica gel column chromatography (PE:EA (v/v)=1:4 to 0:1) to obtain the target compound.

LC-MS (ESI): [M+H]⁺ =897.6;
¹H NMR (400 MHz, DMSO-*d*₆) δ 8.32(s,1H), 8.18 (d, *J =* 7.0 Hz, 1H), 7.64 (d, *J =* 8.8 Hz, 1H), 7.62 - 7.43 (m, 1H), 7.34 (s, 1H), 7.16 (d, *J =* 6.3 Hz, 2H), 7.08 (s, 2H), 6.92 (s, 1H), 6.73 (dd, *J* = 7.4, 1.8 Hz, 1H), 6.41 (d, *J =* 32.5 Hz, 1H), 5.75 (s, 1H), 4.11 (d, *J =* 14.6 Hz, 3H), 3.73 (d, *J* = 9*.*4 Hz, 2H), 3.25 - 3.15 (m, 1H), 2.34 - 2.05 (m, 12H), 1.70 (s, 4H), 1.42 - 1.26 (m, 9H), 1.29 - 1.15 (m, 6H).

### Example 13 Synthesis of 3-(1-(2-(((S)-3'-(3-(1-cyclopropyl-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridin]-5'-carbonyl)-7-((S)-2,2-dimethyltetrahydro-2H-pyran-4-yl)indolizin-3-yl)cyclopropyl)-1,2,4-oxadiazol-5(4H)-one (Compound 13)

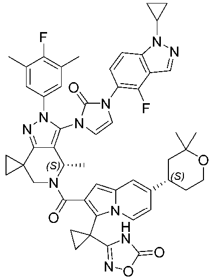

### Step 1: Synthesis of 1-(2-((S)-3'-(3-(1-cyclopropyl-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-7-((S)-2,2-dimethyltetrahydro-2H-pyran-4-yl)indolizin-3-yl)cyclopropane-1-carbonitrile

DIPEA (86 mg, 0.66 mmol) was added to a solution of (*S*)-1-(1-cyclopropyl-4-fluoro-1*H-*indazol-5-yl)-3-(2'-(4-fluoro-3,5-dimethylphenyl)-4'-methyl-2',4',5',6'-tetrahydrospiro[cyclopropane-1,7'-pyrazolo[4,3-c]pyridine]-3'-yl)-1,3-dihydro-2*H*-imidazol-2-one (120 mg, 0.22 mmol), (*S*)-3-(1-cyanocyclopropyl)-7-(2,2-dimethyltetrahydro-2*H*-pyran-4-yl)indolizin-2-carboxylic acid (98 mg, 0.29 mmol), and HATU (126 mg, 0.33 mmol) in DMF (2 mL), and reacted overnight at room temperature. The reaction mixture was diluted with water (20 mL) and extracted with ethyl acetate (15 mL×3). The extract was washed with a saturated saline solution, dried with anhydrous sodium sulfate, filtered, concentrated under reduced pressure, and separated by silica gel column chromatography (PE:EA (v/v)=5:1 to 1:1) to obtain the target compound.

LC-MS (ESI): [M+H]⁺ =862.6.

### Step 2: Synthesis of (Z)-1-(2-((S)-3'-(3-(1-cyclopropyl-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-7-((S)-2,2-dimethyltetrahydro-2H-pyran-4-yl)indolizin-3-yl)-N'-hydroxycyclopropane-1-carboxamidine

Sodium bicarbonate (253 mg, 3.02 mmol) and hydroxylamine hydrochloride (210 mg, 3.02 mmol) were added to a solution of 1-(2-((S)-3'-(3-(1-cyclopropyl-4-fluoro-1*H*-indazol-5-yl)-2-oxo-2,3-dihydro-1*H*-imidazol-1-yl)-2'-(4-fluoro-3,5-dimethylphenyl)-4'-methyl-2',4',5',6'-tetrahydrospiro[cyclopropane-1,7'-pyrazolo[4,3-*c*]pyridine]-5'-carbonyl)-7-((*S*)-2,2-dimethyltetrahydro-2*H*-pyran-4-yl)indolizin-3-yl)cyclopropane-1-carbonitrile (130 mg, 0.15 mmol) in DMSO (4 mL), and reacted at 60°C for 16 h. The reaction mixture was diluted with water (30 mL) and extracted with ethyl acetate (15 mL×3). The extract was washed with a saturated saline solution, dried with anhydrous sodium sulfate, filtered, concentrated under reduced pressure, and separated by silica gel column chromatography (PE:EA (v/v)=5:1 to 1:1) to obtain the target compound.

### Step 3: Synthesis of 3-(1-(2-(((S)-3'-(3-(1-cyclopropyl-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridin]-5'-carbonyl)-7-((S)-2,2-dimethyltetrahydro-2H-pyran-4-yl)indolizin-3-yl)cyclopropyl)-1,2,4-oxadiazol-5(4H)-one

The compound (*Z*)-1-(2-((*S*)-3'-(3-(1-cyclopropyl-4-fluoro-1*H*-indazol-5-yl)-2-oxo-2,3-dihydro-1*H*-imidazol-1-yl)-2'-(4-fluoro-3,5-dimethylphenyl)-4'-methyl-2',4',5',6'-tetrahydrospiro[cyclopropane-1,7'-pyrazolo[4,3-*c*]pyridine]-5'-carbonyl)-7-((*S*)-2,2-dimethyltetrahydro-2*H-*pyran-4-yl)indolizin-3-yl)-*N*'-hydroxycyclopropane-1-carboxamidine (89 mg, 0.13 mmol), CDI (32 mg, 0.2 mmol), and DBU (38 mg, 0.25 mmol) were added to DMSO (5 mL), and reacted at room temperature for 2 h. The reaction mixture was diluted with water (30 mL) and extracted with ethyl acetate (15 mL×3). The extract was washed with a saturated saline solution, dried with anhydrous sodium sulfate, filtered, concentrated under reduced pressure, and separated by silica gel column chromatography (PE:EA (v/v)=3:1 to 0:1) to obtain the target compound.

¹H NMR (500 MHz, DMSO-*d*₆) *δ* 12.00 (s, 1H), 8.31 - 8.09 (m, 2H), 7.76 - 7.62 (m, 1H), 7.62 - 7.45 (m, 1H), 7.36 - 7.26 (m, 1H), 7.17 - 7.02 (m, 2H), 7.00 - 6.82 (m, 1H), 6.74 - 6.62 (m, 1H), 6.49 - 6.30 (m, 1H), 4.90 - 4.85 (m, 1H), 3.97 - 3.80 (m, 2H), 3.74 - 3.66 (m, 2H), 3.42 - 3.38 (m, 1H), 2.95 - 2.91 (m, 1H), 2.26 - 2.17 (m, 6H), 1.73 - 1.62 (m, 3H), 1.53 - 1.38 (m, 4H), 1.33 - 1.24 (m, 11H), 1.20 - 1.12 (m, 6H), 0.87 - 0.84 (m, 2H).

### Example 14 Synthesis of 3-(1-(7-(S)-2,2-dimethyltetrahydro-2H-pyran-4-yl)-2-((S)-3'-(3-(4-fluoro-1-methyl-indazol-5-yl)-2-oxo-2,3-dihydro-1H-imidazol-1-yl)-2'-(4-fluoro-3,5-dimethylphenyl)-4'-methyl-2',4',5',6'-tetrahydrospirolcyclopropane-1,7'-pyrazolo[4,3-c]pyridine]-5'-carbonyl)indolizin-3-yl)cyclopropyl)-1,2,4-oxadiazol-5(4H)-one (Compound 14)

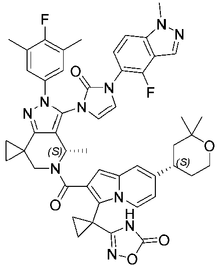

### Step 1: Synthesis of 1-(7-((S)-2,2-dimethyltetrahydro-2H-pyran-4-yl)-2-(S)-3'-(3-(4-fluoro-1-methyl-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-7'-pyrazolo[4,3]pyridine]-5'-carbonyl)indolizin-3-yl)cyclopropane-1-carbonitrile

DIPEA (100 mg, 0.78 mmol) was added to a solution of (*S*)-1-(4-fluoro-1-methyl-1*H*-indazol-5-yl)-3-(2'-(4-fluoro-3,5-dimethylphenyl)-4'-methyl-2',4',5',6'-tetrahydrospiro[cyclopropane-7'-pyrazolo[4,3-*c*]pyridine]-3'-yl)-1,3-dihydro-2*H*-imidazol-2-one (160 mg, 0.31 mmol), (*S*)-1-(*S*)-3-(1-cyanocyclopropyl)-7-(2,2-dimethyltetrahydro-2*H*-pyran-4-yl)indolizin-2-carboxylic acid (105 mg, 0.31 mmol), and HATU (177 mg, 0.47 mmol) in DMF (2 mL), and reacted overnight at room temperature. The reaction mixture was diluted with water (30 mL) and extracted with ethyl acetate (15 mL×3). The extract was washed with a saturated saline solution (20 mL×2), dried with anhydrous sodium sulfate, filtered, concentrated under reduced pressure, and separated by silica gel column chromatography (PE:EA (v/v)=5:1 to 1:1) to obtain the target compound.

LC-MS (ESI): [M+H]⁺ =836.6.

### Step 2: Synthesis of (Z)-1-(7-((S)-2,2-dimethyltetrahydro-2H-pyran-4-yl)-2-(S)-3'-(3-(4-fluoro-1-methyl-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-7'-pyrazolo[4,3-c]pyridine]-5'-carbonyl)indolizin-3-yl)-N'-hydroxycyclopropane-1-carboxamidine

Sodium bicarbonate (271 mg, 3.23 mmol) and hydroxylamine hydrochloride (225 mg, 3.23 mmol) were added to a solution of 1-(7-((*S*)-2,2-dimethyltetrahydro-2H-pyran-4-yl)-2-(*S*)-3'-(3-(4-fluoro-1-methyl-1*H*-indazol-5-yl)-2-oxo-2,3-dihydro-1*H*-imidazol-1-yl)-2'-(4-fluoro-3,5-dimethylphenyl)-4'-methyl-2',4',5',6'-tetrahydrospiro[cyclopropane-7'-pyrazolo[4,3]pyridine]-5'-carbonyl)indolizin-3-yl)cyclopropane-1-carbonitrile (135 mg, 0.16 mmol) in DMSO (5 mL), and reacted at 60°C for 16 h. The reaction mixture was diluted with water (30 mL) and extracted with ethyl acetate (15 mL×3). The extract was washed with a saturated saline solution (20 mL×2), dried with anhydrous sodium sulfate, filtered, concentrated under reduced pressure, and separated by silica gel column chromatography (PE:EA (v/v)=5:1 to 1:1) to obtain the target compound.

LC-MS (ESI): [M+H]⁺ =869.9.

### Step 3: Synthesis of 3-(1-(7-(S)-2,2-dimethyltetrahydro-2H-pyran-4-yl)-2-((S)-3'-(3-(4-fluoro-1-methyl-indazol-5-yl)-2-oxo-2,3-dihydro-1H-imidazol-1-yl)-2'-(4-fluoro-3,5-dimethylphenyl)-4'-methyl-2',4',5',6'-tetrahydrospirolcyclopropane-1,7'-pyrazolo[4,3-c]pyridine]-5'-carbonyl)indolizin-3-yl)cyclopropyl)-1,2,4-oxadiazol-5(4H)-one

The compound (*Z*)-1-(7-((*S*)-2,2-dimethyltetrahydro-2*H*-pyran-4-yl)-2-(*S*)-3'-(3-(4-fluoro-1-methyl-1*H*-indazol-5-yl)-2-oxo-2,3-dihydro-1*H*-imidazol-1-yl)-2'-(4-fluoro-3,5-dimethylphenyl)-4'-methyl-2',4',5',6'-tetrahydrospiro[cyclopropane-7'-pyrazolo[4,3-c]pyridine]-5'-carbonyl)indolizin-3-yl)-*N*'-hydroxycyclopropane-1-carboxamidine (110 mg, 0.13 mmol), CDI (41 mg, 0.25 mmol), and DBU (48 mg, 0.32 mmol) were added to DMSO (5 mL), and reacted at room temperature for 2 h. The reaction mixture was diluted with water (30 mL) and extracted with ethyl acetate (15 mL×3). The extract was washed with a saturated saline solution (20 mL×2), dried with anhydrous sodium sulfate, filtered, concentrated under reduced pressure, and separated by silica gel column chromatography (PE:EA (v/v)=3:1 to 0:1) to obtain the target compound.

¹H NMR (500 MHz, DMSO-*d*₆) δ 11.98 (s, 1H), 8.30 (m, 2H), 7.66 - 7.61 (m, 1H), 7.55 - 7.41 (m, 1H), 7.35 - 7.21 (m, 1H), 7.19 - 7.03 (m, 2H), 6.99 - 6.83 (m, 1H), 6.75 - 6.66 (m, 1H), 6.46 - 6.24 (m, 1H), 5.82 (s, 1H), 4.13 - 4.06 (m, 3H), 3.74 - 3.68 (m, 2H), 3.48 - 3.32 (m, 2H), 2.94 - 2.91 (m, 1H), 2.25 - 2.18 (m, 6H), 1.79 - 1.59 (m, 3H), 1.55 - 1.35 (m, 4H), 1.31 - 1.24 (m, 9H), 1.19 - 1.16 (m, 4H), 0.85 - 0.82 (m, 2H).

### Example 15 Synthesis of 3-(1-(2-((S)-3'-(3-(1-cyclopropyl-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-5-((S)-2,2-dimethyltetrahydro-2H-pyran-4-yl)-1H-indol-1-yl)cyclopropyl)-1,2,4-oxadiazol-5(4H)-one (Compound 15)

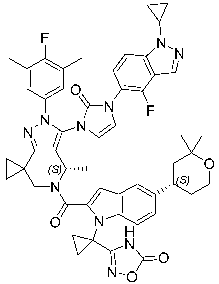

### Step 1: Synthesis of (S)-5-(2,2-dimethyltetrahydro-2H-pyran-4-yl)-1H-indol-2-carboxylic acid

The compound ethyl (*S*)-5-(2,2-dimethyltetrahydro-2*H*-pyran-4-yl)-1*H*-indol-2-carboxylate (1.80 g, 5.97 mmol) was dissolved in methanol (18 mL). A solution of sodium hydroxide (0.60 g, 14.93 mmol) in water (4.5 mL) was added dropwise under stirring, and reacted at 65°C for 2 h. The reaction mixture was cooled to room temperature and the pH was adjusted to about 5 with 6N hydrochloric acid. A large amount of solid was separated out and filtered, and the filter cake was washed with water and dried to obtain the target compound.

LC-MS (ESI): [M+H]⁺=274.1.

### Step 2: Synthesis of (S)-5-(2,2-dimethyltetrahydro-2H-pyran-4-yl)-N-methyl-N-phenyl-1H-indol-2-carboxamide

The compound (*S*)-5-(2,2-dimethyltetrahydro-2*H*-pyran-4-yl)-1*H*-indol-2-carboxylic acid (1.50 g, 5.49 mmol) was dissolved in *N*,*N*-dimethylacetamide (15 mL). Thionyl chloride (0.78 g, 6.59 mmol) was added dropwise under stirring at 0°C, and reacted at room temperature for 1 h. *N-*methylaniline (0.71 g, 6.59 mmol) and triethylamine (1.67 g, 16.46 mmol) were added under stirring at 0°C, and reacted at room temperature for 1 h. The reaction was quenched with water (25 mL). A large amount of solid was separated out and filtered, and the filter cake was washed with water and dried to obtain the target compound.

LC-MS (ESI): [M+H]⁺ =363.3.

### Step 3: Synthesis of (S)-1-(cyanomethyl)-5-(2,2-dimethyltetrahydro-2H-pyran-4-yl)-N-methyl-N-phenyl-1H-indol-2-carboxamide

A 8N potassium hydroxide aqueous solution (2.0 mL, 15.73 mmol) was added to a solution of (*S*)-5-(2,2-dimethyltetrahydro-2*H*-pyran-4-yl)-*N*-methyl-*N*-phenyl-1*H*-indol-2-carboxamide (1.90 g, 5.24 mmol) in 1,3-dimethyl-2-imidazolinone (19 mL). Chloroacetonitrile (0.59 g, 7.86 mmol) was slowly added at 0°C, and reacted at 30°C for 3 h. The pH of the system was adjusted to be weakly acidic with 2N dilute hydrochloric acid. The reaction mixture was diluted with water (50 mL) and extracted with ethyl acetate (80 mL×3), and the organic phases were combined. The organic phase was washed with a 20% saline solution (50 mL×3), dried with anhydrous sodium sulfate, concentrated under reduced pressure, and separated and purified by silica gel column chromatography (EA:PE (v/v)=0 to 17%) to obtain the target compound.

LC-MS (ESI): [M+H]⁺ =402.3.

### Step 4: Synthesis of (S)-1-(1-cyanocyclopropyl)-5-(2,2-dimethyltetrahydro-2H-pyran-4-yl)-N-methyl-N-phenyl-1H-indol-2-carboxamide

Lithium bis(trimethylsilyl)amide (1M, 4.3 mL, 9.96 mmol) was slowly added dropwise to a solution of (*S*)-1-(cyanomethyl)-5-(2,2-dimethyltetrahydro-2*H*-pyran-4-yl)-*N*-methyl-*N*-phenyl-1*H*-indol-2-carboxamide (1.0 g, 2.49 mmol) and ethylene sulfate (0.93 g, 7.47 mmol) in THF (15 mL) at 0°C, and reacted at 0°C for 3 h. The reaction was quenched with 1M dilute hydrochloric acid (15 mL), the reaction mixture was extracted with ethyl acetate (20 mL×3), and the organic phases were combined. The organic phase was washed with a saturated sodium bicarbonate solution (20 mL) and a saturated saline solution (20 mL) in sequence, dried with anhydrous sodium sulfate, filtered, and concentrated under reduced pressure to obtain the target product.

LC-MS (ESI): [M+H]⁺ =428.2.

### Step 5: Synthesis of (S)-5-(2,2-dimethyltetrahydro-2H-pyran-4-yl)-N-methyl-1-(1-(5-oxo-4,5-dihydro-1,2,4-oxadiazol-3-yl)cyclopropyl)-N-phenyl-1H-indol-2-carboxamide

Hydroxylamine hydrochloride (0.81 g, 11.69 mmol) and sodium bicarbonate (0.98 g, 11.69 mmol) were added to a solution of (*S*)-1-(1-cyanocyclopropyl)-5-(2,2-dimethyltetrahydro-2*H-*pyran-4-yl)-*N*-methyl-*N*-phenyl-1*H*-indol-2-carboxamide (1.0 g, 2.34 mmol) in DMSO (8 mL), and heated to 60°C to react for 2 h. The reaction mixture was diluted with water (25 mL) and extracted with DCM (20 mL×3), and the organic phases were combined. The organic phase was washed with a saturated saline solution (20 mL), dried with anhydrous sodium sulfate, and concentrated under reduced pressure. The resulting residue was added to a solution of CDI (0.7 g, 4.34 mmol) and DBU (0.83 g, 5.43 mmol) in DMSO (10 mL). The reaction mixture was stirred at room temperature for 1 h, diluted with water (30 mL), and extracted with DCM (20 mL×3), and the organic phases were combined. The organic phase was washed with a saturated saline solution, dried with anhydrous sodium sulfate, and concentrated under reduced pressure to obtain the target product.

LC-MS (ESI): [M+H]⁺ =487.2.

### Step 6: Synthesis of (S)-5-(2,2-dimethyltetrahydro-2H-pyran-4-yl)-1-(1-(5-oxo-4,5-dihydro-1,2,4-oxadiazol-3-yl)cyclopropyl)-1H-indol-2-formic acid

KOH (1.15 g, 20.55 mmol) was added to a solution of (*S*)-5-(2,2-dimethyltetrahydro-2*H-*pyran-4-yl)-*N*-methyl-1-(1-(5-oxo-4,5-dihydro-1,2,4-oxadiazol-3-yl)cyclopropyl)-*N*-phenyl-1*H-*indol-2-carboxamide (1.0 g, 2.06 mmol) in ethylene glycol monomethyl ether (3 mL), and heated to 130°C to react overnight. The reaction mixture was cooled to room temperature, adjusted to a pH of about 3 with dilute hydrochloric acid, and extracted with ethyl acetate (20 mL×3). The organic phase was dried with anhydrous sodium sulfate, concentrated under reduced pressure, and subjected to preparative HPLC to obtain the target product.

LC-MS (ESI): [M+H]+ =398.2.

### Step 7: Synthesis of 3-(1-(2-((S)-3'-(3-(1-cyclopropyl-4-fluoro-1H-indazol-5-yl)-2-oxo-2,3-dihydro-1H-imidazol-1-yl)-2'-(4-fluoro-3,5-dimethylphenyl)-4'-methyl-2',4',5',6'-tetrahydrospiro[cyclopropane-1,7'-pyrazolo[4,3-c]pyridine]-5'-carbonyl)-5-((S)-2,2-dimethyltetrahydro-2H-pyran-4-yl)-1H-indol-1-yl)cyclopropyl)-1,2,4-oxadiazol-5(4H)-one

(*S*)-1-(1-cyclopropyl-4-fluoro-1*H*-indazol-5-yl)-3-(2'-(4-fluoro-3,5-dimethylphenyl)-4'-methyl-2',4',5',6'-tetrahydrospiro[cyclopropane-1,7'-pyrazolo[4,3-*c*]pyridine]-3'-yl)-1,3-dihydro-2*H*-imidazol-2-one (1 g, 1.85 mmol), 5-((*S*)-2,2-dimethyltetrahydro-2H-pyran-4-yl)-1-(1-(5-oxo-4,5-dihydro-1,2,4-oxadiazol-3-yl)cyclopropyl)-1*H*-indol-2-formic acid (0.73 g, 1.85 mmol), EDCI (0.84 g, 4.28 mmol), and HOAT (0.34 g, 2.49 mmol) were dissolved in DMF (20 mL). Then, DIPEA (0.72 g, 5.54 mmol) was added to react overnight at room temperature. The reaction mixture was diluted with water (50 mL) and extracted with ethyl acetate (20 mL×3), and the organic phases were combined. The organic phase was washed with a saturated saline solution, dried with anhydrous sodium sulfate, filtered, concentrated under reduced pressure, and purified by preparative HPLC to obtain the target compound.

¹H NMR (400 MHz, CDCl₃) *δ* 11.27 (s, 1H), 8.01 (s, 1H), 7.56 - 7.50 (m, 1H), 7.47 - 7.31 (m, 2H), 7.27 - 7.08 (m, 2H), 7.06 - 6.91 (m, 2H), 6.62 (d, *J=* 5.0 Hz, 1H), 6.55 - 6.48 (m, 1H), 6.27 - 6.23 (m, 1H), 5.82 - 5.77 (m, 1H), 4.12 - 3.93 (m, 1H), 3.84 - 3.64 (m, 2H), 3.58 - 3.42 (m, 2H), 3.07 - 2.86 (m, 1H), 2.22 - 2.09 (m, 6H), 2.08 - 1.99 (m, 1H), 1.75 - 1.67 (m, 2H), 1.68 - 1.59 (m, 4H), 1.58 - 1.48 (m, 3H), 1.44 - 1.28 (m, 2H), 1.28 (s, 3H), 1.25 - 1.19 (m, 4H), 1.19 - 1.09 (m, 4H), 1.07 - 0.88 (m, 1H), 0.78 - 0.65 (m, 1H).

### Example 16 Synthesis of 3-(1-(2-((S)-3-(3-(1-cyclopropyl-4-fluoro-1H-indazol-5-yl)-2-oxo-2,3-dihydro-1H-imidazol-1-yl)-2-(4-fluoro-3,5-dimethylphenyl)-4,7,7-trimethyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)cyclopropyl)-1,2,4-oxadiazol-5(4H)-one (Compound 16)

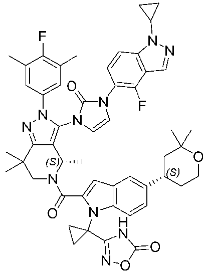

### Step 1: Synthesis of ethyl 2,2-dimethyl-3-oxopropionate

Dess-Martin periodinane (21.76 g, 51.3 mmol) was added in batches to a solution of ethyl 3-hydroxyl-2,2-dimethylpropionate (5 g, 34.2 mmol) in dichloromethane (100 mL) at 0°C, and heated to room temperature to react for 2 h. In an ice bath for cooling, the reaction was quenched with a saturated sodium thiosulfate solution, and then a saturated sodium bicarbonate solution was added. The reaction mixture was extracted with dichloromethane (100 mL×3), and the organic phases were combined. The organic phase was washed with a saturated saline solution (100 mL), dried with anhydrous sodium sulfate, filtered, concentrated under reduced pressure, and purified by silica gel column chromatography (PE:EA (v/v)=10:1 to 5:1) to obtain the target compound.

### Step 2: Synthesis of ethyl (S)-3-((1-cyanopropan-2-yl)amino)-2,2-dimethylpropionate

(*S*)-3-aminobutanenitrile hydrochloride (300 mg, 2.49 mmol), methanol (10 mL), and diisopropylethylamine (322 mg, 2.49 mmol) were added to a reaction flask. The reaction mixture was stirred for 20 min. Then, acetic acid (134 mg, 2.24 mmol) and ethyl 2,2-dimethyl-3-oxopropionate (470 mg, 3.26 mmol) were added and stirred for 2 h. Then sodium triacetoxyborohydride (1.05 g, 4.98 mmol) was added and stirred at room temperature for 6 h. The reaction mixture was diluted with water and extracted with ethyl acetate (3×30 mL), and the organic phases were combined. The organic phase was washed with a saturated sodium bicarbonate solution (50 mL) and a saturated saline solution (50 mL) in sequence, dried with anhydrous sodium sulfate, filtered, and concentrated under reduced pressure to obtain the target compound to be directly used for the next step.

### Step 3: Synthesis of ethyl (S)-3-((tert-butoxycarbonyl)(1-cyanopropan-2-yl)amino)-2,2-dimethylpropionate

The crude product of ethyl (*S*)-3-((1-cyanopropan-2-yl)amino)-2,2-dimethylpropionate and di-tert-butyl dicarbonate (2 mL) were added to a reaction flask. The reaction solution was stirred at room temperature overnight, concentrated under reduced pressure, and separated and purified by silica gel column chromatography (PE:EA (v/v)=10:1 to 5:1) to obtain the target compound.

LC-MS (ESI): [M+H-Boc]⁺ =213.1.

### Step 4: Synthesis of tert-butyl (2S)-3-cyano-2,5,5-trimethyl-4-oxopiperidine-1-formate

A 1.0 M solution of potassium tert-butoxide in THF (1.3 mL, 1.26 mmol) was added to a solution of ethyl (*S*)-3-((tert-butoxycarbonyl)(1-cyanopropan-2-yl)amino)-2,2-dimethylpropionate (263 mg, 0.84 mmol) in tetrahydrofuran (6 mL). After 1 h of reaction at room temperature, the reaction was quenched with a saturated ammonium chloride solution, the reaction mixture was extracted with ethyl acetate (3×15 mL), and the organic phases were combined. The organic phase was washed with a saturated saline solution (30 mL), dried with anhydrous sodium sulfate, filtered, and concentrated under reduced pressure to obtain the target compound.

LC-MS (ESI): [M+H]⁺ =267.3.

### Step 5: Synthesis of tert-butyl (S)-3-amino-2-(4-fluoro-3,5-dimethylphenyl)-4,7,7-trimethyl-2,4,6,7-tetrahydro-5H-pyrazolo[4,3-c]pyridine-5-carboxylate

(4-fluoro-3,5-dimethylphenyl)hydrazine (200 mg, 1.3 mmol) and tert-butyl (2*S*)-3-cyano-2,5,5-trimethyl-4-oxopiperidine-1-formate (173 mg, 0.65 mmol) were dissolved in ethanol (10 mL). A 4.0 M dioxane hydrochloride solution (0.16 mL) was added in a nitrogen atmosphere, and the reaction mixture was heated to 80°C to react for 1.5 h. The reaction mixture was cooled to room temperature, a saturated saline solution (30 mL) was added, the reaction mixture was extracted with ethyl acetate (3×15 mL), and the organic phases were combined. The organic phase was dried with anhydrous sodium sulfate, filtered, concentrated under reduced pressure, and separated and purified by silica gel column chromatography (PE:EA (v/v)=10:1 to 3:1) to obtain the target compound.

LC-MS (ESI): [M+H]⁺ =403.4.

### Step 6: Synthesis of tert-butyl (S)-3-(3-(2,2-dimethoxyethyl)ureido)-2-(4-fluoro-3,5-dimethylphenyl)-4,7,7-trimethyl-2,4,6,7-tetrahydro-5H-pyrazolo[4,3-c]pyridine-5-carboxylate

Tert-butyl (*S*)-3-amino-2-(4-fluoro-3,5-dimethylphenyl)-4,7,7-trimethyl-2,4,6,7-tetrahydro-5*H*-pyrazolo[4,3-*c*]pyridine-5-carboxylate (173 mg, 0.43 mmol) and DIPEA (277 mg, 2.15 mmol) were dissolved in tetrahydrofuran (6 mL), and phenyl chloroformate (134 mg, 0.86 mmol) was slowly added, and reacted at room temperature for 1 h. 2,2-dimethoxyethane-1-amine (181 mg, 1.72 mmol) was added to react at room temperature for 1 h. The reaction was quenched with a saturated saline solution (30 mL), the reaction mixture was extracted with ethyl acetate (3×15 mL), and the organic phases were combined. The organic phase was dried with anhydrous sodium sulfate, filtered, concentrated under reduced pressure, and separated by silica gel column chromatography (PE:EA (v/v)=3:1 to 2:1) to obtain the target compound.

LC-MS (ESI): [M+H]⁺ =534.4.

### Step 7: Synthesis of tert-butyl (S)-2-(4-fluoro-3,5-dimethylphenyl)-4,7,7-trimethyl-3-(2-oxo-2,3-dihydro-1H-imidazol-1-yl)-2,4,6,7-tetrahydro-5H-pyrazolo[4,3-c]pyridine-5-carboxylate

Methanesulfonic acid (34.52 mg, 0.36 mmol) was added to a solution of tert-butyl (*S*)-3-(3-(2,2-dimethoxyethyl)ureido)-2-(4-fluoro-3,5-dimethylphenyl)-4,7,7-trimethyl-2,4,6,7-tetrahydro-5*H*-pyrazolo[4,3-*c*]pyridine-5-carboxylate (213 mg, 0.4 mmol) in tetrahydrofuran (5 mL), and heated to 60°C to react for 1 h. The reaction mixture was cooled to room temperature, a saturated saline solution (30 mL) was added, the reaction mixture was extracted with ethyl acetate (3×10 mL), and the organic phases were combined. The organic phase was dried with anhydrous sodium sulfate, filtered, concentrated under reduced pressure, and separated by silica gel column chromatography (PE:EA (v/v)=3:1 to 2:1) to obtain the target compound.

LC-MS (ESI): [M+H]⁺ =470.3.

### Step 8: Synthesis of tert-butyl (S)-3-(3-(1-cyclopropyl-4-fluoro-1H-indazol-5-yl)-2-oxo-2,3-dihydro-1H-imidazol-1-yl)-2-(4-fluoro-3,5-dimethylphenyl)-4,7,7-trimethyl-2,4,6,7-tetrahydro-5H-pyrazolo[4,3-c]pyridine-5-carboxylate

Cuprous iodide (69.6 mg, 0.2 mmol), (1*S*,2*S*)-(+)-*N,N'*-dimethyl-1,2-cyclohexanediamine (31 mg, 0.2 mmol), and potassium carbonate (181.9 mg, 1.32 mmol) were added to a solution of tert-butyl (*S*)-2-(4-fluoro-3,5-dimethylphenyl)-4,7,7-trimethyl-3-(2-oxo-2,3-dihydro-1*H*-imidazol-1-yl)-2,4,6,7-tetrahydro-5*H*-pyrazolo[4,3-*c*]pyridine-5-carboxylate (206 mg, 0.4 mmol) and 5-bromo-1-cyclopropyl-4-fluoro-1*H*-indazole (145 mg, 0.6 mmol) in NMP (5 mL), and heated to 130°C in a nitrogen atmosphere to react for 2 h. The reaction mixture was cooled to room temperature, a saturated saline solution (30 mL) was added, the reaction mixture was extracted with ethyl acetate (3×10 mL), and the organic phases were combined. The organic phase was dried with anhydrous sodium sulfate, filtered, concentrated under reduced pressure, and separated and purified by silica gel column chromatography (PE:EA (v/v)=3:1 to 2:1) to obtain the target compound.

LC-MS (ESI): [M+H]⁺ =644.3.

### Step 9: Synthesis of (S)-1-(1-cyclopropyl-4-fluoro-1H-indazol-5-yl)-3-(2-(4-fluoro-3,5-dimethylphenyl)-4,7,7-trimethyl-4,5,6,7-tetrahydro-2H-pyrazolo[4,3-c]pyridin-3-yl)-1,3-dihydro-2H-imidazol-2-one

Tert-butyl (*S*)-3-(3-(1-cyclopropyl-4-fluoro-1*H*-indazol-5-yl)-2-oxo-2,3-dihydro-1*H-*imidazol-1-yl)-2-(4-fluoro-3,5-dimethylphenyl)-4,7,7-trimethyl-2,4,6,7-tetrahydro-5*H-*pyrazolo[4,3-*c*]pyridine-5-carboxylate (200 mg, 0.3 mmol) was added to a 4N dioxane hydrochloride solution (5.1 mL), and reacted at room temperature for 1 h. The reaction mixture was directly concentrated under reduced pressure to obtain the target compound.

LC-MS (ESI): [M+H]⁺ =544.3.

### Step 10: Synthesis of 3-(1-(2-((S)-3-(3-(1-cyclopropyl-4-fluoro-1H-indazol-5-yl)-2-oxo-2,3-dihydro-1H-imidazol-1-yl)-2-(4-fluoro-3,5-dimethylphenyl)-4,7,7-trimethyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)cyclopropyl)-1,2,4-oxadiazol-5(4H)-one

(*S*)-1-(1-cyclopropyl-4-fluoro-1*H*-indazol-5-yl)-3-(2-(4-fluoro-3,5-dimethylphenyl)-4,7,7-trimethyl-4,5,6,7-tetrahydro-2*H*-pyrazolo[4,3-*c*]pyridin-3-yl)-1,3-dihydro-2*H*-imidazol-2-one (55 mg, 0.10 mmol), 5-((*S*)-2,2-dimethyltetrahydro-2*H*-pyran-4-yl)-1-(1-(5-oxo-4,5-dihydro-1,2,4-oxadiazol-3-yl)cyclopropyl)-1*H*-indol-2-carboxylic acid (40 mg, 0.10 mmol), EDCI (48 mg, 0.25 mmol), and HOAT (21 mg, 0.15 mmol) were dissolved in DMF (2 mL). DIPEA (39 mg, 0.30 mmol) was added to react overnight at room temperature. The reaction mixture was diluted with water (20 mL) and extracted with ethyl acetate (15 mL×3), and the organic phases were combined. The organic phase was washed with a saturated saline solution (20 mL), dried with anhydrous sodium sulfate, filtered, concentrated under reduced pressure, and subjected to preparative HPLC to obtain the target compound.

¹H NMR (400 MHz, DMSO-*d*₆) δ 12.10 (s, 1H), 8.25 (d, *J =* 18.9 Hz, 1H), 7.76 - 7.59 (m, 1H), 7.49 (d, *J =* 8.7 Hz, 2H), 7.28 - 7.05 (m, 4H), 6.92 (d, *J =* 38.9 Hz, 1H), 6.72 (d, *J=* 32.3 Hz, 1H), 5.83 - 5.22 (m, 1H), 3.82 (s, 2H), 3.72 (d, *J =* 8.8 Hz, 2H), 3.04 (s, 2H), 2.25 (s, 6H), 2.10 - 1.93 (m, 1H), 1.84 (s, 1H), 1.68 (s, 4H), 1.55 (d, *J=* 12.6 Hz, 4H), 1.36 (s, 4H), 1.26 (d, *J= 16.9* Hz, 8H), 1.19 (s, 6H).

### Example 17 Synthesis of 3-[[5-[(4S)-2,2-dimethyl-3,4,5,6-tetrahydro-2H-pyran-4-yl]-2-[[(4'S)-3'[3-(1-cyclopropyl-6-fluoroindazol-5-yl)-2-oxo-2,3-dihydro-1H-imidazol-1-yl]-2'-(4-fluoro-3,5-dimethylphenyl)-4'-methyl-5',6'-dihydro-4'H-spiro(cyclopropane-1,7'-pyrazolo[4,3-c]pyridine)-5'-yl]carbonyl]indol-1-yl]cyclopropyl]-1,2,4-oxadiazol-5(4H)-one (Compound 17)

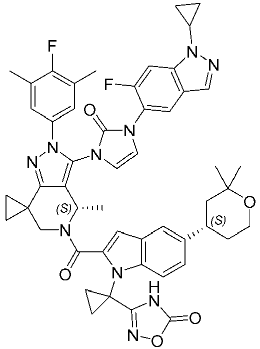

### Step 1: Synthesis of tert-butyl (S)-3'-[3-(1-cyclopropyl-6-fluoro-1H-indazol-5-yl)-2-oxo-2,3-dihydro-1H-imidazol-1-yl]-2'-(4-fluoro-3,5-dimethylphenyl)-4'-methyl-2',4'-dihydrospiro(cyclopropyl-1,7'-pyrazolo[4,3-c]pyridine)-5'(6'H)-carboxylate

*N*-methylpyrrolidone (5 mL), tert-butyl (*S*)-2'-(4-fluoro-3,5-dimethylphenyl)-4'-methyl-3'-(2-oxo-2,3-dihydro-1*H*-imidazol-1-yl)-2',4'-dihydrospiro(cyclopropyl-1,7'-pyrazolo[4,3-*c*]pyridine)-5'(6'*H*)-carboxylate (500 mg, 1.07 mmol), 5-bromo-1-cyclopropyl-6-fluoroindazole (410 mg, 1.60 mmol), (1*S*,2*S*)-(+)-*N,N*-dimethylcyclohex-1,2-diamine (60 mg, 0.43 mmol), potassium carbonate (440 mg, 3.21 mmol), and cuprous iodide (70 mg, 0.21 mmol) were added in sequence to a reaction flask, and heated to 100°C in a nitrogen atmosphere to react for 12 h. After the reaction was completed, the reaction mixture was cooled to room temperature, the reaction was quenched with water (50 mL), and the reaction mixture was extracted with ethyl acetate (50 mL×2). The organic phases were combined, washed with a saturated sodium chloride aqueous solution (50 mL), dried with anhydrous sodium sulfate, filtered, concentrated under reduced pressure, and separated and purified by reverse phase column chromatography (column: Phenomenex luna C18 150*25 mm* 10 µm; mobile phase: [A: H₂O (0.225% FA); B: ACN]; B%: 62.00% to 92.00%, 15.00 min; flow rate: 60.00 ml/min) to obtain the target compound.

LC-MS (ESI): [M+H]⁺ =642.4.

### Step 2: Synthesis of 3-(1-cyclopropyl-6-fluoro-1H-indazol-5-yl)-1-[(4'S)-2'-(4-fluoro-3,5-dimethylphenyl)-4'-methyl-5',6'-dihydro-4'H-spiro(cyclopropane-1,7'-pyrazolo[4,3-c]pyridine)-3'-yl]-2,3-dihydro-1H-imidazol-2-one

Tert-butyl (*S*)-3'-[3-(1-cyclopropyl-6-fluoro-1*H*-indazol-5-yl)-2-oxo-2,3-dihydro-1*H-*imidazol-1-yl]-2'-(4-fluoro-3,5-dimethylphenyl)-4'-methyl-2',4'-dihydrospiro(cyclopropyl-1,7'-pyrazolo[4,3-*c*]pyridine)-5'(6'*H*)-carboxylate (550 mg, 0.86 mmol) and a 2M dioxane hydrochloride solution (20 mL) were added in sequence to a reaction flask and stirred at 40°C for 1 h. After the reaction was completed, the reaction solution was directly concentrated under reduced pressure to obtain the target compound.

LC-MS (ESI): [M+H]⁺ =542.3.

### Step 3: Synthesis of 3-(1-cyclopropyl-6-fluoro-1H-indazol-5-yl)-1-[(4'S)-2'-(4-fluoro-3,5-dimethylphenyl)-5'-([5-[(4S)-2,2-dimethyl-3,4,5,6-tetrahydro-2H-pyran-4-yl]-1H-indol-2-yl]carbonyl)-4'-methyl-5',6'-dihydro-4'H-spiro(cyclopropane-1,7'-pyrazolo[4,3-c]pyridine)-3'-yl]-2,3-dihydro-1H-imidazol-2-one

*N*,*N*-dimethylformamide (5 mL), 3-(1-cyclopropyl-6-fluoro-1*H*-indazol-5-yl)-1-[(4'*S*)-2'-(4-fluoro-3,5-dimethylphenyl)-4'-methyl-5',6'-dihydro-4'*H*-spiro(cyclopropane-1,7'-pyrazolo[4,3-c]pyridine)-3'-yl]-2,3-dihydro-1*H*-imidazol-2-one (460 mg, 0.85 mmol), 5-[(4*S*)-2,2-dimethyl-3,4,5,6-tetrahydro-2*H*-pyran-4-yl]-1*H*-indol-2-formic acid (278 mg, 1.02 mmol), *N,N-*diisopropylethylamine (439 mg, 3.40 mmol), and HATU (484 mg, 1.27 mmol) were added in sequence to a reaction flask to react at 25°C for 3 h. After the reaction was completed, the reaction solution was slowly poured into water (50 mL) for quenching, and extracted with ethyl acetate (50 mL×2). The organic phases were combined, washed with a saturated sodium chloride aqueous solution (50 mL), dried with anhydrous sodium sulfate, filtered, concentrated under reduced pressure, and separated and purified by silica gel column chromatography (EA:PE (v/v)=0% to 60%) to obtain the target compound.

LC-MS (ESI): [M+H]⁺ =797.3.

### Step 4: Synthesis of [5-[(4S)-2,2-dimethyl-3,4,5,6-tetrahydro-2H-pyran-4-yl]-2-[[(4'S)-3'-[3-(1-cyclopropyl-6-fluoro-1H-indazol-5-yl)-2-oxo-2,3-dihydro-1H-imidazol-1-yl]-2'-(4-fluoro-3,5-dimethylphenyl)-4'-methyl-5',6'-dihydro-4'H-spiro(cyclopropane-1,7'-pyrazolo[4,3-c]pyridine)-5'-yl]carbonyl]indol-1-yl]acetonitrile

*N*,*N*-dimethylformamide (5 mL) and 3-(1-cyclopropyl-6-fluoro-1*H*-indazol-5-yl)-1-[(4'*S*)-2'-(4-fluoro-3,5-dimethylphenyl)-5'-([5-[(4*S*)-2,2-dimethyl-3,4,5,6-tetrahydro-2*H*-pyran-4-yl]-1*H-*indol-2-yl]carbonyl)-4'-methyl-5',6'-dihydro-4'*H*-spiro(cyclopropane-1,7'-pyrazolo[4,3-c]pyridine)-3'-yl]-2,3-dihydro-1*H*-imidazol-2-one (400 mg, 0.50 mmol) were added to a reaction flask. The system was cooled to 0°C, and then sodium hydride (200 mg, 5.02 mmol, 60%) was added in a nitrogen atmosphere and stirred at 0°C for 0.5 h. Then, chloroacetonitrile (493 mg, 6.53 mmol) was added and stirred at 0°C for 1.5 h. After the reaction was completed, the reaction solution was poured into a saturated ammonium chloride aqueous solution (50 mL) for quenching, and extracted with ethyl acetate (50 mL×2). The organic layers were combined, dried with anhydrous sodium sulfate, filtered, concentrated under reduced pressure, and separated and purified by silica gel column chromatography (EA:PE (v/v)=0% to 60%) to obtain the target compound.

LC-MS (ESI): [M+H]⁺ =836.4.

### Step 5: Synthesis of 1-[5-[(4S)-2,2-dimethyl-3,4,5,6-tetrahydro-2H-pyran-4-yl]-2-[[(4'S)-3'-[3-(1-cyclopropyl-6-fluoro-1H-indazol-5-yl)-2-oxo-2,3-dihydro-1H-imidazol-1-yl]-2'-(4-fluoro-3,5-dimethylphenyl)-4'-methyl-5',6'-dihydro-4'H-spiro(cyclopropane-1,7'-pyrazolo[4,3-c]pyridine)-5'-yl]carbonyl]indol-1-yl]cyclopropane-1-carbonitrile

Ethylene sulfate (140 mg, 1.14 mmol) was added to a solution of [5-[(4*S*)-2,2-dimethyl-3,4,5,6-tetrahydro-2*H*-pyran-4-yl]-2-[[(4'*S*)-3'-[3-(1-cyclopropyl-6-fluoro-1*H*-indazol-5-yl)-2-oxo-2,3-dihydro-1*H*-imidazol-1-yl]-2'-(4-fluoro-3,5-dimethylphenyl)-4'-methyl-5',6'-dihydro-4'*H*-spiro(cyclopropane-1,7'-pyrazolo[4,3-*c*]pyridine)-5'-yl]carbonyl]indol-1-yl]acetonitrile (380 mg, 0.45 mmol) in tetrahydrofuran (5 mL) and cooled to 0°C. Then a 1M solution of lithium bis(trimethylsilyl)amide (1.8 mL, 1.82 mmol) in toluene was slowly added dropwise to react at 0°C for 2 h. After the reaction was completed, the reaction solution was slowly poured into a saturated ammonium chloride aqueous solution (50 mL) for quenching, and extracted with ethyl acetate (50 mL×3). The organic phases were combined, dried with anhydrous sodium sulfate, filtered, concentrated under reduced pressure, and separated and purified by silica gel column chromatography (EA:PE (v/v)=0% to 60%) to obtain the target compound.

LC-MS (ESI): [M+H]⁺ =862.3.

### Step 6: Synthesis of 1-[2-[(S)-3'-[3-(1-cyclopropyl-6-fluoro-1H-indazol-5-yl)-2-oxo-2,3-dihydro-1H-imidazol-1-yl]-2'-(4-fluoro-3,5-dimethylphenyl)-4'-methyl-2',4',5',6'-tetrahydrospiro(cyclopropane-1,7'-pyrazolo[4,3-c]pyridine)-5'-carbonyl]-5-[(S)-2,2-dimethyltetrahydro-2H-pyran-4-yl]-1H-indol-1-yl]-N-hydroxycyclopropane-1-carboxamidine

A 50% aqueous solution of hydroxylamine (517 mg, 7.83 mmol) was added to a solution of 1-[5-[(4*S*)-2,2-dimethyl-3,4,5,6-tetrahydro-2*H*-pyran-4-yl]-2-[[(4'*S*)-3'-[3-(1-cyclopropyl-6-fluoroindazol-5-yl)-2-oxo-2,3-dihydro-1*H*-imidazol-1-yl]-2'-(4-fluoro-3,5-dimethylphenyl)-4'-methyl-5',6'-dihydro-4'*H*-spiro(cyclopropane-1,7'-pyrazolo[4,3-*c*]pyridine)-5'-yl]carbonyl]indol-1-yl]cyclopropane-1-carbonitrile (270 mg, 0.31 mmol) in dimethyl sulfoxide (5 mL), and stirred at 25°C for 3 h. After the reaction was completed, the reaction was quenched with water (50 mL), and the reaction mixture was extracted with ethyl acetate (50 mL×2). The organic phases were combined, rinsed with a saturated sodium chloride aqueous solution (50 mL), dried with anhydrous sodium sulfate, filtered, and concentrated under reduced pressure to obtain the target compound.

LC-MS (ESI): [M+H]⁺ =895.4.

### Step 7: Synthesis of 3-([5-[(4S)-2,2-dimethyl-3,4,5,6-tetrahydro-2H-pyran-4-yl]-2-[[(4'S)-3'-[3-(1-cyclopropyl-6-fluoro-1H-indazol-5-yl)-2-oxo-2,3-dihydro-1H-imidazol-1-yl]-2'-(4-fluoro-3,5-dimethylphenyl)-4'-methyl-5',6'-dihydro-4'H-spiro(cyclopropane-1,7'-pyrazolo[4,3-c]pyridine)-5'-yl]carbonyl]indol-1-yl]cyclopropyl]1,2,4-oxadiazol-5(4H)-one

Dimethyl sulfoxide (2 mL), 1-(2-((*S*)-3'-(3-(1-cyclopropyl-6-fluoro-1*H*-indazol-5-yl)-2-oxo-2,3-dihydro-1*H*-imidazol-1-yl)-2'-(4-fluoro-3,5-dimethylphenyl)-4'-methyl-2',4',5',6'-tetrahydrospiro[cyclopropane-1,7'-pyrazolo[4,3-*c*]pyridine]-5'-carbonyl)-5-((S)-2,2-dimethyltetrahydro-2*H*-pyran-4-yl)-1*H*-indol-1-yl)-*N*-hydroxycyclopropane-1-carboxamidine (140 mg, 0.16 mmol), *N*,*N*'-carbonyldiimidazole (63 mg, 0.39 mmol), and 1.8-diazabicyclo[5.4.0]undecane-7-ene (95 mg, 0.63 mmol) were added in sequence to a reaction flask to react at 25°C for 12 h. After the reaction was completed, the reaction solution was directly separated and purified by reverse phase column chromatography (column: Waters Xbridge C18 150*25 mm*5 um; mobile phase: [A: H₂O (0.05% NH₃H₂O); B: ACN]; B%: 10.00% to 40.00%, 11.00 min; flow rate: 25.00 ml/min) to obtain the target compound.

LC-MS (ESI): [M+H]⁺ =921.6;
¹H NMR (400 MHz, CDCl₃) *δ* = 11.41 - 11.30 (m, 1H), 8.00 (s, 1H), 7.87 - 7.69 (m, 1H), 7.65 - 7.57 (m, 1H), 7.56 - 7.47 (m, 1H), 7.46 - 7.31 (m, 1H), 7.14 - 7.01 (m, 2H), 6.73 - 6.40 (m, 2H), 6.37 - 6.26 (m, 1H), 6.25 - 6.02 (m, 1H), 5.92 - 5.26 (m, 1H), 4.27 - 3.93 (m, 1H), 3.92 - 3.81 (m, 2H), 3.80 - 3.49 (m, 2H), 3.13 - 2.98 (m, 1H), 2.29 - 2.23 (m, 6H), 1.82 - 1.72 (m, 3H), 1.70 - 1.57 (m, 6H), 1.48 - 1.39 (m, 2H), 1.35 - 1.33 (m, 3H), 1.32 - 1.27 (m, 4H), 1.26 - 1.17 (m, 5H), 1.11 - 0.93 (m, 1H), 0.86 - 0.37 (m, 1H).

### Example 18 Synthesis of 3-([5-[(4S)-2,2-dimethyl-3,4,5,6-tetrahydro-2H-pyran-4-yl]-2-[[(4'S)-3'-[3-(1-cyclopropyl-7-fluoroindazol-5-yl)-2-oxo-2,3-dihydro-1H-imidazol-1-yl]-2'-(4-fluoro-3,5-dimethylphenyl)-4'-methyl-5',6'-dihydro-4'H-spiro(cyclopropane-1,7'-pyrazolo[4,3-c]pyridine)-5'-yl]carbonyl]indol-1-yl]cyclopropyl]-1,2,4-oxadiazol-5(4H)-one (Compound 18)

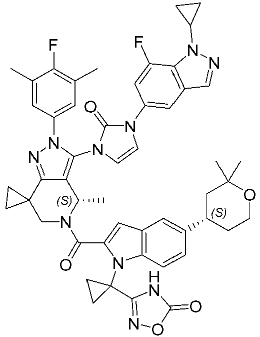

### Step 1: Synthesis of tert-butyl (S)-3'-[3-(1-cyclopropyl-7-fluoro-1H-indazol-5-yl)-2-oxo-2,3-dihydro-1H-imidazol-1-yl]-2'-(4-fluoro-3,5-dimethylphenyl)-4'-methyl-2',4'-dihydrospiro(cyclopropyl-1,7'-pyrazolo[4,3-c]pyridine)-5'(6'H)-carboxylate

Tert-butyl (*S*)-2'-(4-fluoro-3,5-dimethylphenyl)-4'-methyl-3'-(2-oxo-2,3-dihydro-1*H-*imidazol-1-yl)-2',4'-dihydrospiro(cyclopropyl-1,7'-pyrazolo[4,3-*c*]pyridine)-5'(6'*H*)-carboxylate (500 mg, 1.07 mmol), 5-bromo-1-cyclopropyl-7-fluoroindazole (409.19 mg, 1.60 mmol), (1*S*,2*S*)-(+)-*N,N*-dimethylcyclohex-1,2-diamine (76.06 mg, 0.53 mmol), potassium carbonate (443.38 mg, 3.21 mmol), and cuprous iodide (203.67 mg, 1.07 mmol) were added to a reaction flask, and then *N*-methylpyrrolidone (8 mL) was added to react in a nitrogen atmosphere at 100°C for 16 h. After the reaction was completed, the reaction solution was poured into water (100 mL), ammonia water (15 mL) was added, and the reaction mixture was extracted with ethyl acetate (100 mL×2). The organic phases were combined, dried with anhydrous sodium sulfate, filtered, concentrated under reduced pressure, and separated and purified by silica gel column chromatography (EA:PE (v/v)=50%) to obtain the target compound.

LC-MS (ESI): [M+H]⁺ =642.3.

### Step 2: Synthesis of 3-(1-cyclopropyl-7-fluoro-1H-indazol-5-yl)-1-[(4'S)-2'-(4-fluoro-3,5-dimethylphenyl)-4'-methyl-5',6'-dihydro-4'H-spiro(cyclopropane-1,7'-pyrazolo[4,3-c]pyridine)-3'-yl]-2,3-dihydro-1H-imidazol-2-one

Tert-butyl (*S*)-3'-[3-(1-cyclopropyl-7-fluoro-1*H*-indazol-5-yl)-2-oxo-2,3-dihydro-1*H-*imidazol-1-yl]-2'-(4-fluoro-3,5-dimethylphenyl)-4'-methyl-2',4'-dihydrospiro(cyclopropyl-1,7'-pyrazolo[4,3-*c*]pyridine)-5'(6'*H*)-carboxylate (550 mg, 0.86 mmol) was dissolved in 2M dioxane hydrochloride (10 mL) to react at 40°C for 2 h. After the reaction was completed, the reaction mixture was directly concentrated under reduced pressure to obtain the target compound.

LC-MS (ESI): [M + H]⁺ =542.2.

### Step 3: Synthesis of 3-(1-cyclopropyl-7-fluoro-1H-indazol-5-yl)-1-[(4'S)-2'-(4-fluoro-3,5-dimethylphenyl)-5'-([5-[(4S)-2,2-dimethyl-3,4,5,6-tetrahydro-2H-pyran-4-yl]-1H-indol-2-yl]carbonyl)-4'-methyl-5',6'-dihydro-4'H-spiro(cyclopropane-1,7'-pyrazolo[4,3-c]pyridine)-3'-yl]-2,3-dihydro-1H-imidazol-2-one

3-(1-cyclopropyl-7-fluoro-1*H*-indazol-5-yl)-1-[(4'*S*)-2'-(4-fluoro-3,5-dimethylphenyl)-4'-methyl-5',6'-dihydro-4'*H*-spiro(cyclopropane-1,7'-pyrazolo[4,3-*c*]pyridine)-3'-yl]-2,3-dihydro-1*H*-imidazol-2-one (460 mg, 0.85 mmol) and 5-[(4S)-2,2-dimethyl-3,4,5,6-tetrahydro-2*H*-pyran-4-yl]-1*H*-indol-2-formic acid (255.36 mg, 0.93 mmol) were dissolved in *N*,*N-*dimethylformamide (8 mL). Then, *N*,*N*-diisopropylethylamine (329.32 mg, 2.55 mmol) and HATU (419.83 mg, 1.10 mmol) were added in sequence to react at 20°C for 16 h. After the reaction was completed, the reaction solution was poured into water (100 mL) and extracted with ethyl acetate (100 mL×3). The organic phases were washed with a saturated saline solution (50 mL×2), dried with anhydrous sodium sulfate, filtered, concentrated under reduced pressure, and separated and purified by silica gel column chromatography (EA:PE (v/v)=50%) to obtain the target compound.

LC-MS (ESI): [M+ H]⁺ =797.3.

### Step 4: Synthesis of [5-[(4S)-2,2-dimethyl-3,4,5,6-tetrahydro-2H-pyran-4-yl]-2-[[(4'S)-3'-[3-(1-cyclopropyl-7-fluoro-1H-indazol-5-yl)-2-oxo-2,3-dihydro-1H-imidazol-1-yl]-2'-(4-fluoro-3,5-dimethylphenyl)-4'-methyl-5',6'-dihydro-4'H-spiro(cyclopropane-1,7'-pyrazolo[4,3-c]pyridine)-5'-yl]carbonyl]indol-1-yl]acetonitrile

3-(1-cyclopropyl-7-fluoro-1*H*-indazol-5-yl)-1-[(4'*S*)-2'-(4-fluoro-3,5-dimethylphenyl)-5'-([5-[(4*S*)-2,2-dimethyl-3,4,5,6-tetrahydro-2*H*-pyran-4-yl]-1*H*-indol-2-yl]carbonyl)-4'-methyl-5',6'-dihydro-4'*H*-spiro(cyclopropane-1,7'-pyrazolo[4,3-*c*]pyridine)-3'-yl]-2,3-dihydro-1*H*-imidazol-2-one (300 mg, 0.38 mmol) was dissolved in *N*,*N*-dimethylformamide (8 mL), and cooled to 0°C. Sodium hydride (150.58 mg, 3.76 mmol, 60%) was added in a nitrogen atmosphere. After 0.5 h, chloroacetonitrile (369.49 mg, 4.89 mmol) was added and stirred at 0°C for 2.5 h. After the reaction was completed, the reaction solution was poured into a saturated ammonium chloride aqueous solution (50 mL) for quenching, and extracted with ethyl acetate (50 mL×3). The organic phases were combined, dried with anhydrous sodium sulfate, filtered, concentrated under reduced pressure, and separated and purified by silica gel column chromatography (EA:PE (v/v)=50%) to obtain the target compound.

LC-MS (ESI): [M + H]⁺ =836.5.

### Step 5: Synthesis of 1-[5-[(4S)-2,2-dimethyl-3,4,5,6-tetrahydro-2H-pyran-4-yl]-2-[[(4'S)-3'-[3-(1-cyclopropyl-7-fluoro-1H-indazol-5-yl)-2-oxo-2,3-dihydro-1H-imidazol-1-yl]-2'-(4-fluoro-3,5-dimethylphenyl)-4'-methyl-5',6'-dihydro-4'H-spiro(cyclopropane-1,7'-pyrazolo[4,3-c]pyridine)-5'-yl]carbonyl]indol-1-yl]cyclopropane-1-carbonitrile

[5-[(4*S*)-2,2-dimethyl-3,4,5,6-tetrahydro-2*H*-pyran-4-yl]-2-[[(4'*S*)-3'-[3-(1-cyclopropyl-7-fluoro-1*H*-indazol-5-yl)-2-oxo-2,3-dihydro-1*H*-imidazol-1-yl]-2'-(4-fluoro-3,5-dimethylphenyl)-4'-methyl-5',6'-dihydro-4'*H*-spiro(cyclopropane-1,7'-pyrazolo[4,3-*c*]pyridine)-5'-yl]carbonyl]indol-1-yl]acetonitrile (300 mg, 0.36 mmol) and ethylene sulfate (111.35 mg, 0.90 mmol) were dissolved in tetrahydrofuran (6 mL), and cooled to 0°C. A 1M solution of lithium bis(trimethylsilyl)amide in toluene (1.4 mL, 1.44 mmol) was slowly added to react at 0°C for 2 h. After the reaction was completed, the reaction solution was poured into a saturated ammonium chloride aqueous solution (30 mL) for quenching, and extracted with ethyl acetate (30 mL×3). The organic phases were combined, dried with anhydrous sodium sulfate, filtered, concentrated under reduced pressure, and separated and purified by silica gel column chromatography (EA:PE (v/v)=50%) to obtain the target compound.

LC-MS (ESI): [M + H]⁺ =862.3.

### Step 6: Synthesis of 1-[2-[(S)-3'-[3-(1-cyclopropyl-7-fluoro-1H-indazol-5-yl)-2-oxo-2,3-dihydro-1H-imidazol-1-yl]-2'-(4-fluoro-3,5-dimethylphenyl)-4'-methyl-2',4',5',6'-tetrahydrospiro(cyclopropane-1,7'-pyrazolo[4,3-c]pyridine)-5'-carbonyl]-5-[(S)-2,2-dimethyltetrahydro-2H-pyran-4-yl]-1H-indol-1-yl]-N-hydroxycyclopropane-1-carboxamidine

A 50% aqueous solution of hydroxylamine (0.5 mL, 16.32 mmol) was added to a solution of 1-[5-[(4*S*)-2,2-dimethyl-3,4,5,6-tetrahydro-2*H*-pyran-4-yl]-2-[[(4'*S*)-3'-[3-(1-cyclopropyl-7-fluoroindazol-5-yl)-2-oxo-2,3-dihydro-1*H*-imidazol-1-yl]-2'-(4-fluoro-3,5-dimethylphenyl)-4'-methyl-5',6'-dihydro-4'H-spiro(cyclopropane-1,7'-pyrazolo[4,3-c]pyridine)-5'-yl]carbonyl]indol-1-yl]cyclopropane-1-carbonitrile (220 mg, 0.26 mmol) in dimethyl sulfoxide (5 mL), and reacted at 30°C for 3 h. After the reaction was completed, the reaction solution was poured into water (60 mL) and extracted with ethyl acetate (60 mL×2). The organic phases were combined, washed with a saturated saline solution (30 mL×2), dried with anhydrous sodium sulfate, filtered, and concentrated under reduced pressure to obtain the target compound.

LC-MS (ESI): [M + H]⁺ =895.4.

### Step 7: Synthesis of 3-([5-[(4S)-2,2-dimethyl-3,4,5,6-tetrahydro-2H-pyran-4-yl]-2-[[(4'S)-3'-[3-(1-cyclopropyl-7-fluoro-1H-indazol-5-yl)-2-oxo-2,3-dihydro-1H-imidazol-1-yl]-2'-(4-fluoro-3,5-dimethylphenyl)-4'-methyl-5',6'-dihydro-4'H-spiro(cyclopropane-1,7'-pyrazolo[4,3-c]pyridine)-5'-yl]carbonyl]indol-1-yl]cyclopropyl]1,2,4-oxadiazol-5(4H)-one

1-(2-((*S*)-3'-(3-(1-cyclopropyl-7-fluoro-1*H*-indazol-5-yl)-2-oxo-2,3-dihydro-1*H*-imidazol-1-yl)-2'-(4-fluoro-3,5-dimethylphenyl)-4'-methyl-2',4',5',6'-tetrahydrospiro[cyclopropane-1,7'-pyrazolo[4,3-*c*]pyridine]-5'-carbonyl)-5-((*S*)-2,2-dimethyltetrahydro-2H-pyran-4-yl)-1*H*-indol-1-yl)-*N*-hydroxycyclopropane-1-carboxamidine (100 mg, 0.11 mmol) was dissolved in dimethyl sulfoxide (2 mL). *N*,*N'*-carbonyldiimidazole (45.29 mg, 0.28 mmol) and 1.8-diazabicyclo[5.4.0]undecane-7-ene (68.04 mg, 0.45 mmol) were added to react at 30°C for 12 h. After the reaction was completed, the reaction solution was directly separated and purified by reverse phase column chromatography (column: Phenomenex Luna C18 150*25 mm*10 um; mobile phase: [A: H₂O (0.225% FA); B: ACN]; B%: 65.00% to 95.00%, 10.00 min; flow rate: 25.00 ml/min) to obtain the target compound.

LC-MS (ESI): [M + H]⁺ =921.5;
¹H NMR (400 MHz, DMSO-*d*6) *δ* = 12.23 - 11.95 (m, 1H), 8.21 - 8.01 (m, 1H), 7.94 - 7.75 (m, 1H), 7.64 - 7.60 (m, 1H), 7.56 - 7.32 (m, 3H), 7.21 (d, *J =* 8.4 Hz, 1H), 7.17 - 6.97 (m, 3H), 6.78 - 6.63 (m, 1H), 5.98 - 5.18 (m, 1H), 4.23 - 3.88 (m, 2H), 3.78 - 3.61 (m, 3H), 3.09 - 2.95 (m, 1H), 2.23 - 2.13 (m, 6H), 1.87 - 1.59 (m, 6H), 1.58 - 1.48 (m, 2H), 1.36 - 1.30 (m, 4H), 1.27 (s, 3H), 1.21 - 1.06 (m, 8H), 1.02 - 0.70 (m, 2H).

### Example 19 Synthesis of 3-((1S,2S)-1-(5-((S)-2,2-dimethyltetrahydro-2H-pyran-4-yl)-2-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4,7,7-trimethyl-4,5,6,7-tetrahydro-2H-pyrazole[4,3-c]pyridine-5-carbonyl)-1H-indol-1-yl)-2-methylcyclopropyl)-1,2,4-oxadiazol-5(4H)-one (Compound 19)

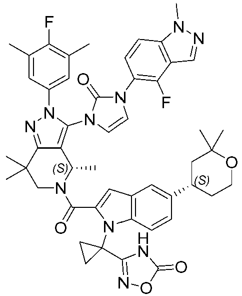

### Step 1: Synthesis of tert-butyl (S)-3-(3-(4-fluoro-1-methyl-1H-indazol-5-yl)-2-oxo-2,3-dihydro-1H-imidazol-1-yl)-2-(4-fluoro-3,5-dimethylphenyl)-4,7,7-tetramethyl-2,4,6,7-tetrahydro-5H-pyrazole[4,3-c]pyridine-5-carboxylate

Tert-butyl (*S*)-2-(4-fluoro-3,5-dimethylphenyl)-4,7,7-trimethyl-3-(2-oxo-2,3-dihydro-1*H-*imidazol-1-yl)-2,4,6,7-tetrahydro-5*H*-pyrazolo[4,3-*c*]pyridine-5-carboxylate (100 mg, 0.21 mmol), 5-bromo-1-cyclopropyl-4-fluoro-1*H*-indazole (97.56 mg, 0.43 mmol), cuprous iodide (13.52 mg, 0.04 mmol), (15,25)-(+)-*N,N'*-dimethyl-1,2-cyclohexanediamine (12.12 mg, 0.09 mmol), potassium carbonate (88.3 mg, 0.64 mmol), and NMP (3 mL) were added to a reaction flask, and heated to 130°C in a nitrogen atmosphere to react for 2 h. A saturated saline solution (30 mL) was added, and the reaction mixture was extracted with ethyl acetate (10 mL×3). The organic phases were combined, dried with anhydrous sodium sulfate, and separated and purified by silica gel column chromatography (EA:PE (v/v)=0% to 60%) to obtain the target compound.

### Step 2: Synthesis of (S)-3-(3-(4-fluoro-1-methyl-1H-indazol-5-yl)-2-oxo-2,3-dihydro-1H-imidazol-1-yl)-2-(4-fluoro-3,5-dimethylphenyl)-4,7,7-trimethyl-2,4,6,7-tetrahydro-5H-pyrazolo[4,3-c]pyridin-5 -one

Tert-butyl (*S*)-3-(3-(4-fluoro-1-methyl-1*H*-indazol-5-yl)-2-oxo-2,3-dihydro-1*H*-imidazol-1-yl)-2-(4-fluoro-3,5-dimethylphenyl)-4,7,7-tetramethyl-2,4,6,7-tetrahydro-5*H*-pyrazole[4,3-*c*]pyridine-5-carboxylate (66 mg, 0.11 mmol) and a 4M dioxane hydrochloride solution (0.3 mL) were added in sequence to a reaction flask to react at room temperature for 1 h, and the reaction mixture was directly concentrated under reduced pressure to obtain the target compound.

### Step 3: Synthesis of 3-((1S,2S)-1-(5-((S)-2,2-dimethyltetrahydro-2H-pyran-4-yl)-2-((S)-3-(3-(4-fluoro-1-methyl-1H-indazol-5-yl)-2-oxo-2,3-dihydro-1H-imidazol-1-yl)-2-(4-fluoro-3,5-dimethylphenyl)-4,7,7-trimethyl-4,5,6,7-tetrahydro-2H-pyrazole[4,3-c]pyridine-5-carbonyl)-1H-indol-1-yl)-2-methylcyclopropyl)-1,2,4-oxadiazol-5(4H)-one

DIPEA (74.92 mg, 0.58 mmol) was added to a solution of (*S*)-3-(3-(4-fluoro-1-methyl-1*H-*indazol-5-yl)-2-oxo-2,3-dihydro-1*H*-imidazol-1-yl)-2-(4-fluoro-3,5-dimethylphenyl)-4,7,7-trimethyl-2,4,6,7-tetrahydro-5*H*-pyrazolo[4,3-*c*]pyridin-5-one (100 mg, 0.19 mmol), 5-((*S*)-2,2-dimethyltetrahydro-2*H*-pyran-4-yl)-1-(1-(5-oxo-4,5-dihydro-1,2,4-oxadiazol-3-yl)cyclopropyl)-1*H*-indol-2-carboxylic acid (76.79 mg, 0.19 mmol), and HATU (110.2 mg, 0.29 mmol) in DMF (2 mL), and reacted overnight at room temperature. The reaction mixture was diluted with water (20 mL) and extracted with ethyl acetate (15 mL×3). The organic phases were combined, washed with a saturated saline solution (20 mL), dried with anhydrous sodium sulfate, filtered, concentrated under reduced pressure, and separated and purified by preparative HPLC to obtain the target compound.

¹H NMR (400 MHz, CDCl₃) δ 8.18 - 8.09 (m, 1H), 7.69 - 7.42 (m, 4H), 7.30 - 7.28 (m, 3H), 7.19 - 7.06 (m, 3H), 6.89 - 5.75 (m, 5H), 4.14 - 4.01 (m, 2H), 3.90 - 3.80 (m, 2H), 3.66 - 3.33 (m, 1H), 3.17 - 2.96 (m, 1H), 2.34 - 2.25 (m, 6H), 1.85 - 1.73 (m, 4H), 1.65 - 1.57 (m, 4H), 1.58 - 1.50 (m, 2H), 1.45 - 1.41 (m, 3H), 1.39 - 1.30 (m, 6H), 1.28 - 1.22 (m, 3H).

### Example 20 Synthesis of 3-[1-[2-((S)-3-[3-(1-cyclopropyl-6-fluoro-1H-indazol-5-yl)-2-oxo-2,3-dihydro-1H-imidazol-1-yl]-2-(4-fluoro-3,5-dimethylphenyl)-4,7,7-trimethyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)cyclopropyl)-1,2,4-oxadiazol-5(4H)-one (Compound 20)

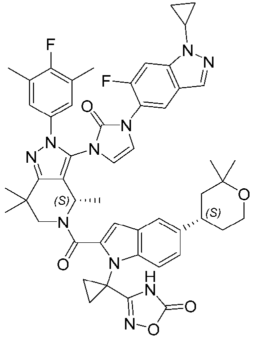

### Step 1: Synthesis of tert-butyl (S)-3-[3-(1-cyclopropyl-6-fluoro-1H-indazol-5-yl)-2-oxo-2,3-dihydro-1H-imidazol-1-yl]-2-(4-fluoro-3,5-dimethylphenyl)-4,7,7-trimethyl-2,4,6,7-tetrahydro-5H-pyrazolo[4,3-c]pyridine-5-carboxylate

*N*-methylpyrrolidone (5 mL), (*S*)-2-(4-fluoro-3,5-dimethylphenyl)-4,7,7-trimethyl-3-(2-oxo-2,3-dihydro-1*H*-imidazol-1-yl)-2,4,6,7-tetrahydro-5*H*-pyrazolo[4,3-*c*]pyridine-5-carboxylate (500 mg, 1.06 mmol), 5-bromo-1-cyclopropyl-6-fluoroindazole (407.44 mg, 1.60 mmol), (1*S*,2*S*)-(+)-*N,N*-dimethylcyclohex-1,2-diamine (60.59 mg, 0.43 mmol), potassium carbonate (441.48 mg, 3.19 mmol), and cuprous iodide (67.58 mg, 0.21 mmol) were added in sequence to a reaction flask, and heated to 100°C in a nitrogen atmosphere to react for 12 h. After the reaction was completed, the reaction mixture was cooled to room temperature, the reaction was quenched with water (300 mL), and the reaction mixture was extracted with ethyl acetate (300 mL×3). The organic phases were combined, dried with anhydrous sodium sulfate, filtered, concentrated under reduced pressure, and separated and purified by silica gel column chromatography (EA:PE (v/v)=50%) to obtain the target compound.

LC-MS (ESI): [M + H]⁺ =644.4.

### Step 2: Synthesis of (S)-1-(1-cyclopropyl-6-fluoro-1H-indazol-5-yl)-3-[2-(4-fluoro-3,5-dimethylphenyl)-4,7,7-trimethyl-2,4,6,7-tetrahydro-2H-pyrazolo[4,3-c]pyridin-3-yl]-1,3-dihydro-2H-imidazol-2-one

A dioxane hydrochloride solution (20 mL, 20.00 mmol) and tert-butyl (*S*)-3-[3-(1-cyclopropyl-6-fluoro-1*H*-indazol-5-yl)-2-oxo-2,3-dihydro-1*H*-imidazol-1-yl]-2-(4-fluoro-3,5-dimethylphenyl)-4,7,7-trimethyl-2,4,6,7-tetrahydro-5*H*-pyrazolo[4,3-*c*]pyridine-5-carboxylate (550 mg, 0.85 mmol) were added in sequence to a reaction flask and stirred at 40°C for 1 h. After the reaction was completed, the reaction solution was directly concentrated under reduced pressure to obtain the target compound.

LC-MS (ESI): [M+H]⁺ =544.3.

### Step 3: Synthesis of 3-(1-cyclopropyl-6-fluoro-1H-indazol-5-yl)-1-[(4S)-2-(4-fluoro-3,5-dimethylphenyl)-5-[5-[(4S)-2,2-dimethyl-3,4,5,6-tetrahydro-2H-pyran-4-yl]-1H-indol-2-carbonyl]-4,7,7-trimethyl-4,5,6,7-tetrahydro-2H-pyrazolo[4,3-c]pyridin-3-yl]-1,3-dihydro-2H-imidazol-2-one

*N*,*N*-dimethylformamide (5 mL), (*S*)-1-(1-cyclopropyl-6-fluoro-1*H*-indazol-5-yl)-3-[2-(4-fluoro-3,5-dimethylphenyl)-4,7,7-trimethyl-2,4,6,7-tetrahydro-2*H*-pyrazolo[4,3-*c*]pyridin-3-yl]-1,3-dihydro-2*H*-imidazol-2-one (460 mg, 0.85 mmol), 5-[(4*S*)-2,2-dimethyl-3,4,5,6-tetrahydro-2*H*-pyran-4-yl]-1*H*-indol-2-formic acid (247.38 mg, 0.91 mmol), *N*,*N*-diisopropylethylamine (389.92 mg, 3.02 mmol), and HATU (430.17 mg, 1.13 mmol) were added in sequence to a reaction flask, and reacted at 25°C for 3 h. After the reaction was completed, the reaction solution was slowly poured into water (100 mL) for quenching, and then extracted with ethyl acetate (100 mL×3). The organic phases were combined, dried with anhydrous sodium sulfate, filtered, concentrated under reduced pressure, and separated and purified by silica gel column chromatography (EA:PE (v/v)=0% to 60%) to obtain the target compound.

LC-MS (ESI): [M+H ]⁺ =799.3.

### Step 4: Synthesis of 2-[2-[(S)-3-[3-(1-cyclopropyl-6-fluoro-1H-indazol-5-yl)-2-oxo-2,3-dihydro-1H-imidazol-1-yl]-2-(4-fluoro-3,5-dimethylphenyl)-4,7,7-trimethyl-4,5,6,7-tetrahydro-2H-pyrazolo[4,3-c]pyridine-5-carbonyl]-5-[(S)-2,2-dimethyltetrahydro-2H-pyran-4-yl]-1H-indol-1-yl]acetonitrile

*N,N*-dimethylformamide (5 mL) and 3-(1-cyclopropyl-6-fluoro-1*H*-indazol-5-yl)-1-[(4*S*)-2-(4-fluoro-3,5-dimethylphenyl)-5-[5-[(4*S*)-2,2-dimethyl-3,4,5,6-tetrahydro-2*H*-pyran-4-yl]-1*H-*indol-2-carbonyl]-4,7,7-trimethyl-4,5,6,7-tetrahydro-2*H*-pyrazolo[4,3-*c*]pyridin-3-yl]-1,3-dihydro-2*H*-imidazol-2-one (400 mg, 0.50 mmol) were added to a reaction flask. The system was cooled to 0°C, and then sodium hydride (200.77 mg, 5.02 mmol) was added in a nitrogen atmosphere, and stirred at 0°C for 0.5 h under nitrogen protection. Chloroacetonitrile (492.65 mg, 6.53 mmol) was added and stirred at 0°C for 1.5 h. After the reaction was completed, the reaction solution was poured into a saturated ammonium chloride aqueous solution (300 mL) for quenching, and extracted with ethyl acetate (300 mL×3). The organic phases were combined, dried with anhydrous sodium sulfate, filtered, concentrated under reduced pressure, and separated and purified by silica gel column chromatography (EA:PE (v/v)=0% to 60%) to obtain the target compound.

LC-MS (ESI): [M+H]⁺ =838.5.

### Step 5: Synthesis of 1-[2-[(S)-3-[3-(1-cyclopropyl-6-fluoro-1H-indazol-5-yl)-2-oxo-2,3-dihydro-1H-imidazol-1-yl]-2-(4-fluoro-3,5-dimethylphenyl)-4,7,7-trimethyl-4,5,6,7-tetrahydro-2H-pyrazolo[4,3-c]pyridine-5-carbonyl]-5-[(S)-2,2-dimethyltetrahydro-2H-pyran-4-yl]-1H-indol-1-yl]cyclopropane-1-carbonitrile

A 1.0M solution of lithium bis(trimethylsilyl)amide in tetrahydrofuran (1.0 mL, 0.95 mmol) was slowly added at 0°C to a solution of 2-[2-[(*S*)-3-[3-(1-cyclopropyl-6-fluoroindazol-5-yl)-2-oxo-2,3-dihydro-1*H*-imidazol-1-yl]-2-(4-fluoro-3,5-dimethylphenyl)-4,7,7-trimethyl-4,5,6,7-tetrahydro-2*H*-pyrazolo[4,3-*c*]pyridine-5-carbonyl]-5-[(*S*)-2,2-dimethyltetrahydro-2H-pyran-4-yl]-1*H*-indol-1-yl]acetonitrile (200 mg, 0.24 mmol) and ethylene sulfate (74.05 mg, 0.60 mmol) in tetrahydrofuran (10 mL), and stirred at 0°C for 2 h. After the reaction was completed, the reaction solution was poured into a saturated ammonium chloride aqueous solution (200 mL) for quenching, and extracted with ethyl acetate (200 mL×2). The organic phases were combined, dried with anhydrous sodium sulfate, filtered, concentrated under reduced pressure, and separated and purified by silica gel column chromatography (EA:PE (v/v)=33%) to obtain the target compound.

LC-MS (ESI): [M+H]⁺ =864.4.

### Step 6: Synthesis of 1-[2-[(S)-3-[3-(1-cyclopropyl-6-fluoro-1H-indazol-5-yl)-2-oxo-2,3-dihydro-1H-imidazol-1-yl]-2-(4-fluoro-3,5-dimethylphenyl)-4,7,7-trimethyl-4,5,6,7-tetrahydro-2H-pyrazolo[4,3-c]pyridine-5-carbonyl]-5-[(S)-2,2-dimethyltetrahydro-2H-pyran-4-yl]-1H-indol-1-yl]-N-hydroxycyclopropane-1-carboxamidine

A 50% hydroxylamine aqueous solution (0.1 mL, 2.95 mmol) was added to a solution of 1-[2-[(*S*)-3-[3-(1-cyclopropyl-6-fluoro-1*H*-indazol-5-yl)-2-oxo-2,3-dihydro-1*H*-imidazol-1-yl]-2-(4-fluoro-3,5-dimethylphenyl)-4,7,7-trimethyl-4,5,6,7-tetrahydro-2*H*-pyrazolo[4,3-*c*]pyridine-5-carbonyl]-5-[(*S*)-2,2-dimethyltetrahydro-2*H*-pyran-4-yl]-1*H*-indol-1-yl]cyclopropane-1-carbonitrile (85 mg, 0.10 mmol) in dimethyl sulfoxide (1 mL), and stirred at 25°C for 12 h. After the reaction was completed, the reaction solution was poured into water (100 mL) and then extracted with ethyl acetate (100 mL×2). The organic phases were combined, dried with anhydrous sodium sulfate, filtered, and concentrated under reduced pressure to obtain the target compound.

LC-MS (ESI): [M+H]⁺ =897.5.

### Step 7: Synthesis of 3-[1-[2-((S)-3-[3-(1-cyclopropyl-6-fluoro-1H-indazol-5-yl)-2-oxo-2,3-dihydro-1H-imidazol-1-yl]-2-(4-fluoro-3,5-dimethylphenyl)-4,7,7-trimethyl-4,5,6,7-tetrahydro-2H-pyrazolo[4,3-c]pyridine-5-carbonyl)-5-((S)-2,2-dimethyltetrahydro-2H-pyran-4-yl)-1H-indol-1-yl)cyclopropyl)-1,2,4-oxadiazol-5(4H)-one

1,8-diazabicyclo[5.4.0]undecane-7-ene (61.1 mg, 0.40 mmol) was added to a solution of 1-[2-[(*S*)-3-[3-(1-cyclopropyl-6-fluoro-1*H*-indazol-5-yl)-2-oxo-2,3-dihydro-1*H*-imidazol-1-yl]-2-(4-fluoro-3,5-dimethylphenyl)-4,7,7-trimethyl-4,5,6,7-tetrahydro-2*H*-pyrazolo[4,3-*c*]pyridine-5-carbonyl]-5-[(*S*)-2,2-dimethyltetrahydro-2*H*-pyran-4-yl]-1*H*-indol-1-yl]-*N-*hydroxycyclopropane-1-carboxamidine (90 mg, 0.10 mmol) and *N*,*N'*-carbonyldiimidazole (48.81 mg, 0.30 mmol) in dimethyl sulfoxide (1.5 mL), and reacted at 25°C for 12 h. After the reaction was completed, the reaction solution was directly separated and purified by reverse phase column chromatography (column: Phenomenex luna C18 150*25 mm*10 um; mobile phase: [A: H₂O (0.05% HCl); B: ACN]; B%: 62.00% to 92.00%, 13.00 min; flow rate: 25.00 ml/min) to obtain the target compound.

LC-MS (ESI): [M + H]⁺ =923.4;
¹H NMR (400 MHz, DMSO-*d*₆) *δ* = 12.25 - 11.98 (m, 1H), 8.26 - 8.03 (m, 1H), 8.01 - 7.83 (m, 1H), 7.82 - 7.69 (m, 1H), 7.61 - 7.41 (m, 2H), 7.24 - 7.07 (m, 3H), 7.05 - 6.92 (m, 1H), 6.89 - 6.58 (m, 2H), 5.86 - 5.14 (m, 1H), 4.66 - 3.90 (m, 1H), 3.79 - 3.66 (m, 3H), 3.10 - 2.94 (m, 1H), 2.59 - 2.49 (m, 2H), 2.25 (s, 6H), 1.90 - 1.80 (m, 1H), 1.74 - 1.62 (m, 1H), 1.77 - 1.59 (m, 4H), 1.56 - 1.39 (m, 5H), 1.35 (m, 3H), 1.28 (m, 3H), 1.18 (m, 5H), 1.13 (m, 4H).

### Example 21 Synthesis of 3-((1S,2S)-1-(5-((S)-2,2-dimethyltetrahydro-2H-pyran-4-yl)-2-((S)-3-(3-(4-fluoro-1-(oxetan-3-yl)-1H-indazol-5-yl)-2-oxo-2,3-dihydro-1H-imidazol-1-yl)-2-(4-fluoro-3,5-dimethylphenyl)-4,7,7-trimethyl-4,5,6,7-tetrahydro-2H-pyrazole[4,3-c]pyridine-5-carbonyl)-1H-indol-1-yl)-2-methylcyclopropyl)-1,2,4-oxadiazol-5(4H)-one

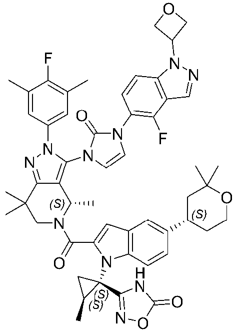

### Step 1: Synthesis of tert-butyl (S)-3-(3-(4-fluoro-1-methyl-1H-indazol-5-yl)-2-oxo-2,3-dihydro-1H-imidazol-1-yl)-2-(4-fluoro-3,5-dimethylphenyl)-4,7,7-tetrahydromethyl-2,4,6,7-tetrahydro-5H-pyrazole[4,3-c]pyridine-5-carboxylate

Tert-butyl (*S*)-2-(4-fluoro-3,5-dimethylphenyl)-4,7,7-trimethyl-3-(2-oxo-2,3-dihydro-1*H-*imidazol-1-yl)-2,4,6,7-tetrahydro-5*H*-pyrazole[4,3-*c*]pyridine-5-carboxylate (300 mg, 0.64 mmol), 5-bromo-4-fluoro-1-(oxetan-3-yl)-1*H*-indazole (173.2 mg, 0.64 mmol), cuprous iodide (40.55 mg, 0.13 mmol), (1S,2S)-(+)-*N*,*N*'-dimethyl-1,2-cyclohexanediamine (36.35 mg, 0.26 mmol), potassium carbonate (264.89 mg, 1.92 mmol), and NMP (5 mL) were added to a reaction flask, and heated to 130°C in a nitrogen atmosphere to react for 2 h. A saturated saline solution (30 mL) was added, and the reaction mixture was extracted with ethyl acetate (10 mL×3). The organic phases were combined, dried with anhydrous sodium sulfate, and separated and purified by silica gel column chromatography (EA:PE (v/v)=0% to 60%) to obtain the target compound.

### Step 2: Synthesis of (S)-1-(4-fluoro-1-(oxetane)-1H-indazol-5-yl)-3-(2-(4-fluoro-3,5-dimethylphenyl)-4,7,7-trimethyl-4,5,6,7-tetrahydro-2H-pyrazole[4,3-c]pyridin-3-yl)-1,3-dihydro-2H-imidazol-2-one

Trifluoroacetic acid (1 mL) was added to a solution (1 mL) of tert-butyl (*S*)-3-(3-(4-fluoro-1-methyl-1*H*-indazol-5-yl)-2-oxo-2,3-dihydro-1*H*-imidazol-1-yl)-2-(4-fluoro-3,5-dimethylphenyl)-4,7,7-tetrahydromethyl-2,4,6,7-tetrahydro-5*H*-pyrazole[4,3-*c*]pyridine-5-carboxylate (100 mg, 0.15 mmol) in dichloromethane, reacted at room temperature for 1 h, and the reaction mixture was directly concentrated under reduced pressure to obtain the target compound.

### Step 3: Synthesis of 3-((1S,2S)-1-(5-((S)-2,2-dimethyltetrahydro-2H-pyran-4-yl)-2-((S)-3-(3-(4-fluoro-1-(oxetan-3-yl)-1H-indazol-5-yl)-2-oxo-2,3-dihydro-1H-imidazol-1-yl)-2-(4-fluoro-3,5-dimethylphenyl)-4,7,7-trimethyl-4,5,6,7-tetrahydro-2H-pyrazole[4,3-c]pyridine-5-carbonyl)-1H-indol-1-yl)-2-methylcyclopropyl)-1,2,4-oxadiazol-5(4H)-one

DIPEA (69.8 mg, 0.54 mmol) was added to a solution of (*S*)-1-(4-fluoro-1-(oxetane)-1*H-*indazol-5-yl)-3-(2-(4-fluoro-3,5-dimethylphenyl)-4,7,7-trimethyl-4,5,6,7-tetrahydro-2*H-*pyrazole[4,3-*c*]pyridin-3-yl)-1,3-dihydro-2*H*-imidazol-2-one (100 mg, 0.21 mmol), 5-((*S*)-2,2-dimethyltetrahydro-2*H*-pyran-4-yl)-1-((1*S*,2*S*)-2-methyl-1-(5-oxo-4,5-dihydro-1,2,4-oxadiazol-3-yl)cyclopropyl)-1*H*-indol-2-carboxylic acid (71 mg, 0.21 mmol), and HATU (110 mg, 0.27 mmol) in DMF (3 mL), and reacted overnight at room temperature. The reaction mixture was diluted with water (20 mL) and extracted with ethyl acetate (15 mL×3). The organic phases were combined, washed with a saturated saline solution (20 mL), dried with anhydrous sodium sulfate, filtered, concentrated under reduced pressure, and separated and purified by preparative HPLC to obtain the target compound.

¹H NMR (400 MHz, CDCl₃) δ 11.30 (s, 1H), 8.33 - 8.23 (m, 1H), 7.68 - 7.58 (m, 2H), 7.56 - 7.49 (m, 1H), 7.40 - 7.28 (m, 5H), 7.23 - 7.03 (m, 3H), 6.89 - 6.08 (m, 3H), 5.81 - 5.71 (m, 2H), 5.27 - 5.15 (m, 4H), 4.19 - 4.05 (m, 2H), 3.65 - 3.54 (m, 2H), 3.44 - 3.22 (m, 1H), 2.95 - 2.83 (m, 1H), 2.33 - 2.44 (m, 6H), 1.87 - 1.80 (m, 3H), 1.60 - 1.57 (m, 15H), 1.23 - 1.09 (m, 3H).

### Experimental Example 1 In vitro cell viability test

### 1. Materials

### (1) Cell strain

This cell was constructed by Wuxi New Drug Development Co., Ltd. Shanghai, as shown in Table 1.

**Table 1**

| Target spot | Host cell |
|---|---|
| GLP-1 | HEK293 |

(2) The reagents are shown in Table 2 below.

**Table 2**

| Name | Item No. | Manufacturer |
|---|---|---|
| Cyclic adenosine monophosphate detection kit | 62AM4PEJ | Cisbio |
| 1M HEPES | 15630-106 | Invitrogen |
| HBSS 1X | 14025 | Invitrogen |
| BSA | B2064-100G | Sigma |
| IBMX | I5879-5G | Sigma |
| Special 384-well plate for liquid handling equipment | PP-0200 | Labcyte |
| 384-well plate | 6007299 | PerkinElmer |

(3) Instruments are shown in Table 3 below.

**Table 3**

| Name | Model | Manufacturer |
|---|---|---|
| Microplate reader | envision2014 | PerkinElmer |
| Cell counter | Vi-CELL^{™} XR Cell Viability Analyzer | Beckman |
| Automatic pipetting workstation | Bravo V11 | Agilent |
| Liquid handling equipment | ECHO 555 | Labcyte |
| Centrifuge | Allegra^{™} 25R Centrifuge | Beckman |

### (4) Compound information

Compounds were prepared into compound solutions with working concentrations of 100 µM or 10 µM with DMSO. 4-fold dilution of 10 points (with a maximum concentration of 1000 nM or 100 nM after dilution) was performed using an automatic pipetting workstation.

### 2. Method

### (1) Experimental materials

Experimental buffers are shown in Table 4 below.

**Table 4**

| Reagent | Final concentration |
|---|---|
| HBSS | 1x |
| HEPES | 5mM |
| 10% BSA | 0.1% |
| IBMX | 0.5 mmol/L |

The final concentrations of the prepared detection reagents are shown in Table 5.

**Table 5**

| Reagent | Final concentration |
|---|---|
| Lysis buffer | about 1x |
| D2-cyclic adenosine monophosphate solution | 1x |
| Cyclic adenosine monophosphate antibody solution | 1x |

### (2) Experimental method

### (a) Preparation of compound plate:

4-fold dilution of 10 points with an initial concentration of 100 µM was performed on a compound to be tested using an automatic pipetting workstation.

### (b) Transfer of compound:

(i) 100 nL of 100x compound was transferred to a 384-well plate using liquid handling equipment.
(ii) The 384-well plate was centrifuged at 1000 rpm for 5 s.

### (c) Preparation of cell suspension

(i) A HEK293 cell cryovial with high expression of hGLP-1R was quickly thawed in warm water at 37°C.
(ii) The cell suspension was transferred to a 15 mL centrifuge tube and gently rinsed with 10 mL HBSS.
(iii) The centrifuge tube was centrifuged at room temperature at 1000 rpm for 1 min.
(iv) The supernatant was discarded.
(v) The cells were gently rinsed with 10 ml HBSS, centrifuged and settled, and finally the cells were resuspended in an experimental buffer.
(vi) The cell density and viability were measured using a cell counter.
(vii) The concentration of GLP-1R cells was diluted to 1.0*10⁵/mL with an experimental buffer.
(viii) 10 µL of a diluted cell suspension was transferred into the 384-well plate.
(ix) The cells were incubated at room temperature for 30 min.

### (d) Detection:

(i) 10 µL of an 800 nM gradient diluted cAMP standard was added to empty wells of the 384-well plate.
(ii) 10 µL of a cAMP detection reagent was added.
(iii) After incubating at room temperature for 60 min, the value on a microplate reader was read.

The results are shown in Table 6, and Table 6 shows the agonism (EC₅₀) of the compounds provided in some examples of the present invention.

**Table 6. Experimental results of agonism of compounds provided in some examples of the present invention**

| Compound | EC₅₀ (nM) |
|---|---|
| 1 | 1.11 |
| 2 | 0.74 |
| 3 | 0.73 |
| 4 | 1.12 |
| 5 | 1.25 |
| 6 | 1.50 |
| 10 | 0.85 |
| 12 | 1.03 |
| 15 | 1.27 |
| 16 | 0.95 |
| 19 | 1.02 |
| LY3502970 | 0.81 |

Results and discussion: The compounds of the present invention exhibited superior agonism against GLP-1 receptors.

### Experimental Example 2: Pharmacokinetic study of compounds of the present invention in mice

### 1. Experimental materials

C57BL/6 mice: Male, 6 to 8 weeks old, weighing 20 to 30 grams, purchased from Vital River (Beijing) Laboratory Animal Technology Co., Ltd.

Reagents: Chromatographic grade acetonitrile was purchased from Thermo Fisher Scientific, chromatographic grade formic acid was purchased from Beijing Dikema Technology Co., Ltd, and the experimental water was ultrapure water. All other reagents were commercially available analytical grade.

Instrument: AB LCMS-5500 tandem mass spectrometer

### 2. Experimental method

The compounds were weighed and dissolved in 10% DMSO/5% Kolliphor-EL/85% HP-β-CD (20%) or other appropriate systems. The formulations were clear solutions and administered intravenously or by gavage to mice. Blood samples were collected 5 min, 15 min, 30 min, 1 h, 2 h, 4 h, 6 h, 8 h, and 24 h after administration. 30 µL of whole blood was collected from each PK sampling point into EDTA-K2 anticoagulant blood collection tubes and centrifuged at 4°C within 30 min to obtain plasma. The whole blood samples were placed on wet ice before centrifugation. All the collected plasma samples were stored on dry ice or frozen until analysis and detection.

About 1 mg of each of the compounds was weighed and dissolved in DMSO, vortexed and sonicated to obtain a 1 mg/mL standard stock solution. The standard stock solution was diluted with a 50% acetonitrile aqueous solution to obtain standard working solutions with concentrations of 5, 10, 20, 50, 100, 500, 1000, 5000, and 10000 ng/mL. Quality control working solutions with concentrations of 10, 20, 500, and 8000 ng/mL were prepared by the same dilution method. 3 µL of each of the standard working solutions (with the concentrations of 5, 10, 20, 50, 100, 500, 1000, 5000, and 10000 ng/mL) was added to 30 µL of blank C57BL/6 mouse plasma to obtain standard curve samples with a total volume of 33 µL and a concentration of 0.5 to 1000 ng/mL (0.5, 1, 2, 5, 10, 50, 100, 500, or 1000 ng/mL). Control samples with concentrations of (1 ng/ml (low-1), 2 ng/ml (low-2), 50 ng/ml (medium), and 800 ng/ml (high)) were prepared separately.

33 µL of a standard sample, 33 µL of a quality control sample, or 33 µL of an unknown sample (30 µL of plasma and 3 µL of blank solution) was added to 200 µL of acetonitrile containing an internal standard (dexamethasone) to precipitate protein. Then the sample was vortexed for 30 s and centrifuged at 4000 g and 4°C for 15 min, and the supernatant was taken and diluted three times with water. 10 µL of the supernatant diluent was injected into an LC-MS/MS system for quantitative analysis under the following detection conditions:
Chromatographic column: Raptor Biphenyl 2.7 µm 2.1×50 mm
Mobile phase: Solution A: 100% water (0.1% formic acid); Solution B: 95% acetonitrile (0.1% formic acid, 5% water). Gradient elution was performed according to the following table.

| Time (min) | Pump A (%) | Pump B (%) |
|---|---|---|
| 0.20 | 85.0 | 15.0 |
| 1.30 | 0.00 | 100 |
| 1.70 | 0.00 | 100 |
| 1.71 | 85.0 | 15.0 |
| 2.00 | 85.0 | 15.0 |

### 3. Data processing

Pharmacokinetic data analysis was performed using a non-compartmental model of Phoenix TM Winnonlin^{®}8.3 software.

The results are shown in Table 7, and Table 7 shows the pharmacokinetic parameters of the mice.

**Table 7. Experimental results of pharmacokinetic parameters of compounds provided in some examples of the present invention**

| Compound | Route of administration | Dose (mg/kg) | Cₘₐₓ (ng/mL) | AUC_{0-24 h} (h*ng/mL) | T_{1/2} (h) |
|---|---|---|---|---|---|
| 1 | po | 10 | 4583 | 66260 | 27.3 |
| 3 | po | 10 | 9050 | 137222 | 10 |
| 4 | po | 10 | 6937 | 87035 | 24.3 |
| 10 | po | 10 | 4090 | 60283 | 20 |
| 15 | po | 10 | 6877 | 124788 | 20.3 |
| 16 | po | 10 | 8757 | 131001 | 8.71 |
| LY3502970 | po | 10 | 3303 | 32528 | 5.52 |

Results and discussion: The compounds of the present invention were orally absorbed well in mice, with higher exposure, half-life, and bioavailability compared to LY3502970.

### Experimental Example 3: Pharmacokinetic study in rats

### 1. Experimental materials

SD rat: Male, 6 to 8 weeks old, weighing 200 to 300 grams, purchased from SPF (Beijing) Biotechnology Co., Ltd.

Reagents: Chromatographic grade acetonitrile was purchased from Thermo Fisher Scientific, chromatographic grade formic acid was purchased from Beijing Dikema Technology Co., Ltd, and the experimental water was ultrapure water. All other reagents were commercially available analytical grade.

### Instrument: AB LCMS-5500 tandem mass spectrometer

### 2. Experimental method

The compounds were weighed and dissolved in 10% DMSO/5% Kolliphor-EL/85% HP-β-CD (20%) or other appropriate systems. The formulations were clear solutions and administered intravenously or by gavage to rats. Blood samples were collected 5 min, 15 min, 30 min, 1 h, 2 h, 4 h, 6 h, 8 h, and 24 h after administration. 200 µL of whole blood was collected from each PK sampling point into EDTA-K2 anticoagulant blood collection tubes and centrifuged at 4°C within 30 min to obtain plasma. The whole blood samples were placed on wet ice before centrifugation. All the collected plasma samples were stored on dry ice or frozen until analysis and detection.

About 1 mg of each of the compounds of the present invention was weighed and dissolved in DMSO, vortexed and sonicated to obtain a 1 mg/mL standard stock solution. The standard stock solution was diluted with a 50% acetonitrile aqueous solution to obtain standard working solutions with concentrations of 5, 10, 20, 50, 100, 500, 1000, 5000, and 10000 ng/mL. Quality control working solutions with concentrations of 10, 20, 500, and 8000 ng/mL were prepared by the same dilution method. The quality control samples were prepared by the same method as the standard curve samples on the day of analysis. 5 µL of each of the standard working solutions (with the concentrations of 5, 10, 20, 50, 100, 500, 1000, 5000, and 10000 ng/mL) was added to 50 µL of blank SD rat plasma to obtain standard curve samples with a total volume of 55 µL and a concentration of 0.5 to 1000 ng/mL (0.5, 1, 2, 5, 10, 50, 100, 500, or 1000 ng/mL). Control samples with concentrations of (1 ng/ml (low-1), 2 ng/ml (low-2), 50 ng/ml (medium), and 800 ng/ml (high)) were prepared separately.

55 µL of a standard sample, 55 µL of a QC sample, or 55 µL of an unknown sample (50 µL of a plasma sample added to 5 µL of a 50% acetonitrile aqueous solution) was added to 200 µL of acetonitrile containing an internal standard (dexamethasone) to precipitate protein. Then the sample was vortexed for 30 s and centrifuged at 4000 g and 4°C for 15 min, and the supernatant was taken and diluted three times with water. 5 µL of the supernatant diluent was injected into an LC-MS/MS system for quantitative analysis under the following detection conditions:
Chromatographic column: HALO 90A Phenly-Hexyl, 2 µm 2.1×30 mm
Mobile phase: Solution A: 100% water (0.1% formic acid); Solution B: 95% acetonitrile (0.1% formic acid, 5% water). Gradient elution was performed according to the following table.

| Time (min) | Pump A (%) | Pump B (%) |
|---|---|---|
| 0.20 | 90.0 | 10.0 |
| 1.20 | 0.00 | 100 |
| 1.90 | 0.00 | 100 |
| 1.91 | 90.0 | 10.0 |
| 2.30 | 90.0 | 10.0 |

### 3. Data processing

Pharmacokinetic data analysis was performed using a non-compartmental model of Phoenix TM Winnonlin^{®}8.3 software.

The results are shown in Table 8, and Table 8 shows the pharmacokinetic parameters of the rats.

**Table 8. Experimental results of pharmacokinetic parameters of compounds provided in some examples of the present invention**

| Compound | Route of administration | Dose (mg/kg) | Cₘₐₓ (ng/mL) | AUC_{0-24 h} (h*ng/mL) | T_{1/2} (h) |
|---|---|---|---|---|---|
| 1 | po | 5 | 2020 | 21158 | 10.9 |
| 3 | po | 5 | 1553 | 19867 | 10.8 |
| 10 | po | 5 | 1156 | 12486 | 8.23 |
| 15 | po | 5 | 2083 | 20358 | 4.44 |
| 16 | po | 5 | 2320 | 19113 | 2.86 |
| LY3502970 | po | 5 | 1297 | 12502 | 6.93 |

Results and discussion: The experimental results indicate that the compounds of the present invention were orally absorbed well in rats, with higher exposure or half-life compared to LY3502970.

### Experimental Example 4: Pharmacokinetic study of compounds of the present invention in Cynomolgus monkeys

### 1. Experimental materials

Cynomolgus monkeys: Male, 2 to 5 years old, weighing 2 to 6 kilograms, purchased from Ankai Yibo (Zhanjiang) Biotechnology Co., Ltd. or Ankai Yibo (Zhaoqing) Biotechnology Co., Ltd.

Reagents: Chromatographic grade acetonitrile was purchased from Thermo Fisher Scientific, chromatographic grade formic acid was purchased from Beijing Dikema Technology Co., Ltd, and the experimental water was ultrapure water. All other reagents were commercially available analytical grade.

Instrument: AB LCMS-5500 tandem mass spectrometer

### 2. Experimental method

6 Cynomolgus monkeys weighing 2 to 6 kg were divided into two groups. One group received intravenous injection at a dose of 1 mg/kg, and blood samples were collected 0.083, 0.25, 0.5, 1, 2, 4, 8, 24, and 48 h after administration. Another group received a single oral administration at a dose of 5 mg/kg, and blood samples were collected 0.25, 0.5, 1, 2, 4, 8, 24, and 48 h after administration. After pre-treatment, a plasma sample was detected by LC/MS/MS in an MRM mode, and a target compound in the plasma sample was quantified by establishing a suitable standard curve, so as to obtain a drug concentration-time curve. Pharmacokinetic parameters were calculated using a non-compartmental model of WinNonlin software.

The results are shown in Table 9, and Table 9 shows the pharmacokinetic parameters of the Cynomolgus monkeys.

**Table 9. Experimental results of pharmacokinetic parameters of compounds provided in some examples of the present invention**

| Compound | Route of administration | Dose (mg/kg) | Cₘₐₓ (ng/mL) | AUC_{0-24 h} (h*ng/mL) | T_{1/2} (h) |
|---|---|---|---|---|---|
| 1 | po | 5 | 406 | 5291 | 10.6 |
| 10 | po | 5 | 193 | 3673 | 12.6 |
| 15 | po | 5 | 298 | 4599 | 6.33 |
| 16 | po | 5 | 376 | 4826 | 9.2 |
| LY3502970 | po | 5 | 123 | 1086 | 5.22 |

Results and discussion: The compounds of the present invention were orally absorbed well in Cynomolgus monkeys, with high exposure, half-life, and bioavailability.

### Experimental Example 5: Stability in liver microsome

### 1. Experimental materials

| Species of liver microsome | Supplier |
|---|---|
| Human | Corning |

### 2. Experimental method

Preparation of working solutions of compounds: High concentration stock solutions were prepared with DMSO for a test substance and a control drug verapamil powder, and were diluted with DMSO into 100 µM working solutions before use. The final concentration of the test substance and the verapamil was 1 µM.

Preparation of phosphate buffer salt (100 mM, pH 7.4): First, 7.098 g of disodium hydrogen phosphate was weighed, added to 500 mL of pure water, and ultrasonically dissolved to obtain a solution A. 3.400 g of potassium dihydrogen phosphate was weighed, added to 250 mL of pure water, and ultrasonically dissolved to obtain a solution B. The solution B was added to the solution A, with a pH value of 7.4.

Preparation of 10 mM NADPH: Before the experiment, an appropriate amount of NADPH was weighed and prepared into a working solution with a concentration of 10 mM with a phosphate solution.

Preparation of an incubation system: The incubation system was prepared according to the table below, and the incubation system was preheated in a 37°C water bath for 10 min before use.

| **Component** | **Concentration of stock solution** | **Volume** | **Final concentration of the system** |
|---|---|---|---|
| Phosphate buffer salt | 100 mM | 216.25 µL | 100 mM |
| Microsome | 20 mg/mL | 6.25 µL | 0.5 mg/mL |

25 µL of the NADPH or the phosphate buffer salt was transferred to the above incubation system, and 2.5 µL of the 100 µM test substance or verapamil was added. Double parallel samples to which the NADPH was added were prepared; and single parallel NADPH-negative samples were prepared.

30 µL of suspension was taken 0.5, 5, 15, 30, and 60 min later, respectively. 150 µL of acetonitrile containing an internal standard was added to terminate the reaction, and the reaction mixture was vortexed for 10 min.

Then, the reaction mixture was centrifuged at 3220 g for 40 min for protein precipitation. 100 µL of the supernatant was transferred to a sample injection plate, and 100 µL of pure water was added and mixed well for analysis by UPLC-MS/MS.

### 3. Data processing

Calculation of all data was performed using Microsoft Excel. A peak area was detected by extracting an ion chromatogram. By linearly fitting the natural logarithm of the elimination percentage of a parent drug with time, an in vitro half-life (t_{1/2}) of the parent drug was detected.

The in vitro half-life (t_{1/2}) was calculated by the slope: t_{1/2}=0.693/k

The in vitro clearance rate (in µL/min/mg) was calculated using the following formula:${CL}_{int} = 0 . 693 / {t}_{1 / 2} * \left(Incubation system volume\left(μL\right)/protein content\left(mg\right)\right)$

The results are shown in Table 10, and Table 10 shows the experimental results of the stability of the compounds provided in some examples of the present invention in human liver microsome.

**Table 10. Experimental results of stability of compounds provided in some examples of the present invention in human liver microsome**

| Compound | Human T_{1/2} (min) |
|---|---|
| 4 | >184 |
| 6 | >184 |
| 10 | >184 |
| 15 | >184 |
| 16 | >184 |
| LY3502970 | 98 |

Results and discussion: The compounds described in the present invention exhibited good stability, which is significantly higher than that of LY3502970.

### Experimental Example 6: Kinetic solubility

2.5 µL of a 20 mM DMSO stock solution was taken and added to a 96-well plate (n=2) containing 497.5 µL of fasted state simulated intestinal fluid (FaSSIF, pH 6.5). The final concentration was 100 µM (containing 0.5% DMSO). After a stirring rod was added to each well, the plate was sealed and oscillated at 25°C and 1100 rpm for 2 h in a thermostatic oscillator. After the oscillation, the sample was placed in a filter plate and filtered using a vacuum filter. 5 µL of the filtrate was taken, 5 µL of DMSO was added, and then an appropriate amount of an ultrapure water and acetonitrile (1:1) mixed solution containing an internal standard was added to dilute the corresponding fold. The mixture was mixed well for injection analysis by LC-MS/MS.

5 µL of a 100 µM DMSO standard solution was taken, 5 µL of each of different buffer solutions was added, and then an appropriate amount of an ultrapure water and acetonitrile (1:1) mixed solution containing an internal standard was added to dilute the corresponding fold. The mixture was mixed well and analyzed by LC-MS/MS together with the sample.

The solubility values of the sample to be tested and the standard control substance were calculated according to the following formula: $\begin{matrix}Concentration of the compound to be tested = \\ \frac{Peak area of the sample to be tested \times Dilution factor \times Concentration of the control substance}{Peak area of the control substance}\end{matrix}$

The calculation results of the kinetic solubility of the tested compounds are shown in Table 11:

**Table 11. Results of kinetic solubility tests of compounds provided in some examples of the present invention**

| Compound | FaSSIF pH 6.5 (µM) |
|---|---|
| 1 | 85.92 |
| 4 | 37.85 |
| 10 | 5.5 |
| 15 | 63.34 |
| 16 | 54.20 |
| LY3502970 | 0.36 |

Results and discussion: The compounds described in the present invention exhibited good kinetic solubility, which is significantly higher than that of LY3502970.

In the description of this specification, the description with reference to the terms "an embodiment", "some embodiments", "an example", "a specific example", "some examples" or the like means that specific features, structures, materials, or characteristics described in combination with the embodiments or examples are included in at least one of the embodiments or examples of the present invention. In this specification, schematic descriptions for the foregoing terms do not necessarily refer to the same embodiments or examples. In addition, the described specific features, structures, materials, or characteristics may be combined in an appropriate manner in any one or more embodiments or examples. Moreover, without contradiction, a person skilled in the art may combine different embodiments or examples and combine features of different embodiments or examples that are described in this specification.

## Claims

1. A compound, being a compound represented by formula (I), or a stereoisomer, geometric isomer, tautomer, oxynitride, hydrate, solvate, metabolite, pharmaceutically acceptable salt, or prodrug of the compound represented by formula (I), 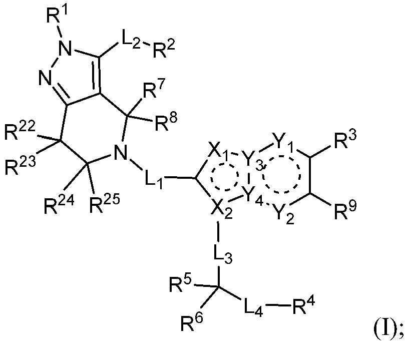 wherein
L₁ is -C(=O)-, -S(=O)₂-, or -CR¹⁰R¹¹-;
L₂ is 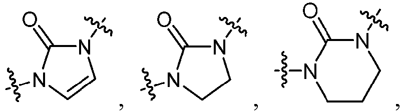 -NR¹²-, -C(=O)NR¹³-, -NR¹⁴C(=O)NR¹⁵-, or - CR¹⁶R¹⁷-;
L₃ is a bond or -CR¹⁸R¹⁹-;
L₄ is a bond or -CR²⁰R²¹-;
X₁ is CH, NH, O, or S;
X₂ is C or N;
Y₁ and Y₂ are each independently CH or N;
Y₃ and Y₄ are each independently C or N;
R¹ is phenyl or 5-10 membered heteroaryl, the phenyl and 5-10 membered heteroaryl being independently and optionally substituted with 1, 2, or 3 R^{1a};
R^{1a} is D, F, Cl, Br, I, CN, hydroxyl, oxo, amino, nitro, C₁₋₆ alkyl, C₁₋₆ alkoxyl, C₁₋₆ alkylthio, C₁₋₆ alkylamino, C₃₋₆ cycloalkyl, or 3-6 membered heterocyclyl, the C₁₋₆ alkyl, C₁₋₆ alkoxyl, C₁₋₆ alkylthio, C₁₋₆ alkylamino, C₃₋₆ cycloalkyl, and 3-6 membered heterocyclyl being independently and optionally substituted with 1, 2, or 3 substituents selected from D, F, Cl, Br, I, hydroxyl, cyano, amino, and nitro;
R² is C₁₋₆ alkyl, C₃₋₈ cycloalkyl, 3-8 membered heterocyclyl, phenyl, or 5-10 membered heteroaryl, the C₁₋₆ alkyl, C₃₋₈ cycloalkyl, 3-8 membered heterocyclyl, phenyl, and 5-10 membered heteroaryl being independently and optionally substituted with 1, 2, or 3 R^{2a};
R^{2a} is D, F, Cl, Br, I, CN, hydroxyl, oxo, amino, nitro, -P(=O)R^{2b}R^{2c}, C₁₋₆ alkyl, C₁₋₆ haloalkyl, C₁₋₆ alkoxyl, C₁₋₆ alkylthio, C₁₋₆ alkylamino, C₃₋₆ cycloalkyl, or 3-6 membered heterocyclyl, the C₁₋₆ alkyl, C₁₋₆ alkoxyl, C₁₋₆ alkylthio, C₁₋₆ alkylamino, C₃₋₆ cycloalkyl, and 3-6 membered heterocyclyl being independently and optionally substituted with 1, 2, or 3 substituents selected from D, F, Cl, Br, I, hydroxyl, cyano, amino, and nitro;
R^{2b} and R^{2c} are each independently C₁₋₆ alkyl or C₃₋₆ cycloalkyl;
or R^{2b} and R^{2c}, together with phosphorus atoms connected therewith, form 5-6 membered heterocyclyl, the 5-6 membered heterocyclyl being optionally substituted with 1, 2, or 3 substituents selected from D, F, Cl, Br, I, hydroxyl, cyano, amino, nitro, methyl, and ethyl;
R³ is 3-8 membered heterocyclyl or 5-10 membered heteroaryl, the 3-8 membered heterocyclyl and 5-10 membered heteroaryl being independently and optionally substituted with 1, 2, or 3 R^{3a};
R^{3a} is D, F, Cl, Br, I, CN, hydroxyl, oxo, amino, nitro, C₁₋₆ alkyl, C₁₋₆ alkoxyl, C₁₋₆ alkylthio, C₁₋₆ alkylamino, C₃₋₆ cycloalkyl, or 3-6 membered heterocyclyl, the C₁₋₆ alkyl, C₁₋₆ alkoxyl, C₁₋₆ alkylthio, C₁₋₆ alkylamino, C₃₋₆ cycloalkyl, and 3-6 membered heterocyclyl being independently and optionally substituted with 1, 2, or 3 substituents selected from D, F, Cl, Br, I, hydroxyl, cyano, amino, and nitro;
R⁴ is 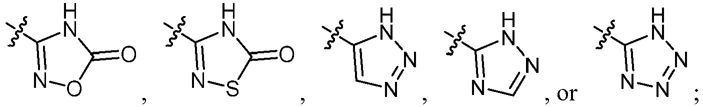
R⁵ and R⁶ are each independently H, D, C₁₋₆ alkyl, C₃₋₆ cycloalkyl, or 3-6 membered heterocyclyl, the C₁₋₆ alkyl, C₃₋₆ cycloalkyl, and 3-6 membered heterocyclyl being independently and optionally substituted with 1, 2, or 3 substituents selected from D, F, Cl, Br, I, hydroxyl, cyano, amino, nitro, and C₃₋₆ cycloalkyl;
or R⁵ and R⁶, together with carbon atoms connected therewith, form C₃₋₆ cycloalkyl, the C₃₋₆ cycloalkyl being optionally substituted with 1, 2, or 3 substituents selected from D, F, Cl, Br, I, hydroxyl, cyano, amino, nitro, C₁₋₃ alkyl, C₁₋₃ haloalkyl, C₁₋₃ alkoxyl, C₁₋₃ alkylthio, and C₁₋₃ alkylamino;
R⁷, R⁸, R¹⁰, R¹¹, R¹⁶ , R¹⁷, R¹⁸ , R¹⁹, R²⁰, R²¹, R²², R²³, R²⁴, and R²⁵ are each independently H, D, F, Cl, Br, I, CN, hydroxyl, oxo, amino, nitro, C₁₋₆ alkyl, C₁₋₆ alkoxyl, C₁₋₆ alkylthio, C₁₋₆ alkylamino, C₃₋₆ cycloalkyl, or 3-6 membered heterocyclyl, the C₁₋₆ alkyl, C₁₋₆ alkoxyl, C₁₋₆ alkylthio, C₁₋₆ alkylamino, C₃₋₆ cycloalkyl, and 3-6 membered heterocyclyl being independently and optionally substituted with 1, 2, or 3 substituents selected from D, F, Cl, Br, I, CN, hydroxyl, amino, or nitro, under a condition that at least one of R²², R²³, R²⁴, and R²⁵ is not hydrogen;
or R²² and R²³, R²⁴ and R²⁵, R²³ and R²⁴, independently and optionally together with carbon atoms connected therewith, form C₃₋₆ cycloalkyl, the C₃₋₆ cycloalkyl being optionally substituted with 1, 2, or 3 substituents selected from D, F, Cl, Br, I, hydroxyl, cyano, amino, nitro, C₁₋₃ alkyl, C₁₋₃ haloalkyl, C₁₋₃ alkoxyl, C₁₋₃ alkylthio, or C₁₋₃ alkylamino;
R⁹ is H, D, F, Cl, Br, I, CN, hydroxyl, amino, nitro, phenyl, C₂₋₆ alkenyl, C₂₋₆ alkynyl, C₁₋₆ alkyl, C₁₋₆ haloalkyl, C₁₋₆ alkoxyl, C₁₋₆ alkylamino, or C₃₋₆ cycloalkyl, the phenyl, C₂₋₆ alkenyl, C₂₋₆ alkynyl, C₁₋₆ alkyl, C₁₋₆ haloalkyl, C₁₋₆ alkoxyl, C₁₋₆ alkylamino, and C₃₋₆ cycloalkyl being independently and optionally substituted with 1, 2, or 3 substituents selected from D, F, Cl, Br, I, CN, hydroxyl, amino, nitro, C₁₋₆ alkoxyl, C₁₋₆ alkylamino, or C₃₋₆ cycloalkyl; and
R¹², R¹³, R¹⁴, and R¹⁵ are each independently H, C₁₋₆ alkyl, C₂₋₆ alkenyl, C₂₋₆ alkynyl, C₃₋₆ cycloalkyl, or 3-6 membered heterocyclyl, the C₁₋₆ alkyl, C₂₋₆ alkenyl, C₂₋₆ alkynyl, C₃₋₆ cycloalkyl, and 3-6 membered heterocyclyl being independently and optionally substituted with 1, 2, or 3 substituents selected from D, F, Cl, Br, I, CN, hydroxyl, amino, and nitro;
wherein formula (I) is not the following compound:

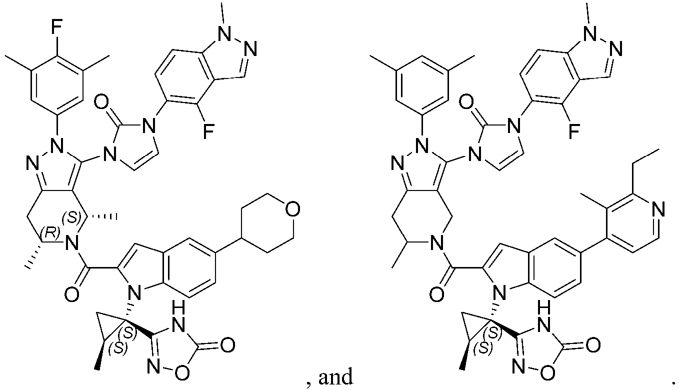

2. The compound according to claim 1, wherein 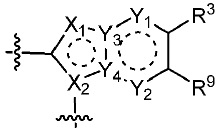 is

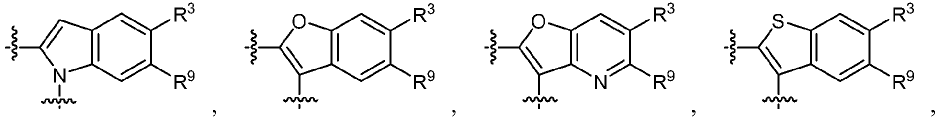

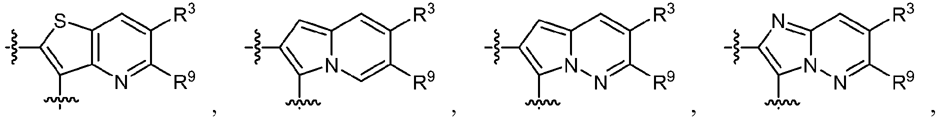

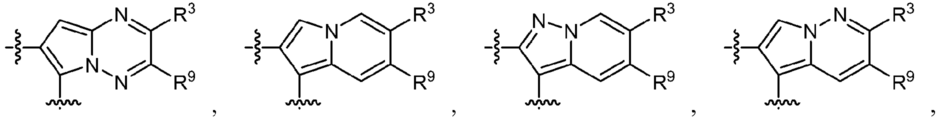

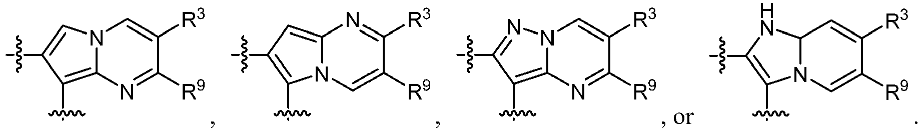

3. The compound according to claim 1 or 2, wherein R¹ is phenyl or 5-10 membered heteroaryl, the phenyl and 5-10 membered heteroaryl being independently and optionally substituted with 1, 2, or 3 R^{1a};
R^{1a} is D, F, Cl, Br, I, CN, hydroxyl, oxo, amino, nitro, C₁₋₃ alkyl, C₁₋₃ alkoxyl, C₁₋₃ alkylthio, C₁₋₃ alkylamino, C₃₋₆ cycloalkyl, or 3-6 membered heterocyclyl, the C₁₋₃ alkyl, C₁₋₃ alkoxyl, C₁₋₃ alkylthio, C₁₋₃ alkylamino, C₃₋₆ cycloalkyl, and 3-6 membered heterocyclyl being independently and optionally substituted with 1, 2, or 3 substituents selected from D, F, Cl, Br, I, hydroxyl, cyano, amino, and nitro;
R² is C₁₋₃ alkyl, C₃₋₆ cycloalkyl, 3-6 membered heterocyclyl, phenyl, or 5-10 membered heteroaryl, the C₁₋₃ alkyl, C₃₋₆ cycloalkyl, 3-6 membered heterocyclyl, phenyl, and 5-10 membered heteroaryl being independently and optionally substituted with 1, 2, or 3 R^{2a};
R^{2a} is D, F, Cl, Br, I, CN, hydroxyl, oxo, amino, nitro, -P(=O)R^{2b}R^{2c}, C₁₋₃ alkyl, C₁₋₃ haloalkyl, C₁₋₃ alkoxyl, C₁₋₃ alkylthio, C₁₋₃ alkylamino, C₃₋₆ cycloalkyl, or 3-6 membered heterocyclyl, the C₁₋₃ alkyl, C₁₋₃ alkoxyl, C₁₋₃ alkylthio, C₁₋₃ alkylamino, C₃₋₆ cycloalkyl, and 3-6 membered heterocyclyl being independently and optionally substituted with 1, 2, or 3 substituents selected from D, F, Cl, Br, I, hydroxyl, cyano, amino, and nitro;
R^{2b} and R^{2c} are each independently C₁₋₆ alkyl or C₃₋₆ cycloalkyl;
or R^{2b} and R^{2c}, together with phosphorus atoms connected therewith, form 5-6 membered heterocyclyl, the 5-6 membered heterocyclyl being optionally substituted with 1, 2, or 3 substituents selected from D, F, Cl, Br, I, hydroxyl, cyano, amino, nitro, methyl, and ethyl;
R³ is 3-6 membered heterocyclyl or 5-10 membered heteroaryl, the 3-6 membered heterocyclyl and 5-10 membered heteroaryl being independently and optionally substituted with 1, 2, or 3 R^{3a}; and
R^{3a} is D, F, Cl, Br, I, CN, hydroxyl, oxo, amino, nitro, C₁₋₃ alkyl, C₁₋₃ alkoxyl, C₁₋₃ alkylthio, C₁₋₃ alkylamino, C₃₋₆ cycloalkyl, or 3-6 membered heterocyclyl, the C₁₋₃ alkyl, C₁₋₃ alkoxyl, C₁₋₃ alkylthio, C₁₋₃ alkylamino, C₃₋₆ cycloalkyl, and 3-6 membered heterocyclyl being independently and optionally substituted with 1, 2, or 3 substituents selected from D, F, Cl, Br, I, hydroxyl, cyano, amino, and nitro.

4. The compound according to any one of claims 1 to 3, wherein R¹ is phenyl, 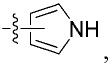 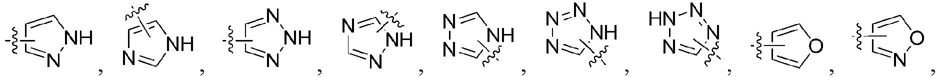 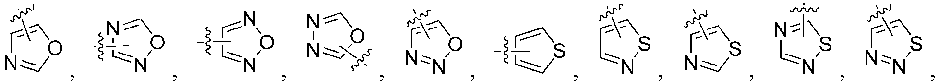 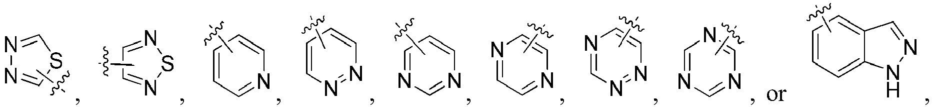 the phenyl, 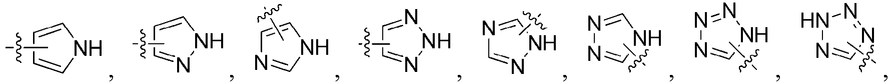 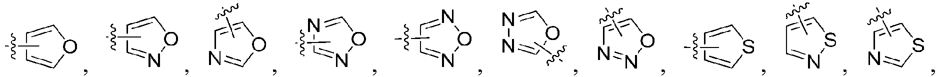 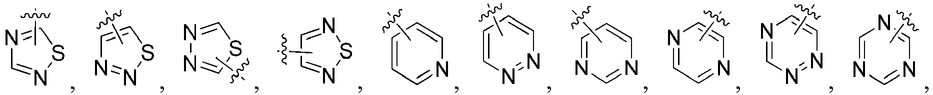 and 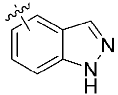 being independently and optionally substituted with 1, 2, or 3 R^{1a};
R^{1a} is D, F, Cl, Br, I, CN, hydroxyl, oxo, amino, nitro, methyl, ethyl, n-propyl, isopropyl, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, or morpholinyl, the methyl, ethyl, n-propyl, isopropyl, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, and morpholinyl being independently and optionally substituted with 1, 2, or 3 substituents selected from D, F, Cl, Br, I, hydroxyl, cyano, amino, and nitro;
R² is methyl, ethyl, n-propyl, isopropyl, cyclopropyl, cyclobutyl, cyclopentyl, cyclohexyl, oxacyclobutyl, azacyclobutyl, pyrrolidinyl, tetrahydrofuranyl, piperidyl, piperazinyl, morpholinyl, phenyl, 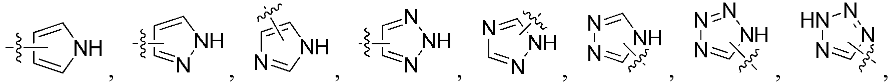 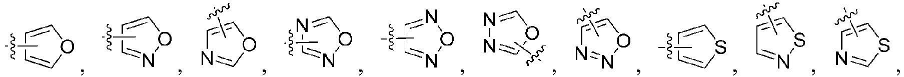 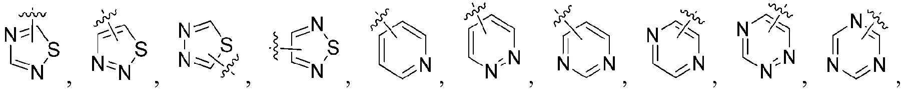 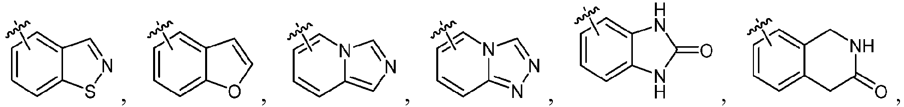 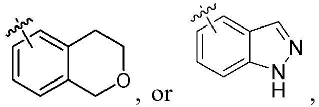 the methyl, ethyl, n-propyl, isopropyl, cyclopropyl, cyclobutyl, cyclopentyl, cyclohexyl, oxacyclobutyl, azacyclobutyl, pyrrolidinyl, tetrahydrofuranyl, piperidyl, piperazinyl, morpholinyl, phenyl, 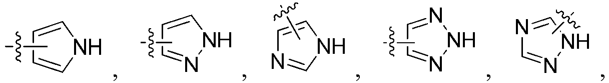 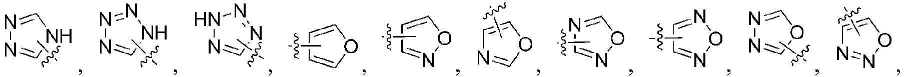 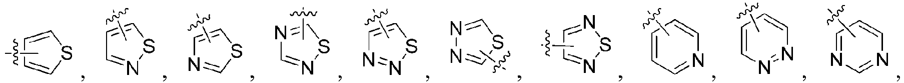 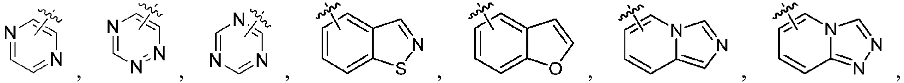 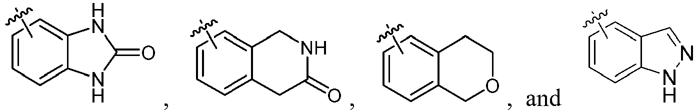 being independently and optionally substituted with 1, 2, or 3 R^{2a};
R^{2a} is D, F, Cl, Br, I, CN, hydroxyl, oxo, amino, nitro, -P(=O)R^{2b}R^{2c}, methyl, ethyl, n-propyl, isopropyl, -CHF₂, -CF₃, -CHFCH₂F, -CF₂CHF₂, -CH₂CF₃, -CH₂CF₂CHF₂, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, or morpholinyl, the methyl, ethyl, n-propyl, isopropyl, -CHF₂, -CHFCH₂F, -CF₂CHF₂, -CH₂CF₃, - CH₂CF₂CHF₂, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, and morpholinyl being independently and optionally substituted with 1, 2, or 3 substituents selected from D, F, Cl, Br, I, hydroxyl, cyano, amino, and nitro;
R^{2b} and R^{2c} are each independently methyl, ethyl, n-propyl, isopropyl, tert-butyl, cyclopropyl, cyclobutyl, cyclopentyl, or cyclohexyl;
or R^{2b} and R^{2c}, together with phosphorus atoms connected therewith, form 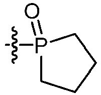
or 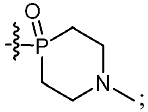
R³ is oxacyclobutyl, azacyclobutyl, pyrrolidinyl, tetrahydrofuranyl, tetrahydropyranyl, piperidyl, piperazinyl, morpholinyl, 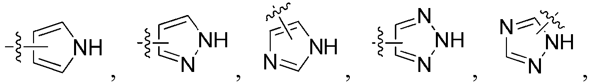 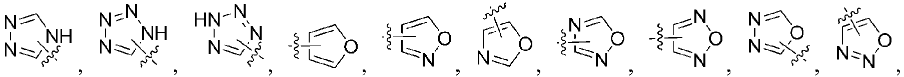 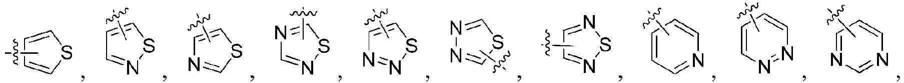 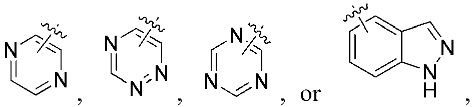 the oxacyclobutyl, azacyclobutyl, pyrrolidinyl, tetrahydrofuranyl, tetrahydropyranyl, piperidyl, piperazinyl, morpholinyl, 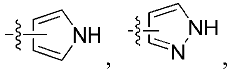 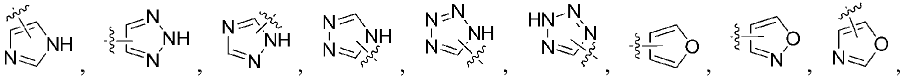 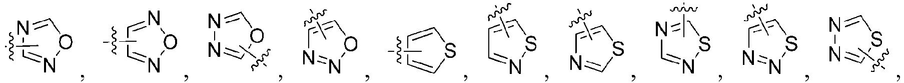 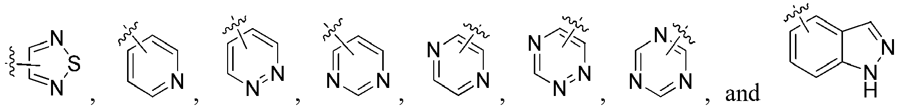 being independently and optionally substituted with 1, 2, or 3 R^{3a}; and
R^{3a} is D, F, Cl, Br, **I,** CN, hydroxyl, oxo, amino, nitro, methyl, ethyl, n-propyl, isopropyl, - CHF₂, -CF₃, -CHFCH₂F, -CF₂CHF₂, -CH₂CF₃, -CH₂CF₂CHF₂, methoxyl, ethoxyl, methylthio, N-methylamino, *N*-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, or morpholinyl, the methyl, ethyl, n-propyl, isopropyl, -CHF₂, -CHFCH₂F, -CF₂CHF₂, -CH₂CF₃, -CH₂CF₂CHF₂, methoxyl, ethoxyl, methylthio, *N*-methylamino, *N*-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, and morpholinyl being independently and optionally substituted with 1, 2, or 3 substituents selected from D, F, Cl, Br, I, hydroxyl, cyano, amino, and nitro.

5. The compound according to any one of claims 1 to 4, wherein R⁵ and R⁶ are each independently H, D, methyl, ethyl, n-propyl, isopropyl, cyclopropyl, cyclobutyl, cyclopentyl, cyclohexyl, oxacyclobutyl, azacyclobutyl, pyrrolidinyl, tetrahydrofuranyl, piperidyl, piperazinyl, or morpholinyl, the methyl, ethyl, n-propyl, isopropyl, cyclopropyl, cyclobutyl, cyclopentyl, cyclohexyl, oxacyclobutyl, azacyclobutyl, pyrrolidinyl, tetrahydrofuranyl, piperidyl, piperazinyl, and morpholinyl being independently and optionally substituted with 1, 2, or 3 substituents selected from D, F, Cl, Br, I, hydroxyl, cyano, amino, nitro, and C₃₋₆ cycloalkyl;
or R⁵ and R⁶, together with carbon atoms connected therewith, form cyclopropyl, cyclobutyl, cyclopentyl, or cyclohexyl, the cyclopropyl, cyclobutyl, cyclopentyl, and cyclohexyl being independently and optionally substituted with 1, 2, or 3 substituents selected from D, F, Cl, Br, I, hydroxyl, cyano, amino, nitro, methyl, ethyl, n-propyl, isopropyl, -CHF₂, -CF₃, -CHFCH₂F, - CF₂CHF₂, -CH₂CF₃, -CH₂CF₂CHF₂, methoxyl, ethoxyl, methylthio, *N*-methylamino, and *N-*ethylamino;
R⁷, R⁸, R¹⁰, R¹¹, R¹⁶, R¹⁷, R¹⁸, R¹⁹, R²⁰, R²¹, R²², R²³, R²⁴, and R²⁵ are each independently H, D, F, Cl, Br, I, CN, hydroxyl, oxo, amino, nitro, methyl, ethyl, n-propyl, isopropyl, -CHF₂, -CF₃, - CHFCH₂F, -CF₂CHF₂, -CH₂CF₃, -CH₂CF₂CHF₂, methoxyl, ethoxyl, methylthio, *N*-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, or morpholinyl, the methyl, ethyl, n-propyl, isopropyl, -CHF₂, -CHFCH₂F, -CF₂CHF₂, -CH₂CF₃, -CH₂CF₂CHF₂, methoxyl, ethoxyl, methylthio, N-methylamino, N-ethylamino, cyclopropyl, cyclobutyl, cyclopentyl, cyclohexyl, oxacyclobutyl, azacyclobutyl, pyrrolidinyl, tetrahydrofuranyl, piperidyl, piperazinyl, and morpholinyl being independently and optionally substituted with 1, 2, or 3 substituents selected from D, F, Cl, Br, I, CN, hydroxyl, amino, and nitro, under a condition that at least one of R²², R²³, R²⁴, and R²⁵ is not hydrogen;
or R²² and R²³, R²⁴ and R²⁵, R²³ and R²⁴, independently and optionally together with carbon atoms connected therewith, form cyclopropyl, cyclobutyl, cyclopentyl, or cyclohexyl, the cyclopropyl, cyclobutyl, cyclopentyl, and cyclohexyl being independently and optionally substituted with 1, 2, or 3 substituents selected from D, F, Cl, Br, I, hydroxyl, cyano, amino, nitro, methyl, ethyl, n-propyl, isopropyl, -CHF₂, -CF₃, -CHFCH₂F, -CF₂CHF₂, -CH₂CF₃, -CH₂CF₂CHF₂, methoxyl, ethoxyl, methylthio, *N*-methylamino, and *N*-ethylamino;
R⁹ is H, D, F, Cl, Br, I, CN, hydroxyl, amino, nitro, phenyl, ethenyl, allyl, ethynyl, propargyl, 1-propynyl, methyl, ethyl, n-propyl, isopropyl, -CHF₂, -CF₃, -CHFCH₂F, -CF₂CHF₂, -CH₂CF₃, - CH₂CF₂CHF₂, methoxyl, ethoxyl, methylthio, *N*-methylamino, *N*-ethylamino, cyclopropyl, cyclobutyl, cyclopentyl, or cyclohexyl, the phenyl, ethenyl, allyl, ethynyl, propargyl, 1-propynyl, methyl, ethyl, n-propyl, isopropyl, -CHF₂, -CHFCH₂F, -CF₂CHF₂, -CH₂CF₃, -CH₂CF₂CHF₂, methoxyl, ethoxyl, methylthio, *N*-methylamino, *N*-ethylamino, cyclopropyl, cyclobutyl, cyclopentyl, and cyclohexyl being independently and optionally substituted with 1, 2, or 3 substituents selected from D, F, Cl, Br, I, CN, hydroxyl, amino, nitro, methoxyl, ethoxyl, methylthio, *N*-methylamino, *N*-ethylamino, cyclopropyl, cyclobutyl, cyclopentyl, and cyclohexyl; and
R¹², R¹³, R¹⁴, and R¹⁵ are each independently H, methyl, ethyl, n-propyl, isopropyl, ethenyl, allyl, ethynyl, propargyl, 1-propynyl, cyclopropyl, cyclobutyl, cyclopentyl, cyclohexyl, oxacyclobutyl, azacyclobutyl, pyrrolidinyl, tetrahydrofuranyl, piperidyl, piperazinyl, or morpholinyl, the methyl, ethyl, n-propyl, isopropyl, ethenyl, allyl, ethynyl, propargyl, 1-propynyl, cyclopropyl, cyclobutyl, cyclopentyl, cyclohexyl, oxacyclobutyl, azacyclobutyl, pyrrolidinyl, tetrahydrofuranyl, piperidyl, piperazinyl, and morpholinyl being independently and optionally substituted with 1, 2, or 3 substituents selected from D, F, Cl, Br, I, CN, hydroxyl, amino, and nitro.

6. The compound according to any one of claims 1 to 5, wherein the compound is a compound having any one of the following structures, or a stereoisomer, geometric isomer, tautomer, oxynitride, hydrate, solvate, metabolite, pharmaceutically acceptable salt, or prodrug of the compound having any one of the following structures: 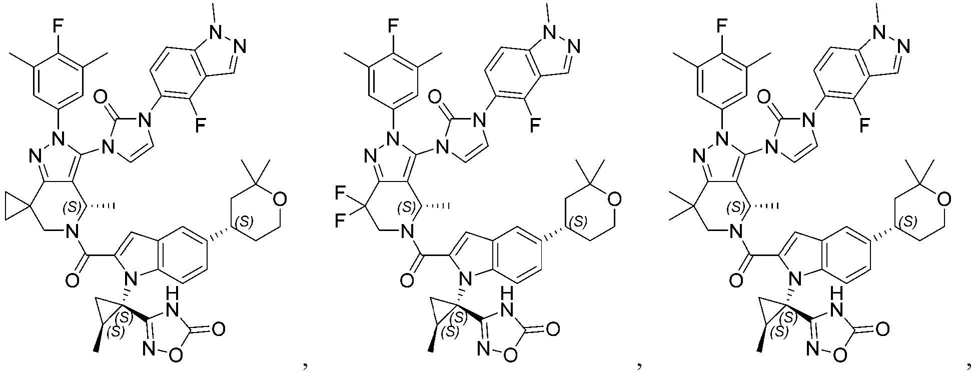 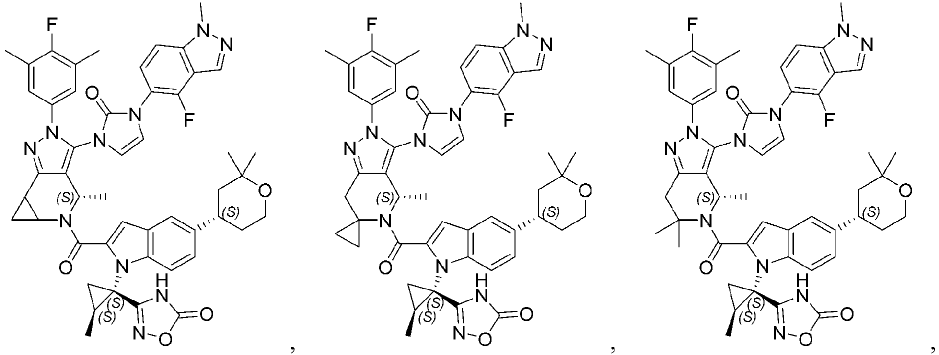 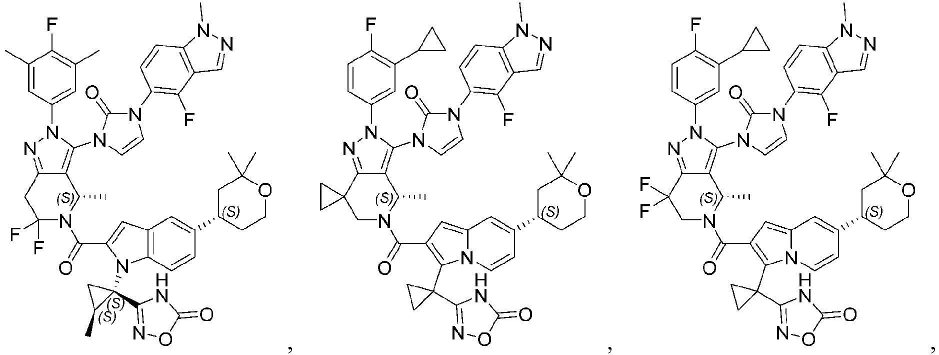 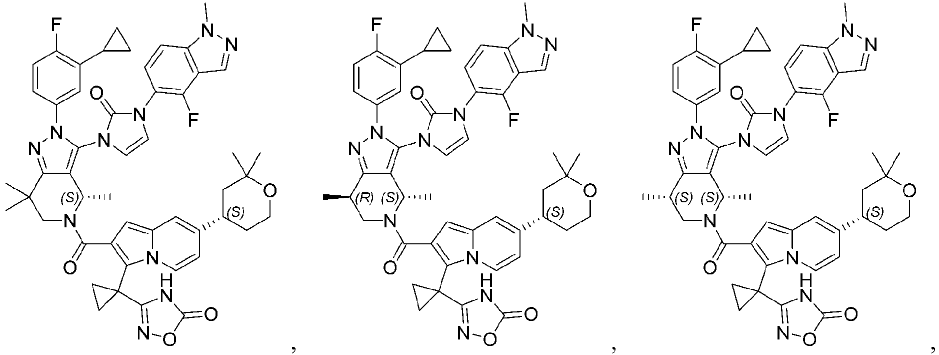 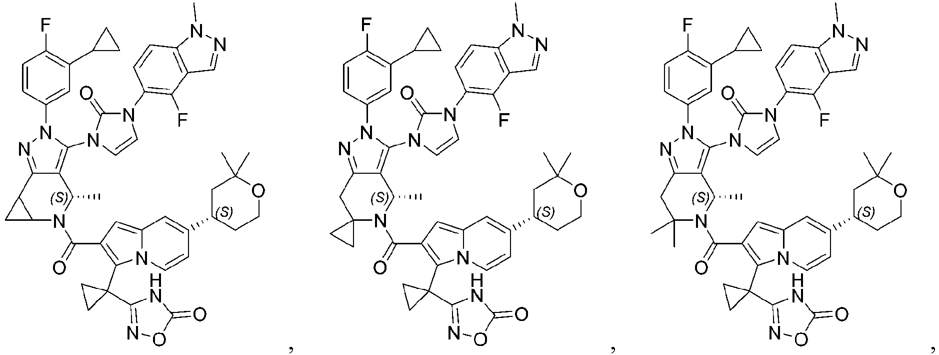 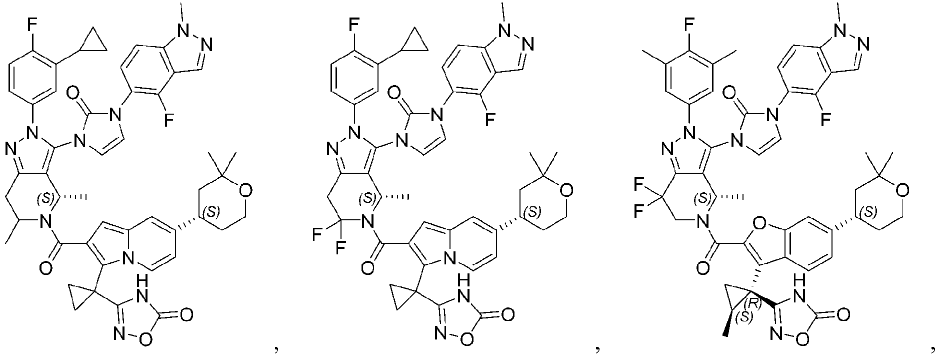 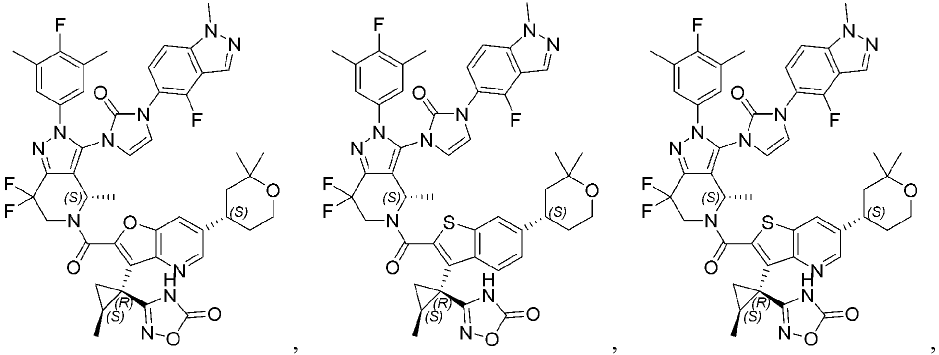 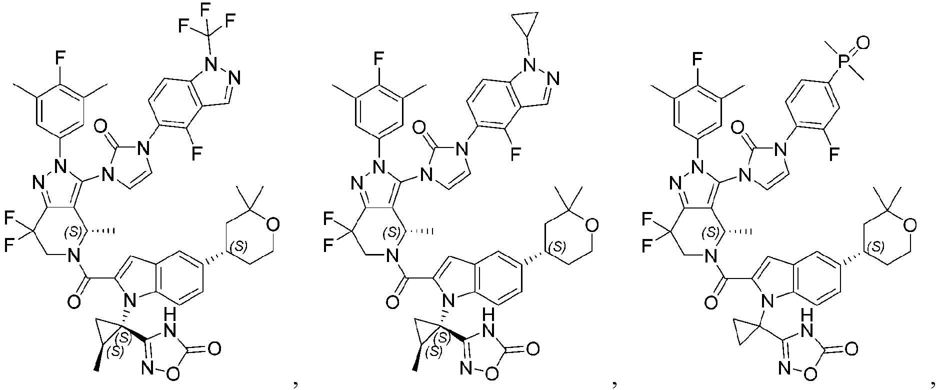 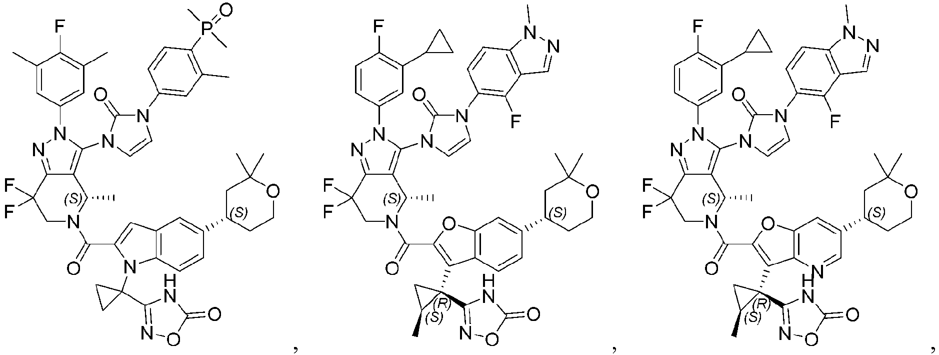 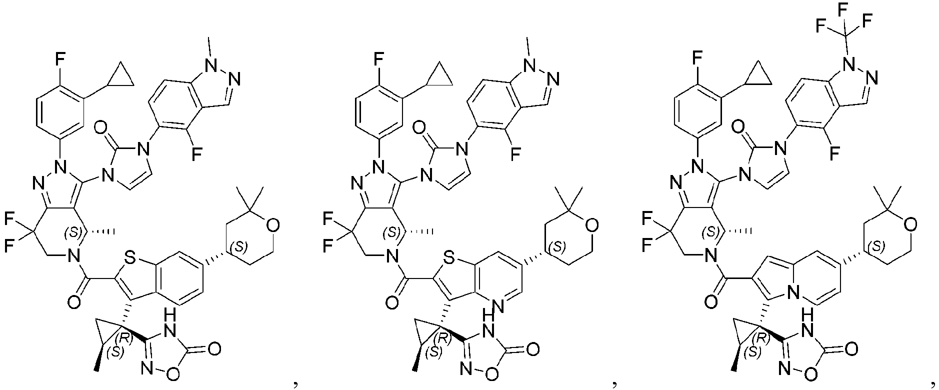 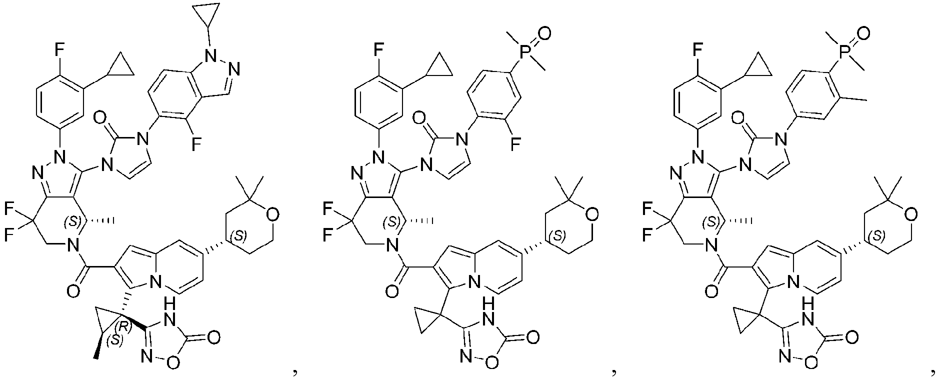 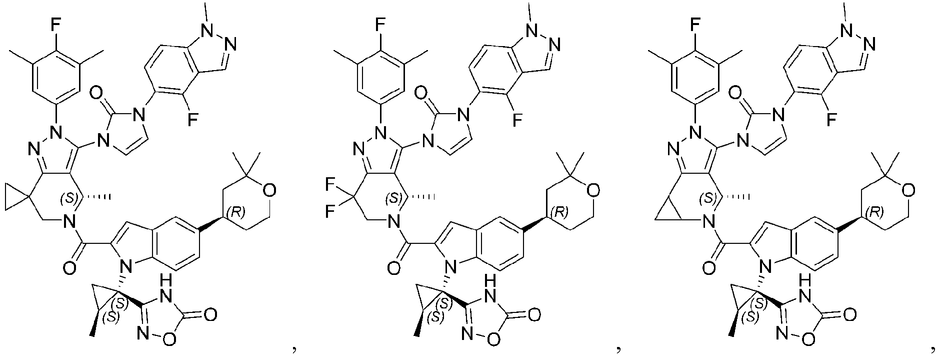 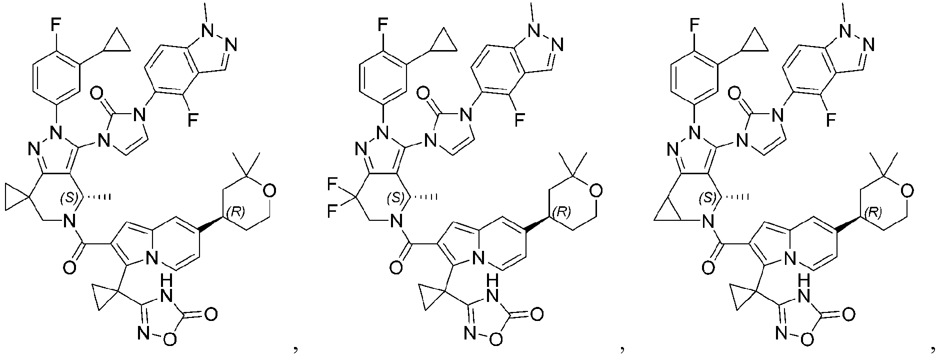 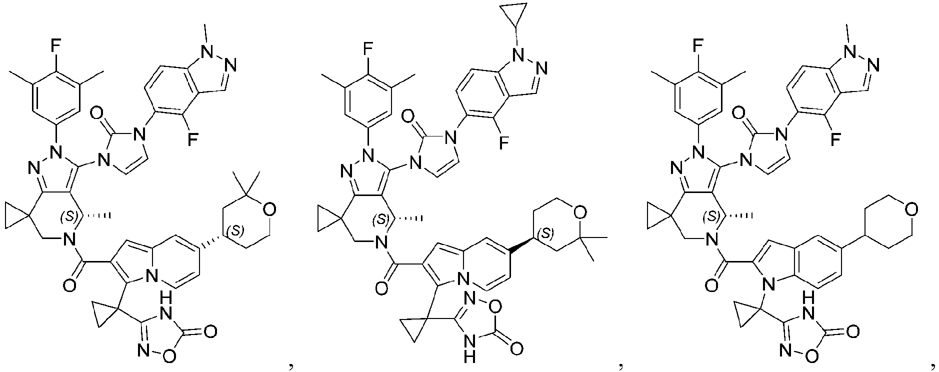 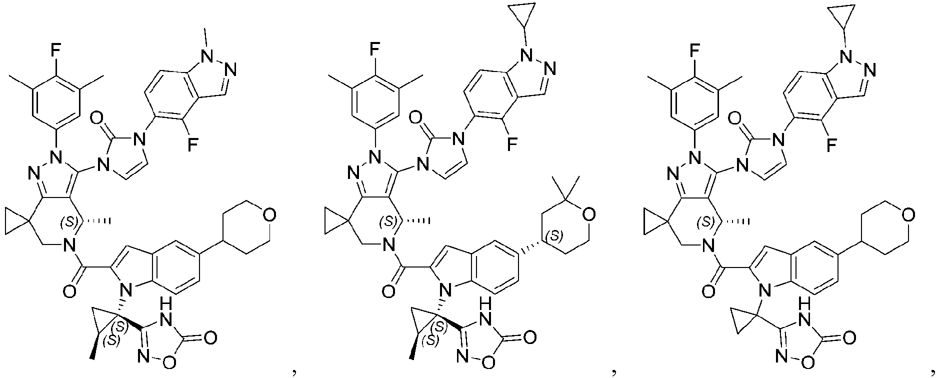 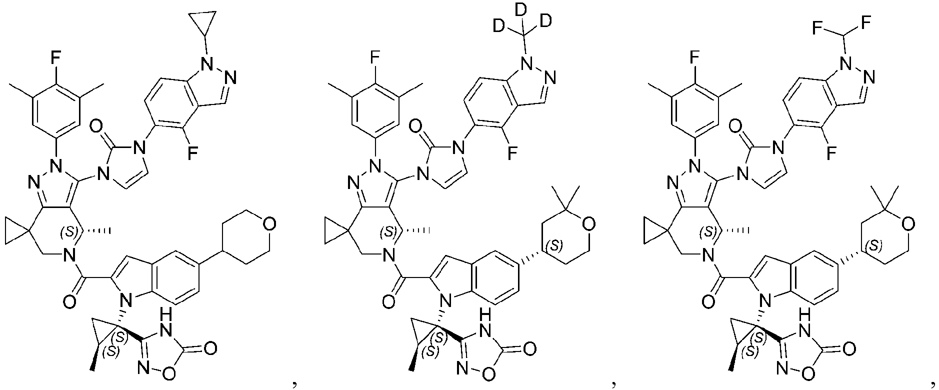 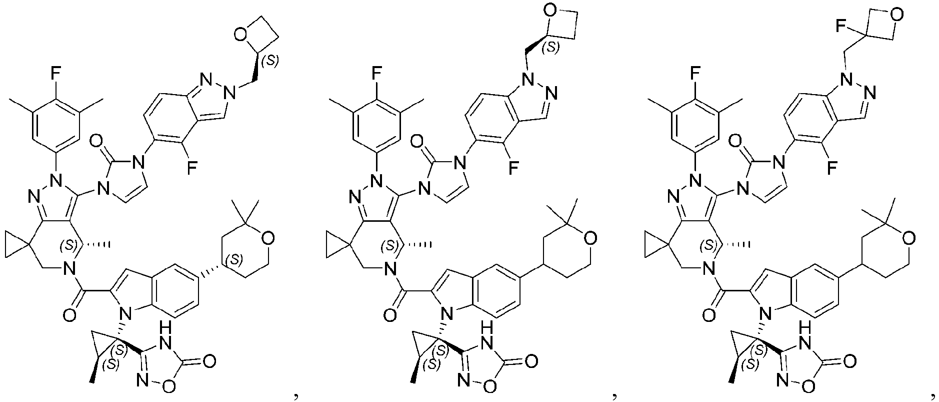 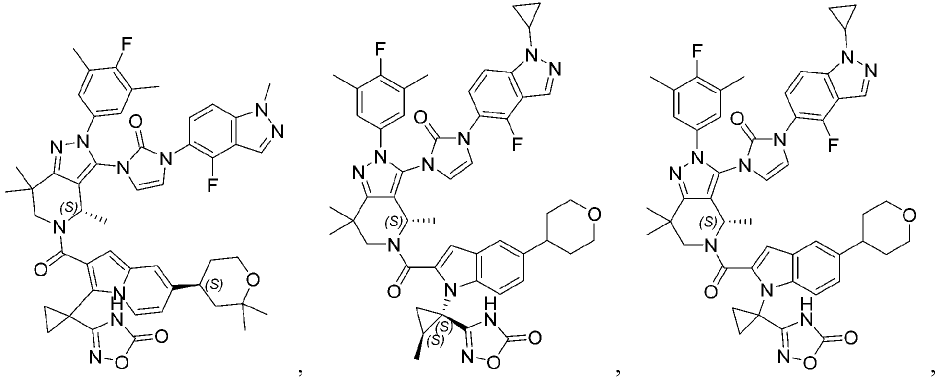 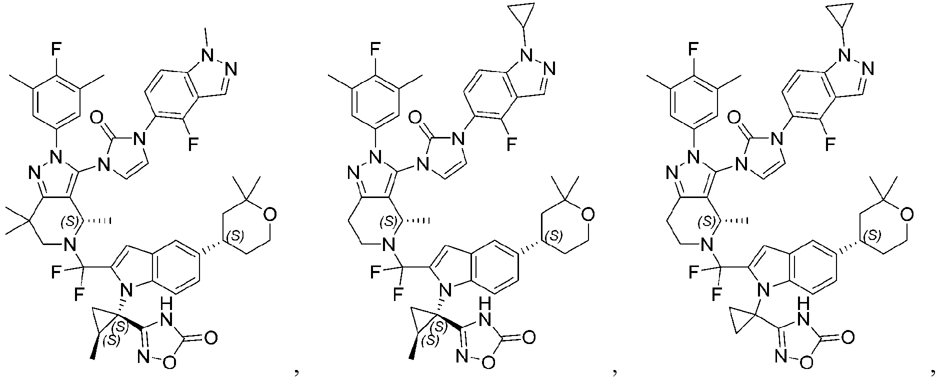 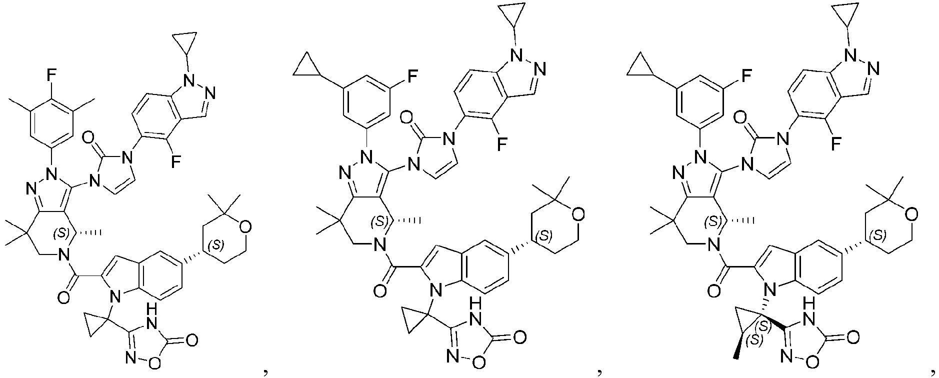 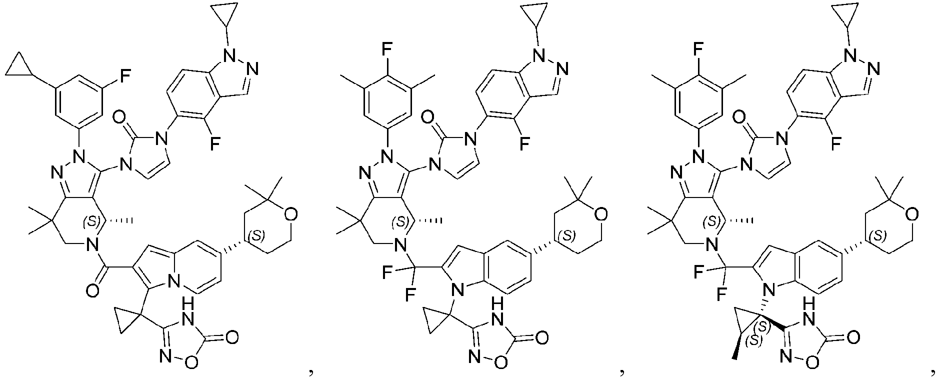 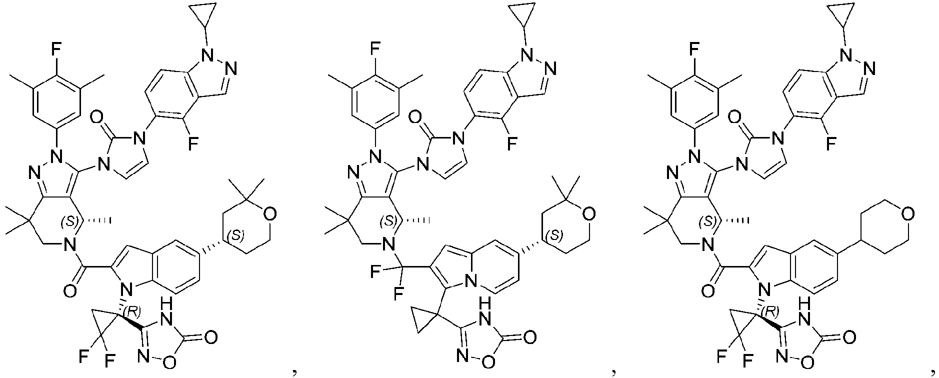 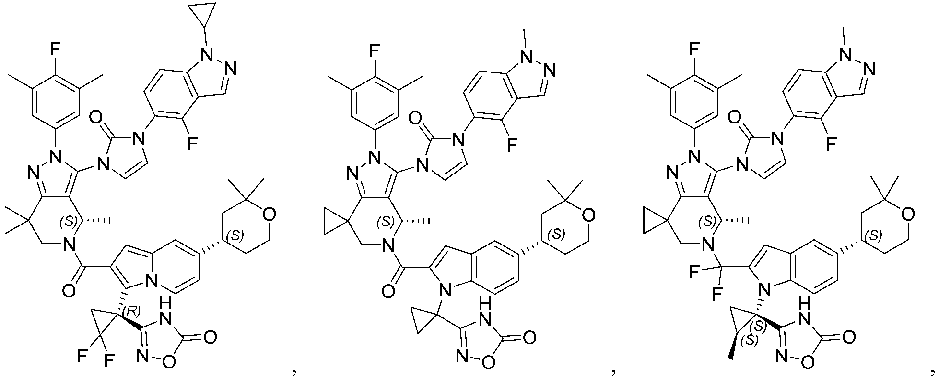 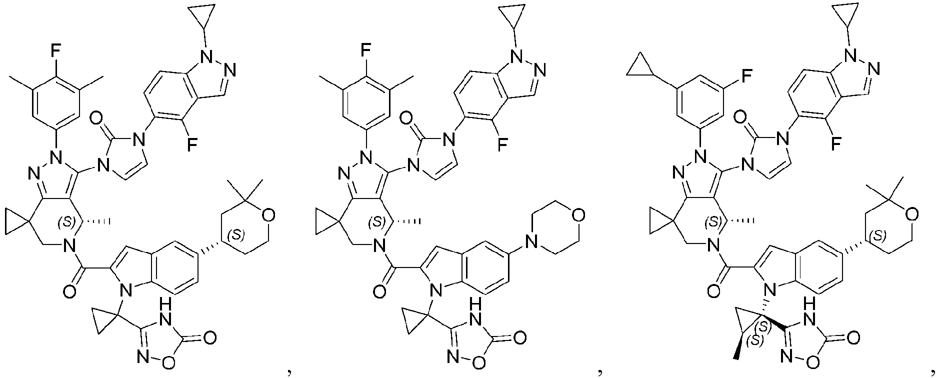 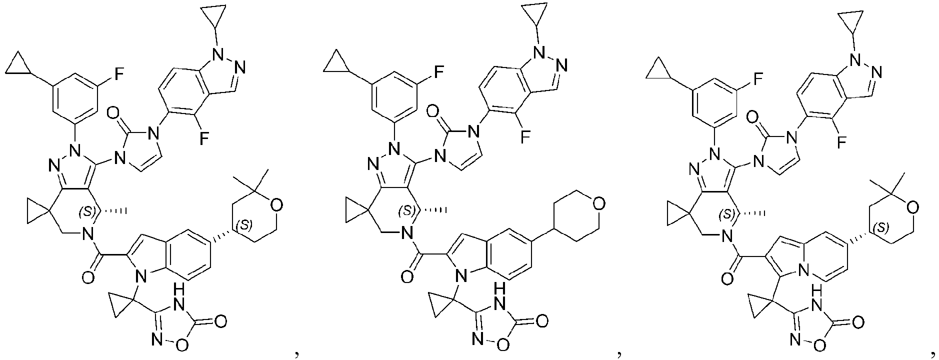 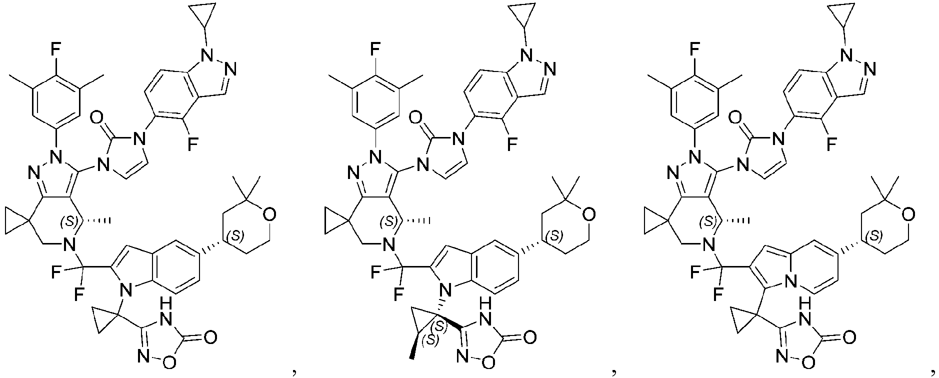 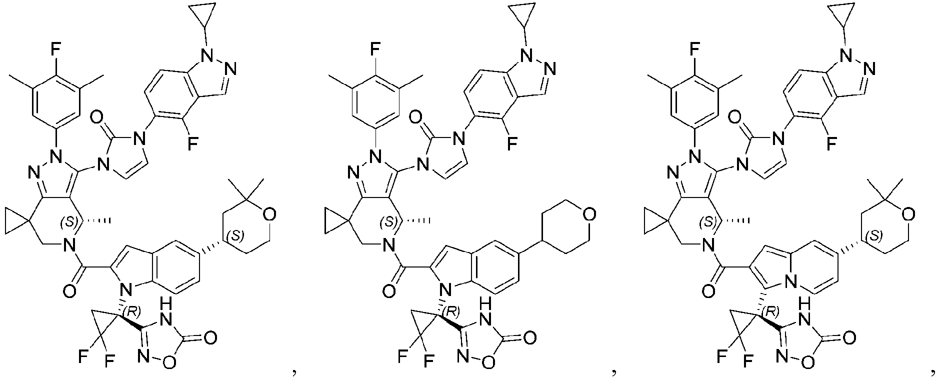 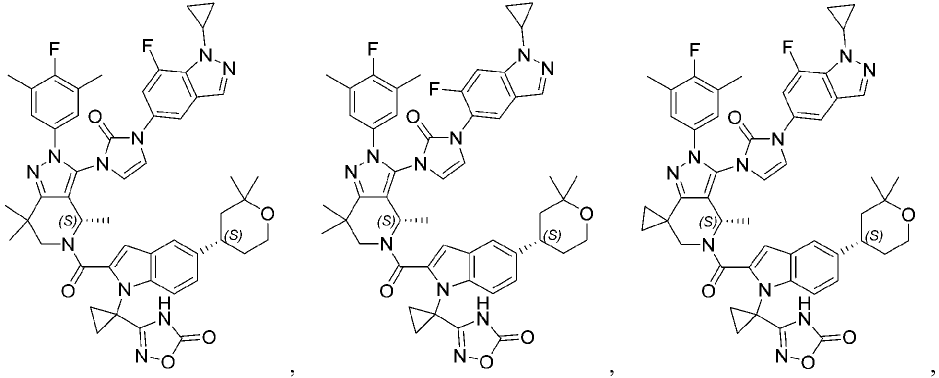 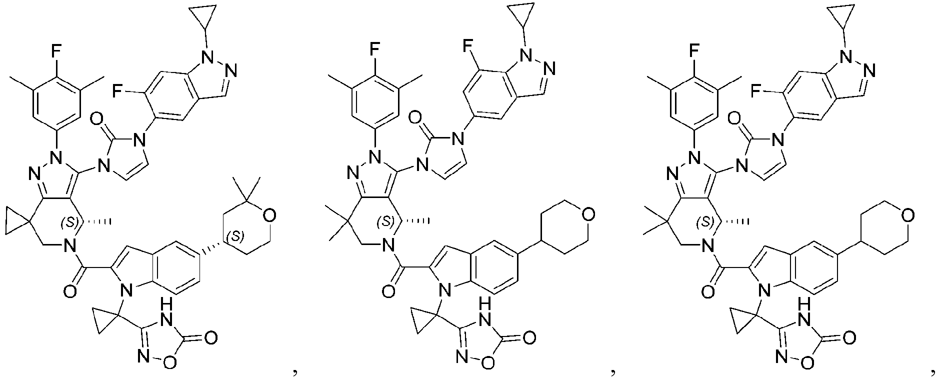 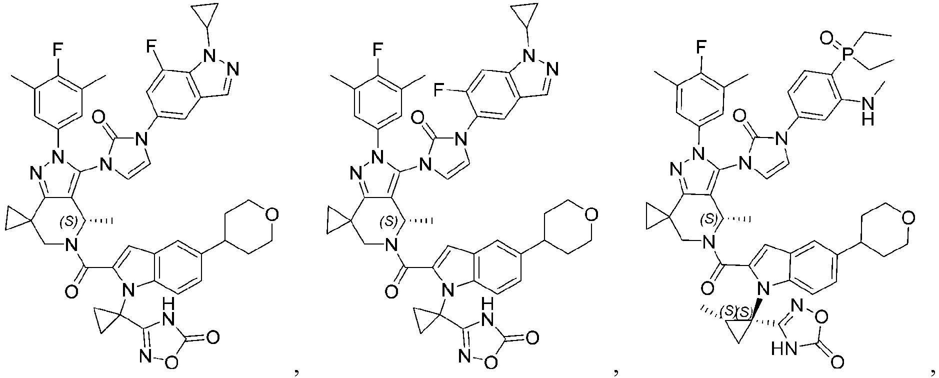 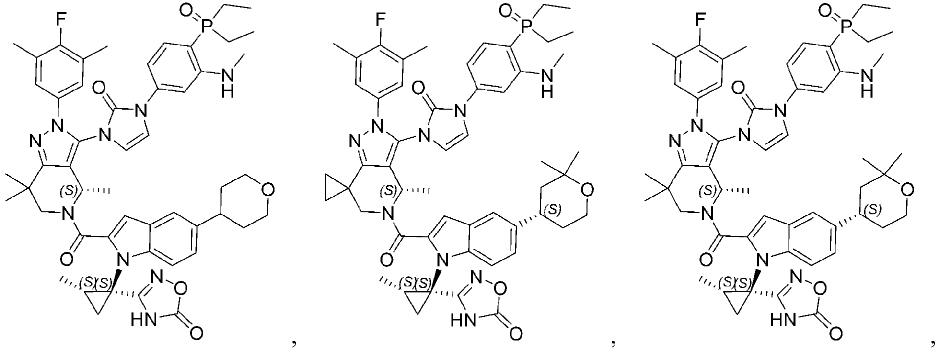 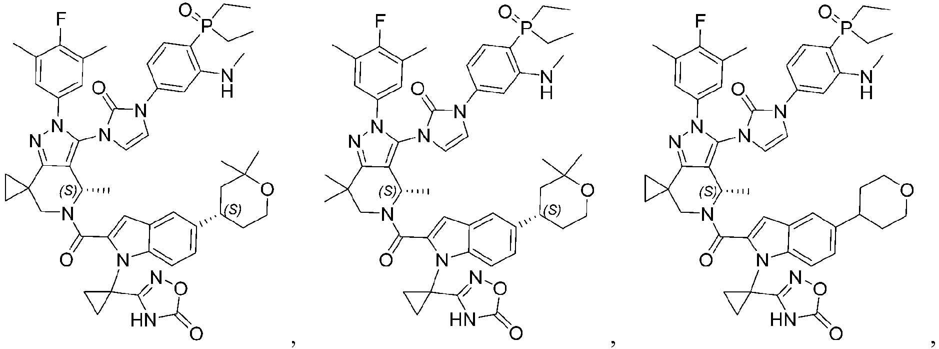 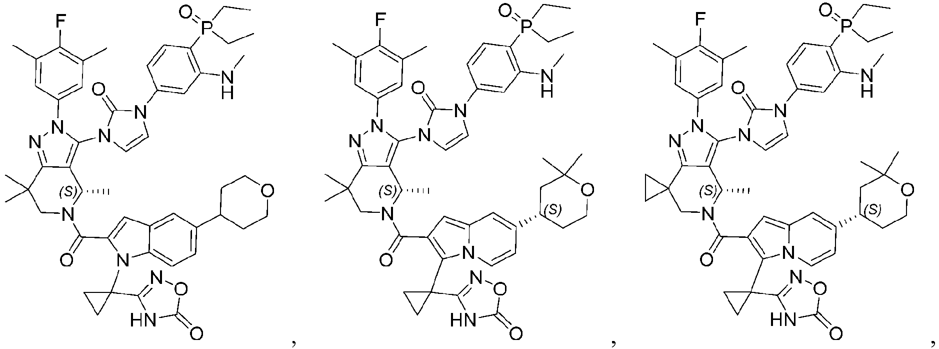 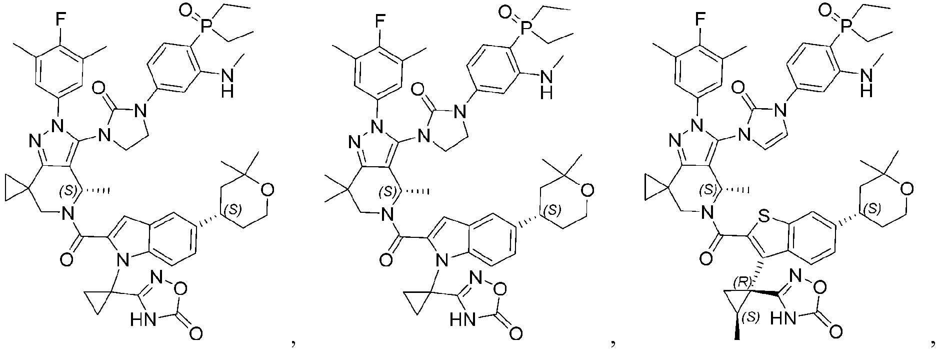 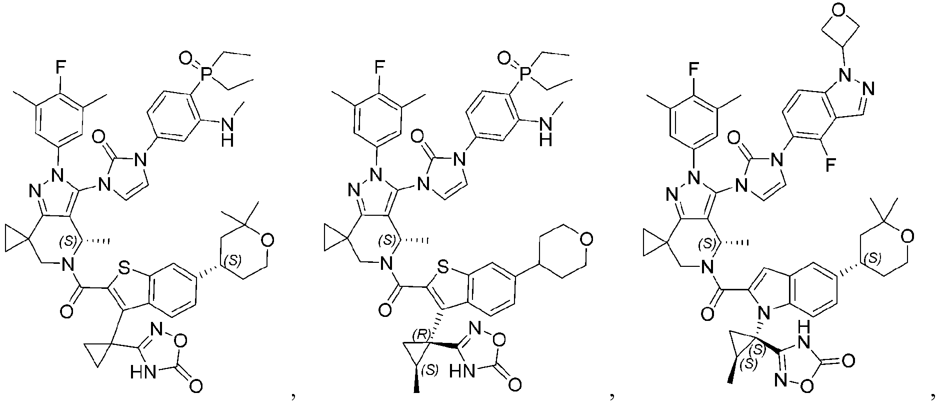 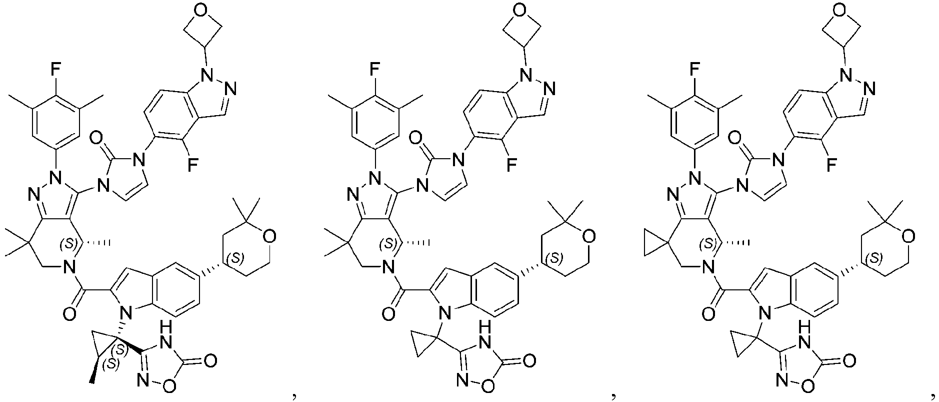 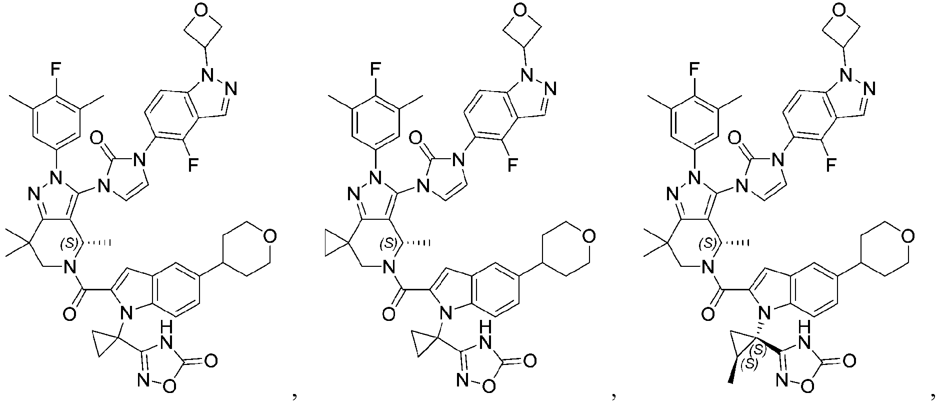 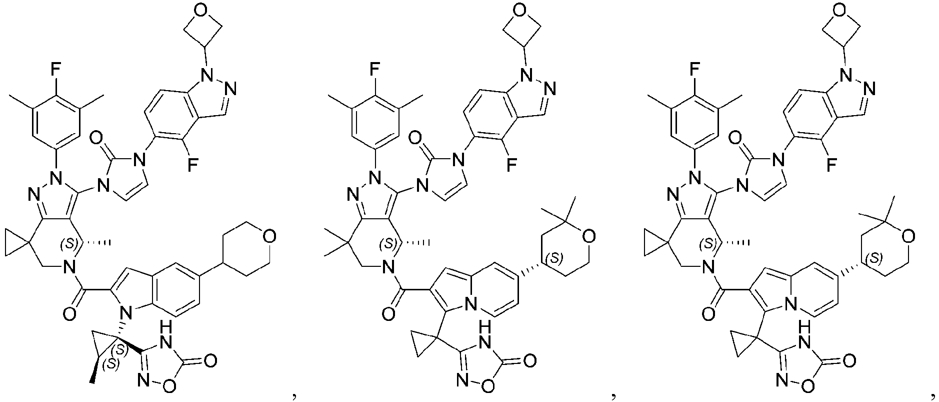 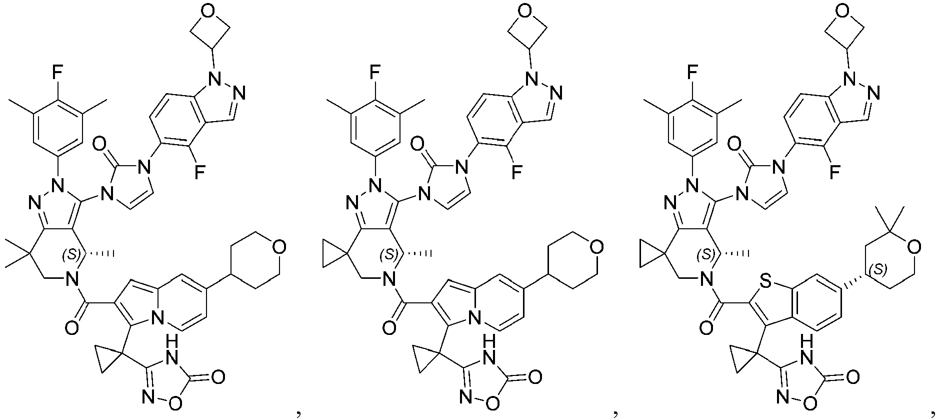 or

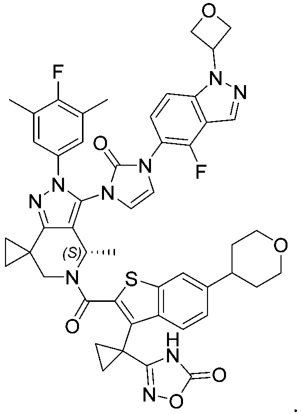

7. A pharmaceutical composition, comprising the compound according to any one of claims 1 to 6, wherein the pharmaceutical composition optionally further comprises a pharmaceutically acceptable excipient, carrier, adjuvant, or any combination thereof.

8. Use of the compound according to any one of claims 1 to 6 or the pharmaceutical composition according to claim 7 in preparation of drugs, wherein the drugs are used for preventing, treating, or alleviating GLP-1 receptor agonist-mediated diseases in patients.

9. The use according to claim 8, wherein the GLP-1 receptor agonist-mediated diseases are diabetes, diabetes complications, non-alcoholic fatty liver disease, obesity, hypertension, hyperlipidemia, hypercholesterolemia, hypertriglyceridemia, atherosclerosis, stroke, coronary heart disease, myocardial infarction, congestive heart failure, arrhythmia, cerebral infarction, diabetic nephropathy, Parkinson's disease, Alzheimer's disease, or dementia.

10. The use according to claim 9, wherein the diabetes is type I diabetes, type II diabetes, gestational diabetes, idiopathic type I diabetes, early-onset type II diabetes, maturity onset diabetes of the young, youth-onset atypical diabetes, malnutrition-related diabetes, or latent autoimmune diabetes in adults.
